# EUROPEAN PATENT APPLICATION

(11) **EP 2 754 715 A1**
(43) Date of publication of application: **16.07.2014**
(21) Application number: 13382006.8
(22) Date of filing: 11.01.2013
(51) Int. Cl.: C12N 15/82, C07K 14/415

(54) **Stress tolerant plants**

(71) Applicant: Consejo Superior De Investigaciones Científicas (CSIC), 41013 Sevilla (ES)
(72) Inventor: Jordano Fraga, Juan, 41012 Sevilla (ES); Almoguera Antolínez, Concepción, 41012 Sevilla (ES); Prieto Dapena, Pilar, 41012 Sevilla (ES); Tejedor Cano, Javier, 41012 Sevilla (ES); Personat Gálvez, Jose Maria, 41012 Sevilla (ES); Carranco Galán, Raúl, 41012 Sevilla (ES)
(74) Representative: Fleck, Barbara

(57) **Abstract**

The invention relates to improving plant traits by expressing plant transcription factors in transgenic plants. The subject of the application is HaHSFA9 and HaHSFA4 and their use for delaying senescence and for improving tolerance to oxidative stress. Chimeric proteins of HaHSFA9 are suggested as well as the expression of both HaHSFA9 and HaHSFA4.

## Description

### STATE OF THE ART

Transcriptional control of the expression of stress-responsive genes is a crucial part of the plant response to a range of abiotic and biotic stresses and developmental processes, including senescence. Research carried out in the past few years has identified transcription factors (TFs) that are important for regulating various plant responses. In some cases, plant TFs have been successfully used to generate transgenic plants with improved traits. However, in the face of increasing food, feed and fuel demand, there remains a need to find alternative ways to increase crop production by the manipulation of plant and plant TFs to improve plant response to a range of abiotic and biotic stresses and developmental processes.

Plant Heat Shock Factors (HSFs) belong to different multigenic families (reviewed by Scharf *et al.,* 2012) and are divided into classes A, B, and C. HSFs from these families are characterised by short conserved sequences (signature sequences) and structural features. These structural features include the length and organization of the oligomerization domain (OD) and flexible linker sequences of variable length (about 15 to 80 amino acid residues) that connect the OD with the DNA-binding domain (DBD). Functional diversification of plant HSFs has been only been analyzed in a few instances and mostly in class A HSFs in tomato, Arabidopsis, sunflower and rice (reviewed by Scharf *et al.,* 2012). The OD of class A HSFs has a characteristic insertion of 21 amino acid residues that extend the OD. That extended OD allows specific homo- and hetero-multimerization between class A HSFs (HSFAs). The same OD sequences are also involved in other specific interactions with non-HSF transcription factors (see for example Díaz-Martín *et al.,* 2005; Carranco *et al.,* 2010). Both kinds of specific interactions contribute to the functional diversification of plant HSFs. A classical example are LpHSFA1 and LpHSFA2 in tomato (Scharf *et al.,* 1998), which preferentially cross-interact; an interaction that prevents nuclear export of LpHSFA2 and combines the activation domain (AD) of the two partner HSFAs in a potent hetero-oligomeric super-activator (Chan-Schaminet *et al.,* 2009). In Arabidopsis, 4 different AtHSFA1s are involved in heat-acclimation responses; these AtHSFA1 hetero-oligomerize (Li *et al.,* 2010) with patterns that could lead to similar AD combination effects as shown in tomato for LpHSFA1 and LpHSFA2. A second example implicates HSFA5 and different HSFA4s in tomato and Arabidopsis (Baniwal *et al.,* 2007). In this case the HSFA hetero-oligomerization leads to repression of transcriptional activation by HSFA4, as HSFA5 interferes with the active oligomeric state and DNA-binding capacity of HSFA4. This could modulate responses to oxidative stress that are mediated by these HSFA4s in rice and Arabidopsis (Yamanouchi *et al.,* 2002; Davletova *et al.,* 2005; reviewed by Scharf *et al.,* 2012). Not all HSFAs can self-oligomerize; neither can they all hetero-oligomerize with other HSFA. Some factors prefer hetero-oligomerization and show specificity: for example, LpHSFA1 prefers LpHSFA2, but does not oligomerize with LpHSFA5 or LpHSFA4b. Conversely, neither LpHSFA5, nor LpHSFA4b, hetero-oligomerize with LpHSFA1 or LpHSFA2. In Arabidopsis, AtHSFA5 represses AtHSFA4a and AtHSFA4c, the sole HSFAs that can hetero-oligomerize with AtHSF5 (Baniwal *et al.,* 2007). None of these specific cross-interactions between plant HSFAs has been shown to regulate defined developmental processes, or the genes involved in such processes.

Different genetic pathways that are active during late stages of zygotic embryogenesis are involved in seed phenotypes such as longevity, basal thermotolerance, and desiccation tolerance. In sunflower, Heat Stress Factor A9 (HaHSFA9) controls one of such pathways, the HaHSFA9 pathway (Almoguera *et al.,* 2002).

HaHSFA9 and the ortholog factors in other plant species (HSFA9) are specialized heat shock transcription factors (HSFs) that are expressed only in seeds, but not in other parts of the plant, and perform functions during embryogenesis at normal growth temperature. The heat stress response in plants involves multiple HSFs, but HSFA9 does not have a role in the vegetative response to high temperature (Carranco, 2010). Thus, the function of HSFA9 is limited to the context of the developing seed.

Over-expression of HaHSFA9 in seeds showed the involvement of this transcription factor in basal thermotolerance and longevity in seeds (Prieto-Dapena *et al.,* 2006). We have also shown that ectopic over-expression of HaHSFA9 conferred dramatic resistance of vegetative tissues from young seedlings to severe dehydration, which was quantified as water loss of up to 98% of total water content (Prieto-Dapena *et al.,* 2008). In both cases, HaHSFA9 activated a genetic pathway that includes specific sHSP genes, which are expressed mainly (or exclusively) during zygotic embryogenesis in seeds; this program does not include genes encoding late embryogenesis abundant (LEA) proteins.

In Arabidopsis, transcription of the HSFA9 gene is activated by ABA-insensitive 3 (ABI3), a key regulator controlling late-seed development (Kotak, 2007). In both sunflower and in Arabidopsis, the HSFA9 program is activated only in seeds.

Environmental stress is the major limiting factor in plant productivity. Much of the injury to plants caused by stress exposure is associated with oxidative damage at the cellular level. Widespread losses of forests and crops due to ozone pollution provide a highly visible example of oxidative stress, but less obvious losses caused by oxidative damage associated with periods of cold or drought also take their toll in the accumulation of incremental setbacks during a growing season.

Reactive oxygen intermediates (ROIs), partially reduced forms of atmospheric oxygen (O₂), are involved in plant oxidative stress damage. They typically result from the excitation of O₂ to form singlet oxygen (O₂¹) or from the transfer of one, two or three electrons to O₂ to form, respectively, a superoxide radical (O₂₋), hydrogen peroxide (H₂O₂) or a hydroxyl radical (HO-).

The role of ROIs in plant stress damage is indicated by the increased production of ROIs and the increased oxidative damage in tissues during stress. In plants, the highly energetic reactions of photosynthesis and an abundant oxygen supply make the chloroplast a particularly rich source of ROIs. High light intensity can lead to excess reduction of PSI so that CO₂, fixation cannot keep pace and NADP+ pools are reduced. Under these conditions, O₂, can compete for electrons from PSI, leading to the generation of ROIs through the Mehler reaction. When CO₂, fixation is limited by environmental conditions such as cold temperatures or low CO₂, availability (closed stomata), excess PSI reduction and increased ROI production can occur even at moderate light intensities. Efficient removal of ROIs from chloroplasts is critical.

In chloroplasts, PSII is a critical site of damage by stress conditions such as dehydration, heat, high-light, and UV-B light [10]. PSI has generally been regarded as a stress-resistant photosystem compared to PSII, at least in normal plants subjected to moderate stress levels. Damage to PSI has been considered to take place only rarely *in vivo,* as in certain plant species or under specific environmental conditions, such as chilling [11]. In particular, desiccation in the dark would irreversibly damage PSI of normal plants, but not of resurrection (desiccation-tolerant) plants (e.g., [12, 13]). ROS, such as superoxide radicals, hydroxyl radicals and H₂O₂, are produced in the stressed chloroplasts. These ROS cause critical oxidation and damage of PSII. Among the intrinsic components of the reaction center of PSII, the chloroplast-encoded D1 protein (PsbA) is particularly vulnerable to ROS damage [14, 15]. The damage of the D1 protein causes reduction of oxygen evolution in the PSII and disruption of the photosynthetic electron flow [10].

Senescence is integral to the flowering plant life-cycle. Senescence-like processes occur also in non-angiosperm land plants, algae and photosynthetic prokaryotes. The term is applied at all levels of plant biology, from processes at the biome-wide scale that define the season of autumn in temperate climates through to the cellular dimension in the terminal development of individual tissues and organs. Plant senescence is commonly considered to be programmed and several genes have been identified as essential for normal initiation and/or execution of the syndrome. Senescence marks the final phase of a leaf's development thereby launching degradation processes integral to the recycling and redistribution of the leaf's nutrients. Plant growth regulators, reproduction, cellular differentiation and hormone levels are internal factors that influence senescence. Environmental stress also influences growth and can promote premature senescence. Certain parts of the plant may be sacrificed to enhance the chances of survival for the rest of the plant. Environmental cues include stress factors that adversely affect plant development and productivity; such as: drought, waterlogging, high or low solar radiation, extreme temperatures, ozone and other air pollutants, excessive soil salinity and inadequate mineral nutrition in the soil. These environmental cues may accelerate leaf senescence by affecting the endogenous factors previously mentioned (Thomas et al).

The importance of research into plant senescence cannot be overemphasized. Senescence processes are unique developmental programs that have a tremendous impact on agriculture. Leaves are the primary organs that absorb light energy from the sun and convert it to chemical energy in the form of sugars via photosynthesis. With the onset of senescence, the photosynthetic capability of a leaf declines sharply. Therefore, leaf senescence limits crop yield and biomass production, and contributes substantially to postharvest loss in vegetable and ornamental crops during transportation, storage and on shelves. In addition, proteins, antioxidants and other nutritional compounds are degraded during senescence. Senescing tissues also become more susceptible to pathogen infection, and some of the pathogens may produce toxins, rendering food unsafe. Mitotic senescence may also determine sizes of leaves, fruits and whole plants. Thus, manipulating senescence for agricultural improvement and enhanced bioenergy production is of great importance to agriculture.

The invention is therefore aimed at mitigating the shortcomings explained above and at providing plants with improved traits.

### SUMMARY OF THE INVENTION

HSFA9 is a seed specific plant transcription factor. The inventors have surprisingly found that overexpression of this TF in germinated plants outside the seed context results in a number of advantageous traits that can be exploited in agriculture. Overexpression of HSFA9 results in plants that are more resistant to oxidative stress and show delayed senescence. The inventors have also shown that the effects of HSFA9 are enhanced when it is co-expressed with HSFA4.

The inventors have found that the HSFA9 pathway includes plastidial small Heat Shock Proteins (sHSPs) that are normally expressed in developing seeds but are present only in lower abundance in heat-stressed leaves. The ectopic overexpression of HaHSFA9 in green organs of tobacco seedlings protected membrane-protein complexes of the two photosystems. It also shielded the D1 protein of PSII and its synthesis from oxidative damage inferred by drastic dehydration or by very harsh treatments with H₂O₂ (e.g., exposure to up to 200 mM H₂O₂ for 24 h in the dark). The PSI core protein PsaB was also protected. Thus, protection at different structural levels explains the limited and recoverable damage of PSI and PSII observed in the 35S:A9 seedlings.

We have cloned and characterized HaHSFA4, an HSF that interacts with HaHSFA9 in plant cells using BiFC assays. HaHSFA4 showed cytosolic and nuclear localization; however, interaction with HaHSFA9 resulted in nuclear retention of HaHSFA4. We have shown that HaHSFA9 and HaHSFA4 synergistically co-activate transcription from promoters of putative target genes of HaHSFA9, as revealed by transient assays performed in sunflower. Using hybrid HSFs containing sequences from HaHSFA9 and from LpHSFA2, a class A HSF unable to act synergistically with HaHSFA4, we determined that only the HSF-interactions that caused nuclear retention of HaHSFA4 resulted in synergistic coactivation.

In transient assays, HaHSFA4 showed no (or little) transcriptional activity by itself, in particular for assays performed in sunflower embryos with a seed-specific sHSP gene promoter. In contrast, HaHSFA4 and HaHSFA9 synergistically activate transcription from the two characterized seed sHSP gene promoters, when tested in sunflower embryos and in leaves. The synergistic interaction of HaHSFA4 and HaHSFA9 in the transcriptional activation of the seed specific G1 promoter involved similar effects, such as an enhancement of nuclear retention of one of the HSFs and the combination of different ADs in each pair of HSFs. Furthermore, HaHSFA4, as HaHSFA9, interacts with HaIAA27, which repressed the synergistic co-activation by HaHSFA9 and HaHSFA4. Our results thus reveal a novel functional interaction of activator plant HSFAs and strongly support that HaHSFA4 is an additional positive regulator of the HaHSFA9 program in sunflower seed embryos. We have shown that plants that combine the overexpression of HaHSFA4 and HaHSFA9 resisted aging better than plants that only overexpress HaHSFA9.

Thus, in a first aspect, the invention relates to a method for delaying senescence of a plant or part thereof said method comprising introducing and expressing a nucleic acid comprising SEQ ID No. 1, a functional homolog, variant or part thereof in a plant or part thereof wherein said plant or part thereof is not a seed.

In a second aspect, the invention relates to a method for improving tolerance to oxidative stress in a plant or part thereof said method comprising introducing and expressing a nucleic acid comprising SEQ ID No. 1, a functional homolog, variant or part thereof in a plant wherein said plant or part thereof is not a seed.

In a third aspect, the invention relates to use of a nucleic acid comprising SEQ ID No. 1, a functional homolog, variant or part thereof in delaying senescence of a plant wherein said plant is not a seed.

In a fourth aspect, the invention relates to a use of a nucleic acid comprising SEQ ID No. 1, a functional homolog, variant or part thereof in improving tolerance to oxidative stress of a plant or part thereof wherein said plant or part thereof is not a seed.

In a another aspect, the invention relates to a method for generating a transgenic plant with delayed senescence comprising introducing and expressing a nucleic acid comprising SEQ ID No. 1, a functional homolog, variant or part thereof in a plant wherein said plant or part thereof is not a seed.

In a another aspect, the invention relates to a method for generating a transgenic plant with improved tolerance to oxidative stress comprising introducing and expressing a nucleic acid comprising SEQ ID No. 1, a functional homolog, variant or part thereof in a plant wherein said plant or part thereof is not a seed.

The invention further relates to a method for improving tolerance to oxidative stress and /or improving tolerance to dehydration in a plant said method comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant.

The invention further relates to a method for delaying senescence of a plant or part thereof said method comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant wherein said plant or part thereof is not a seed.

In another aspect, the invention relates to a use of a nucleic acid comprising SEQ ID No. 1, a functional homolog, variant or part thereof in combination with a nucleic acid comprising SEQ ID No. 3, a functional homolog, variant or part thereof for improving tolerance to oxidative stress and /or improving tolerance to dehydration in a plant.

In another aspect, the invention relates to a use of a nucleic acid comprising SEQ ID No. 1, a functional homolog, variant or part thereof in combination with a nucleic acid comprising SEQ ID No. 3, a functional homolog, variant or part thereof for delaying senescence of a plant or part thereof wherein said plant or part thereof is not a seed.

The invention further relates to a chimeric construct comprising the LNK, OD and AD domains of SEQ ID No. 1, a functional homolog, variant or part thereof fused to N-terminal residues of a different HSF, preferably a class A HSF.

In another aspect, the invention relates to a vector or isolated cell comprising the chimeric construct described above.

In another aspect, the invention relates to an isolated nucleic acid comprising SEQ ID No. 3 or functional variant thereof.

In a final aspect, the invention relates to a transgenic plant co-expressing a nucleic acid comprising SEQ ID No. 3 and a nucleic acid sequence comprising SEQ ID No. 1.

### DESCRIPTION OF THE FIGURES

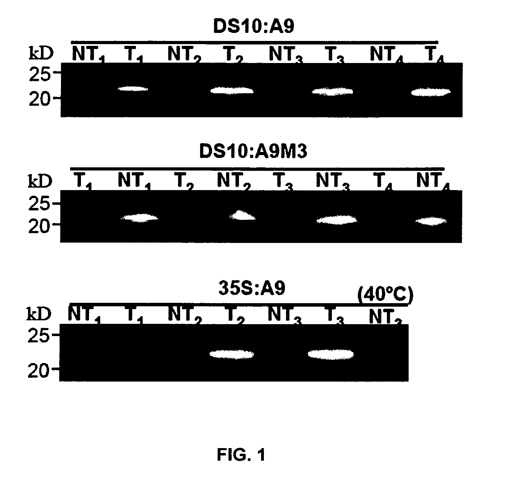

**Figure 1****. Effects of gain and loss of function of HaHSFA9 on the accumulation of plastidial sHSPs in seeds and seedlings.** Immunoblot detection with the anti-HSP21 antibody at 1/2,500 dilution. Different transgenic lines (T) were compared with sibling non-transgenic (NT) lines. From top to bottom: DS10:A9 seeds, DS10:A9M3 seeds, and 35S:A9 seedlings. Protein amounts loaded: 25 µg total protein for the seed samples and for the 40°C seedling sample; 15 µg for the rest of seedling samples. Enhanced detection conditions were used for the DS10:A9M3 samples with respect to DS10:A9 (90 min instead of 5 min exposure, compare the respective NT signals). The high-level accumulation of plastidial sHSPs in unstressed seedlings of the 35S:A9 lines was compared with that in NT seedlings after heat-acclimation for 3 h at 40°C (40°C). Molecular mass markers (kD) are indicated to the left.

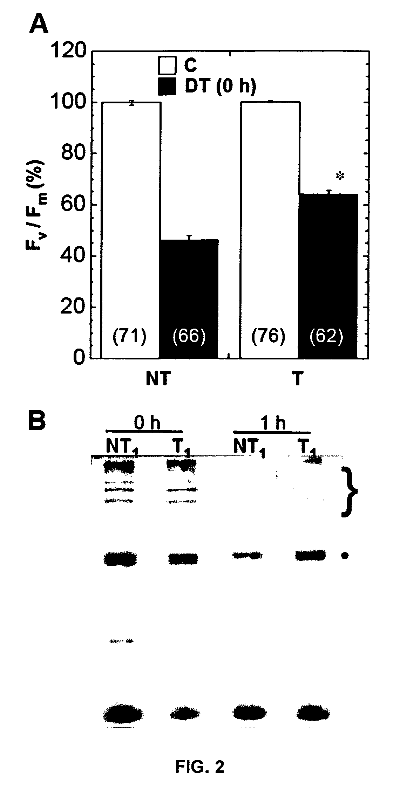

**Figure 2****. Protection of PSII from damage caused by severe dehydration. (A)** Comparison of maximum quantum yield (Fᵥ/Fₘ) values of transgenic seedlings (T) with those for the non-transgenic seedlings (NT), determined in control growth conditions (C) and immediately after the dehydration treatment [DT (0 h)]. Average values obtained in 4 to 5 independent experiments performed with two different T/NT line pairs. The difference observed for the DT (0 h) values was statistically significant (F= 194.41, P= 0.0001) as indicated by the asterisk. Numbers in brackets indicate the different Fᵥ/Fₘ determinations performed in each case. **(B)** Enhanced stability of PSII membrane protein complexes. The complexes were visualized by BN-PAGE using samples prepared from dehydrated seedlings analyzed immediately after the dehydration treatment (0 h), and following rehydration for 1 h (1 h). The gel was stained with colloidal Coomassie blue. Symbols (•, and the bracket on top) mark the dimeric PSII complex and the PSII super-complexes mentioned in the text. Representative results for the T₁/NT₁ sibling pair are shown.

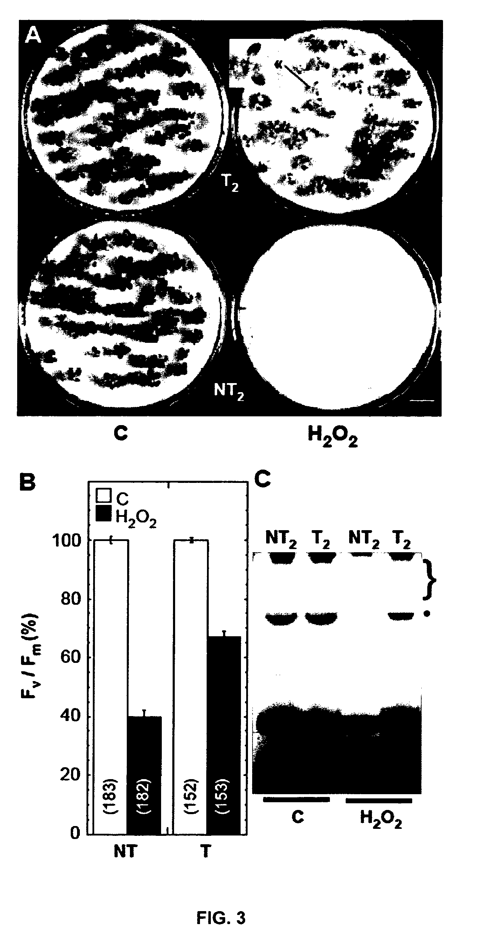

**Figure 3****. Protection of PSII from damage caused by drastic oxidative stress conditions. (A)** The 35S:A9 seedlings survive drastic oxidative stress conditions. Representative results for the T₂/NT₂ line pair are shown. Differences in green color were evident between seedlings grown in control conditions (C). Most 35S:A9 seedlings (T₂) survived treatments for 24 h with 200 mM H₂O₂ (H₂O₂) that caused complete death of sibling NT₂ seedlings. The 3x-magnified inset shows bleaching of the cotyledons in the surviving T₂ seedlings. Scale bar, 1 cm. **(B)** Comparison of maximum quantum yield (Fᵥ/Fₘ) of PSII between 35S:A9 (T) and NT seedlings after control (C) and H₂O₂ treatments (H₂O₂). Average values for nine independent experiments performed with three different T/NT line pairs. Statistical difference between the Fᵥ/Fₘ values (F= 265.58, P= 0.0001) and rest of symbols indicated as in Figure 2A. **(C)** PSII membrane protein complexes of 35S:A9 seedlings partially resisted the H₂O₂ treatments. Thylakoids from control (C) and H₂O₂ treated seedlings (H₂O₂) were analyzed essentially as indicated in Figure 2B. The H₂O₂ samples were prepared immediately after the treatment and the gel was photographed after BN-PAGE without further staining. Symbols for PSII complexes as in Figure 2B.

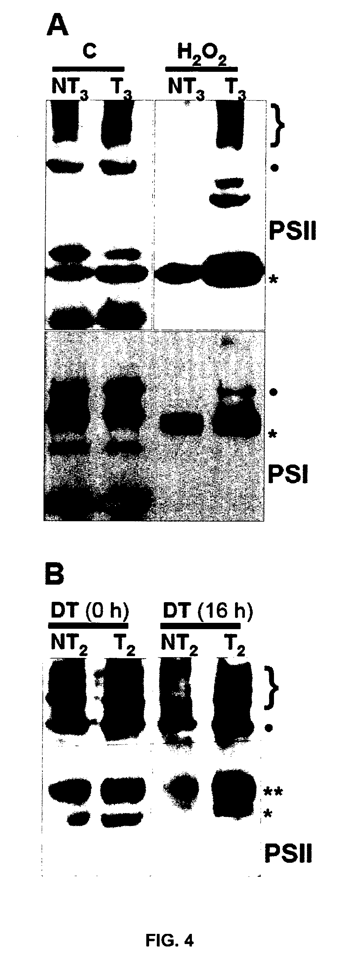

**Figure 4****. Membrane protein complexes of PSII and PSI survive in the 35S:A9 seedlings. (A)** Western detection of complexes after the H₂O₂ treatments. The PSII complexes (top) separated by BN-PAGE were detected using anti-D1 (DE-loop) antibodies at 1/5,000 dilution. The PSI complexes (bottom) were detected using anti-PsaB antibodies at 1/4,000 dilution. PSII symbols: •, and the bracket on top respectively mark the dimeric PSII complex, and the PSII-LHCII super-complexes. The asterisk marks the CVII (CP43-less PSII monomer) complex. PSI symbols: • marks the PSI-LHCI super-complex that co-migrates in our gel system with the dimeric PSII complex; the asterisk marks the PSI monomer. **(B)** The PSII complexes also withstand drastic dehydration. The thylakoid samples were analyzed immediately (0 h) after the dehydration treatment (DT), and following rehydration for 16 h, DT (16 h). In this case the complexes were detected using anti-D1 (C-terminal) antibodies at 1/15,000 dilution. An additional PSII complex mentioned in the text is indicated: CV (**, PSII monomer).

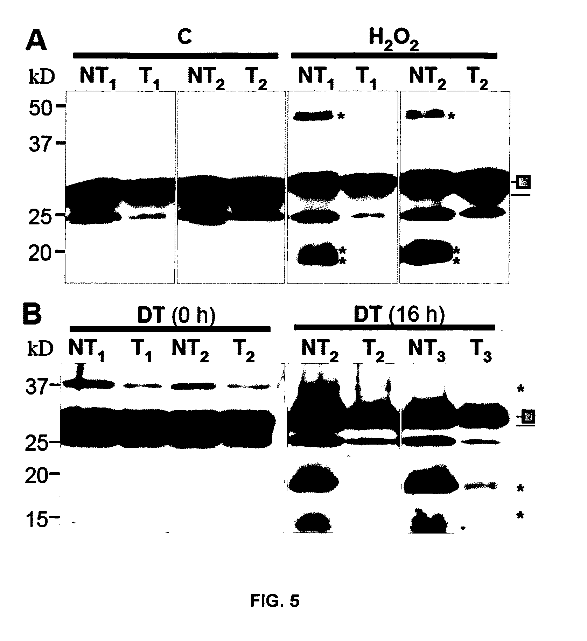

**Figure 5****. The D1 protein of PSII is protected from damage caused by oxidative stress and severe dehydration. (A)** Damage by the H₂O₂ treatments (H₂O₂) compared to control conditions (C). **(B)** Protection from damage by the dehydration treatments (DT). The DT samples were analyzed immediately after the dehydration treatment (0 h), and following rehydration for 16 h (16 h). Thylakoid protein samples from the indicated transgenic lines (T) were compared with sibling non-transgenic (NT) lines. Protein amounts loaded per lane in order to obtain similar initial amounts of the D1 protein: NT₁ (40 µg), T₁ (10 µg), NT₂ (20 µg), T₂ (10 µg), NT₃ (20 µg), and T₃ (10 µg). Immunoblot detection was performed with anti-D1 (DE-loop) antibodies at 1/2,000 (A) or 1/6,000 dilution (B). To the right of each panel, a thin line marks the position of the major D1 band; slightly above, a shaded square marks the retarded mobility D1 band observed after the stress treatments. Asterisks above and below the major D1 bands respectively indicate the cross-linked and degraded D1 protein bands mentioned in the text. Molecular mass markers (kD) are indicated to the left.

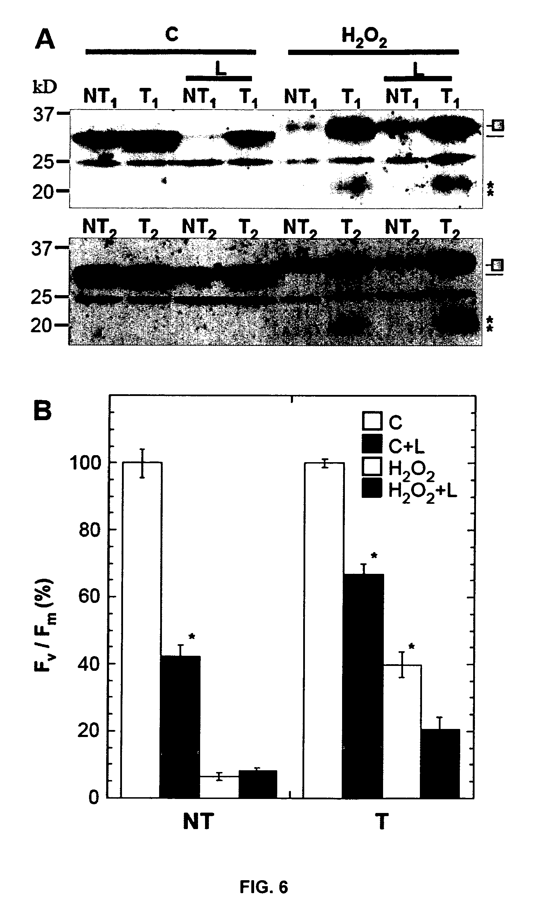

**Figure 6****. Delayed degradation of the D1 protein and protection of the D1 protein synthesis in the 35S:A9 seedlings. (A)** Accumulation of the D1 protein under control growth conditions (C) and after treatments with H₂O₂ (H₂O₂). In each case the seedlings were exposed to 6 h normal light conditions in absence or in presence of 1mM lincomycin (L). Sample labels and rest of symbols as in Figure 5A. Protein amounts loaded per lane as in Figure 5. **(B)** Comparison of maximum quantum yield (Fᵥ/Fₘ) of PSII between 35S:A9 seedlings (T) and sibling NT seedlings in the experimental conditions used in (A). We show average values from a representative experiment performed with three different T/NT line pairs (n= 18, for all conditions). Asterisks indicate the significant statistical differences found between the Fᵥ/Fₘ values obtained in absence and presence of lincomycin: for control NT seedlings (F= 111.6, P= 0.0001); for control T seedlings, (F= 59.21, P= 0.0001); and for stressed T seedlings (F= 37.75, P= 0.0001).

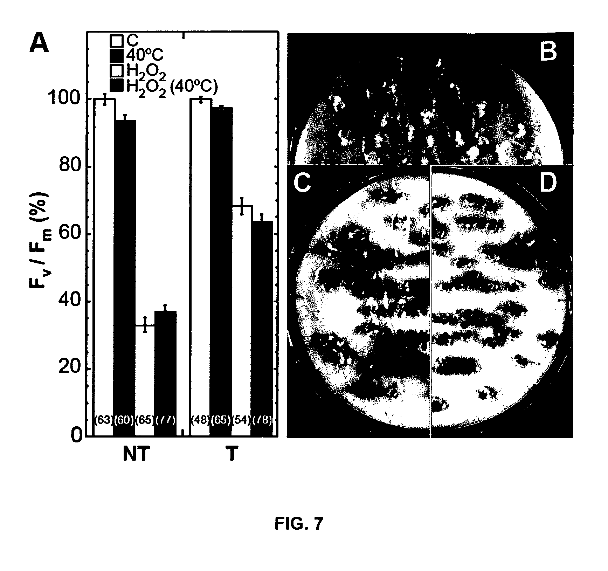

**Figure 7****. Heat acclimation does not protect PSII from damage caused by the drastic oxidative stress conditions. (A)** Maximum quantum yield (Fᵥ/Fₘ) of PSII for 35S:A9 seedlings (T) compared to sibling NT seedlings. The effects of standard H₂O₂ treatments were analyzed as in Figure 3B (compare the C and H₂O₂ samples), and using seedlings that were first heat-acclimated during 3 h at 40°C. The corresponding control and H₂O₂-treated samples are labelled 40°C and H₂O₂ (40°C), respectively. The average values for three to four experiments performed with three different T/NT line pairs are shown. Numbers in brackets indicate the different Fᵥ/Fₘ determinations performed in each case. Heat-acclimation did not induce significant differences in Fᵥ/Fₘ after H₂O₂ treatments in NT (F= 1.94, P= 0.16) or T (F= 3.32, P= 0.07) seedlings. **(B)** The heat-acclimated NT seedlings did not survive the H₂O₂ treatments. **(C)** Survival of non-acclimated T seedlings. **(D)** Survival of heat-acclimated T seedlings.

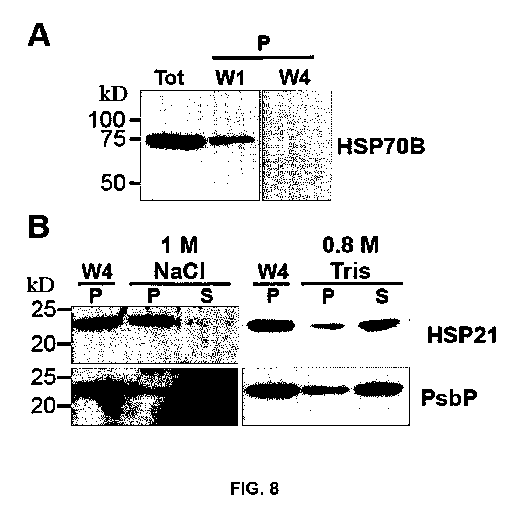

**Figure 8****. The HSP21-like polypeptides in the 35S:A9 seedlings associate with thylakoid membranes at normal growth temperatures.** (A) Washing of thylakoid membranes removes contamination from stromal HSP70B. Comparison of signals obtained for total protein extracted from seedlings (Tot) and from pellets (P) of thylakoid preparations that where washed once (W1) or four times (W4). The amounts of loaded protein corresponded to an equivalent amount of chlorophyll: 2 µg in all lanes. Antibodies against the Arabidopsis HSP70B protein (Agrisera AS08348) were used at 1/8,000 dilution. **(B)** Comparison of the HSP21 and PsbP proteins in pellet (P) and soluble (S) fractions after treatments with 1 M NaCl or 0.8 M Tris-HCl, pH 8.4, for 60 min at 25°C, followed by centrifugation for 20 min at 16,000g and 4°C. Protein amounts in each fraction were compared with the initial total amounts in thylakoid pellets that were washed four times (W4/P). These amounts correspond to 0.5 µg (sHSP-P detection), or 0.2 µg of chlorophyll (PsbP detection). Antibodies against HSP21 or PsbP (Agrisera AS06167) were both used at 1/3,000 dilution.

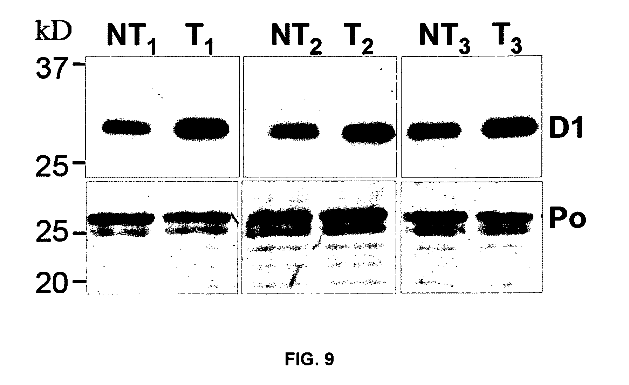

**Figure 9****. Enhanced accumulation of the D1 protein in thylakoid membranes of the 35S:A9 seedlings.** The 3 pairs of T/NT sibling lines were compared. Sample amounts of thylakoid protein loaded: 0.15 µg for D1 detection in all lanes. Equal loading was verified with Ponceau S staining (Po) using a higher amount thylakoid protein from the same samples: 20 µg. Antibodies against the C-terminal region of the D1 protein were used at 1/15,000 dilution. Molecular mass markers (kD) are indicated.

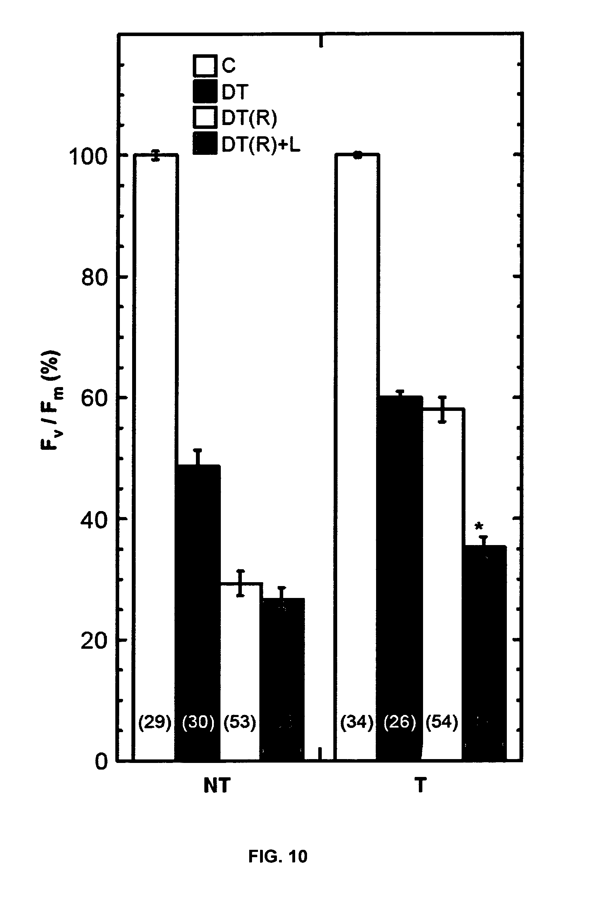

**Figure 10****. Protection of plastidial protein synthesis in the dehydrated 35S:A9 seedlings.** Maximum quantum yield (Fᵥ/Fₘ) of PSII for 35S:A9 seedlings (T) compared to sibling NT seedlings. The effect of 1 mM lincomycin (L) was analyzed. Lincomycin was added during 16 h of rehydration under normal light conditions (R) of seedlings subjected first to dehydration treatments [DT(R)]: compare DT(R) with DT(R)+L. The Fᵥ/Fₘ values obtained immediately after dehydration are also indicated (DT). We show average values from three independent experiments performed with two different T/NT line pairs. Numbers in brackets indicate the total number of Fᵥ/Fₘ determinations in each condition.

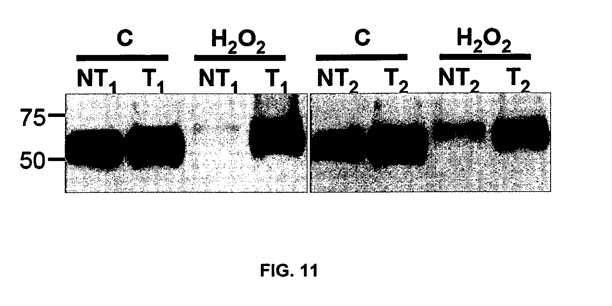

**Figure 11****. The PsaB protein of PSI is protected from damage caused by oxidative stress in the 35S:A9 seedlings.** The same protein samples from experiments analyzed in Figure 7A for D1 protection were used here. Immunoblot detection was performed using anti-PsaB antibodies at 1/5,000 dilution. Sample labels are described in the legend of Figure 7A.

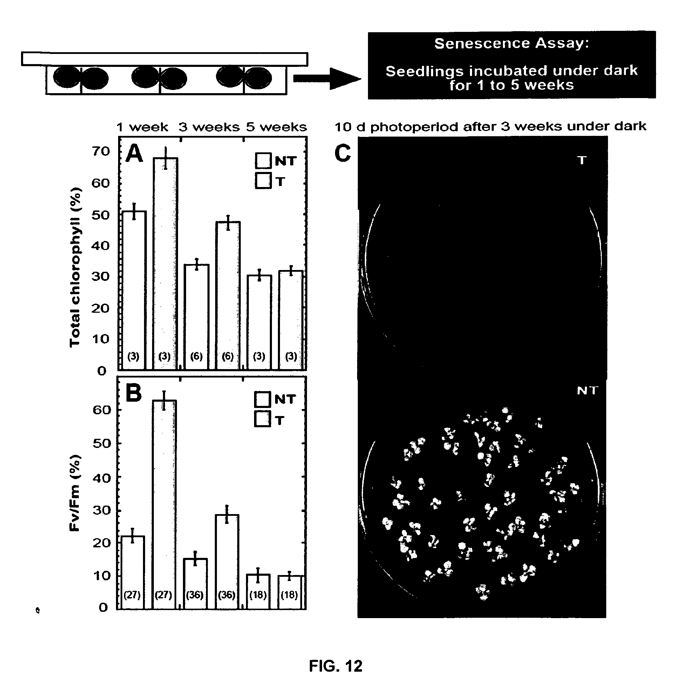

**Figure 12****. Overexpression of HSFA9 delays senescence in seedlings** Resistance to lethal conditions of dark-induced senescence in the 35S:A9 seedlings.

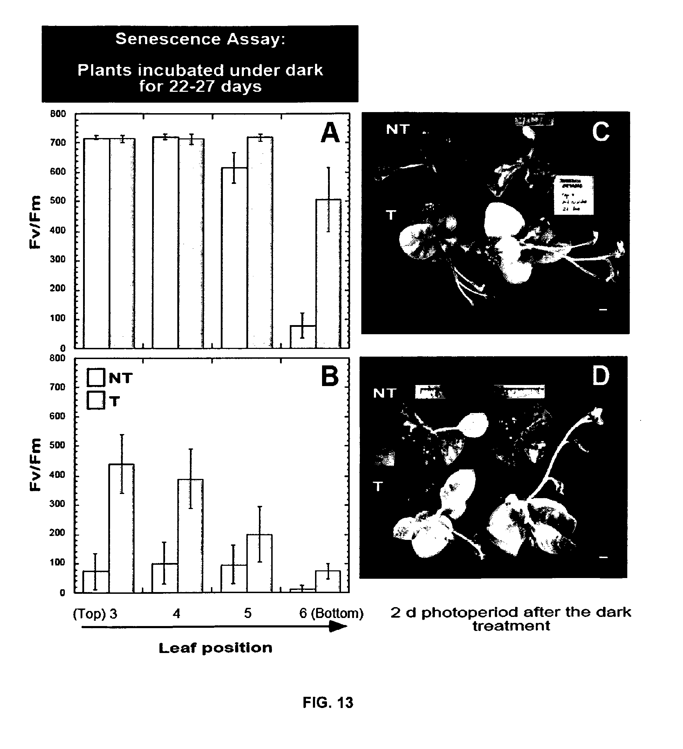

**Figure 13****. Overexpression of HSFA9 delays senescence in seedlings** The enhanced resistance to dark-induced leaf senescence persists in 4-week old 35S:A9 plants grown on soil.

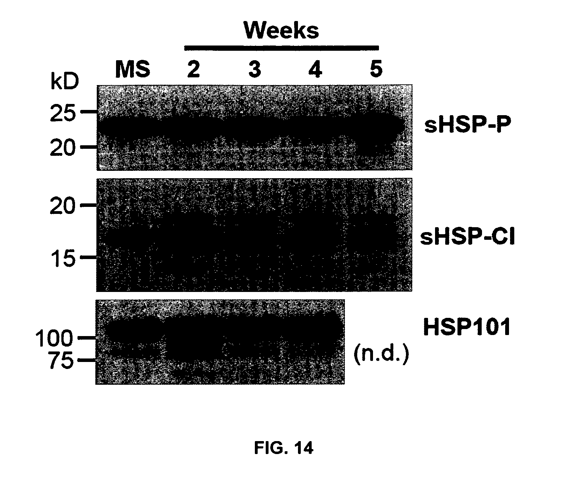

**Figure 14****. Overexpression of HSFA9 delays senescence in seedlings** Persistence of molecular effects of HaHSFA9

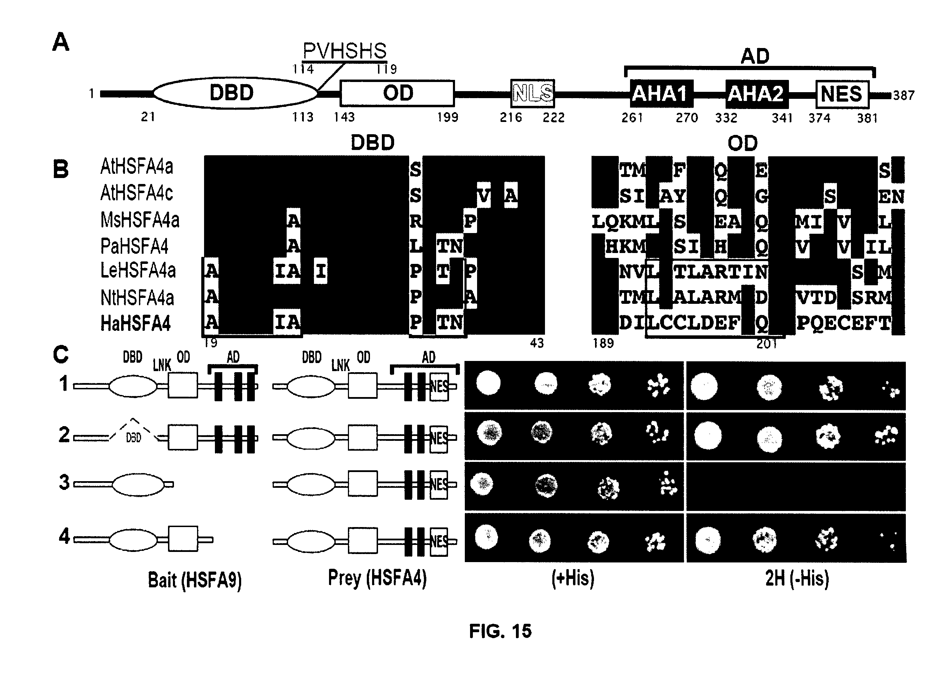

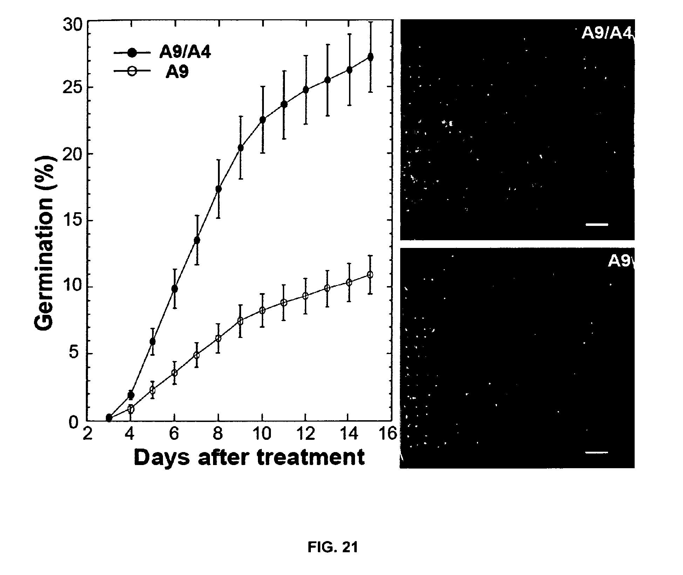

**Figure 15****. HaHSFA4, a peculiar HSF that belongs to Class A4: yeast two-hybrid interaction with HaHSFA9.** (A) localization of protein domains that characterize HaHSFA4: the DNA-binding (DBD), oligomerization (OD) and C-terminal activation (AD) domains are indicated. We also show the positions of nuclear localization (NLS) and nuclear export (NES) sequences, as well as that of the two AHA motifs (AHA1, AHA2) within the AD. The Class A4 signature sequences (PVHSHS) located next to the DBD C-terminal end are depicted. **(B)** Partial sequence alignment of HaHSFA4 with other Class A4 HSFs. Boxes show amino acid residues (marked in yellow) conserved only between HaHSFA4 and similar HSFs from *Fabaceae* and *Solanaceae.* For full sequence details of HaHSFA4, see Figure 21. **(C)** Yeast two-hybrid interaction. Aliquots of yeast strains with the indicated "Bait", HSFA9, and "Prey", HSFA4, plasmids were spotted at decreasing cell density (1:10 dilution steps, from left to right). Interactions were confirmed by observing yeast growth on selective media [2H (-His)], compared with growth on media without selection (+His). The "Bait" plasmids: 1. - HaHSFA9mutAD; 2. - HaHSFA9mutADDBD; 3. - HaHSFA9Δ165; 4. - HaHSFA9Δ248.

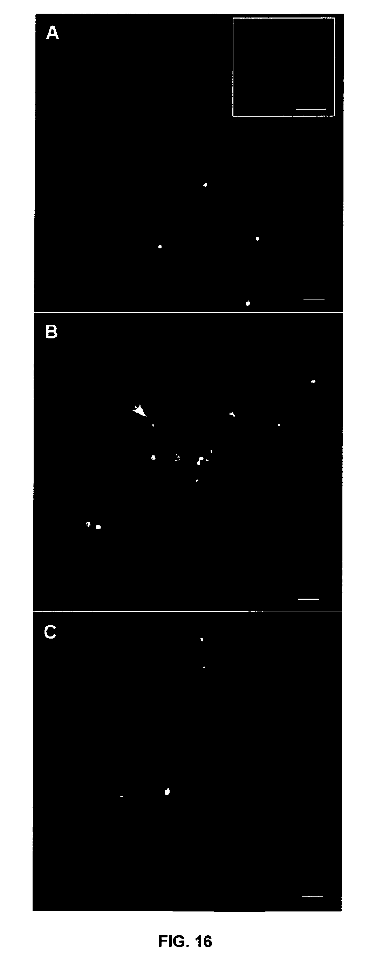

**Figure 16****. BiFC interaction between HaHSFA9 and HaHSFA4 in leaves of *Nicotiana benthamiana.*** Confocal microscopy images of epidermal leaf cells infiltrated with mixtures of *Agrobacterium* cell suspensions harboring constructs that encode the following fusion proteins: A, HaHSFA9:YFP^{N}+HaHSFA4:YFP^{C}; the inset shows nuclear fluorescence at higher magnification. B, HaHSFA4:YFP^{N}+HaHSFA4:YFP^{C}; the arrow marks fluorescence in a cytosolic band. C, HaHSFA9:YFP^{N}+HaHSFA9:YFP^{C}. Scale bars 50 µm and 12.5 µm (inset).

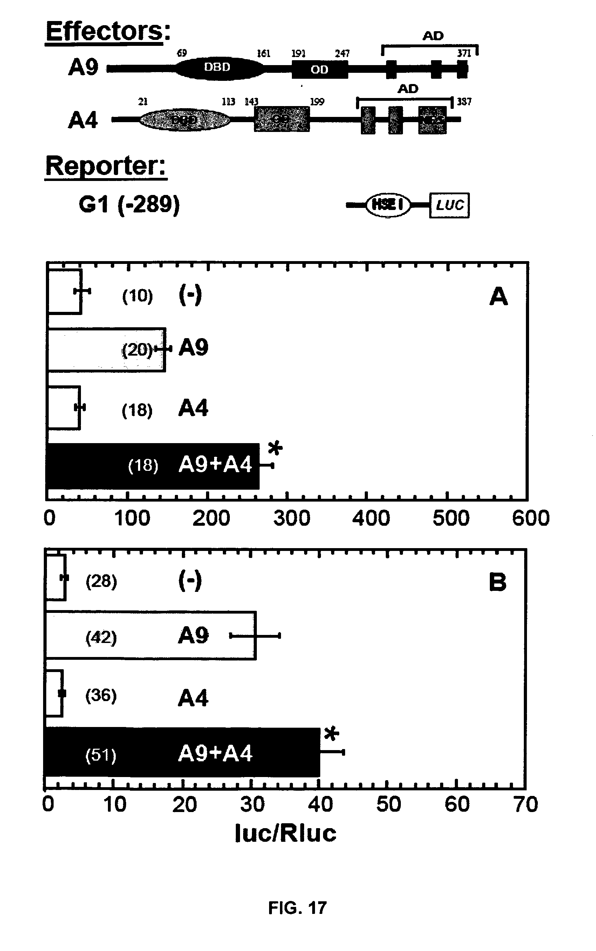

**Figure 17****. Synergistic transcriptional activation of the G1 promoter by HaHSFA9 and HaHSFA4.** Sunflower 20 dpi embryos (A) or leaves (B) were bombarded with the *-289(G1):LUC* [G1 (-289)] reporter gene and the following effector plasmids: none (-), HaHSFA9 (A9), HaHSFA4 (A4), or HaHSFA9+HaHSFA4 (A9+A4). Effector and reporter maps are shown at the top of the figure. Mean *Photinus* luciferase (luc) activity was normalized with *Renilla* luciferase (Rluc). Numbers in parentheses show the number of replicates for each reporter/effector combination. The same bar shading indicates similar reporter activity. Asterisks denote statistically significant synergism between HaHSFA9 and HaHSFA4.

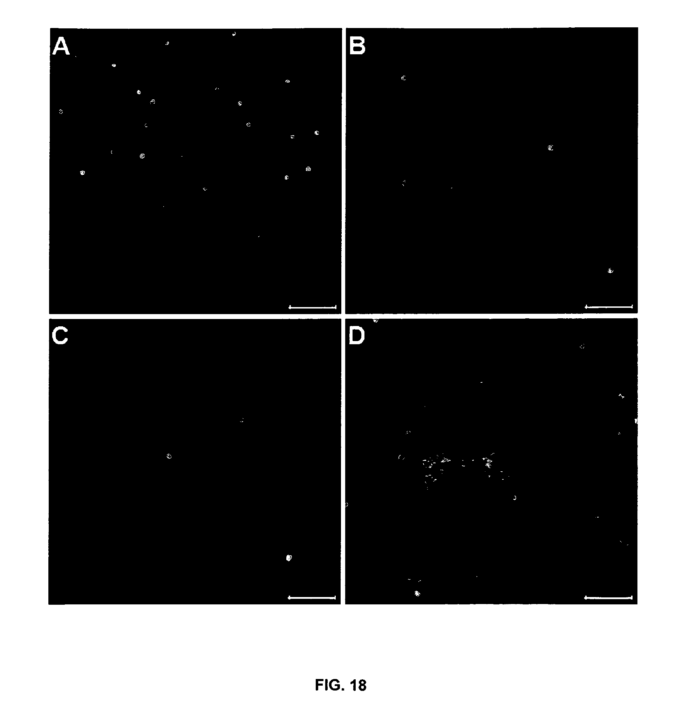

**Figure 18****. Mutation of the HaHSFA4 NES motif, or co-expression of HaHSFA4 with HaHSFA9 enhances nuclear localization of HaHSFA4 in *Nicotiana benthamiana.*** Leaves were infiltrated with mixtures of *Agrobacterium* suspensions, and then analyzed by confocal microscopy. The analyzed mixtures expressed the following proteins: A, HaHSFA4(NESmut):GFP; B, HaHSFA4:GFP + HaHSFA9 (non-fluorescent); C, HaHSFA4:GFP + A2:A9₂hybrid protein (non-fluorescent); D, HaHSFA4:GFP + A2:A9₁hybrid protein (non-fluorescent). Scale bars, 100 µm.

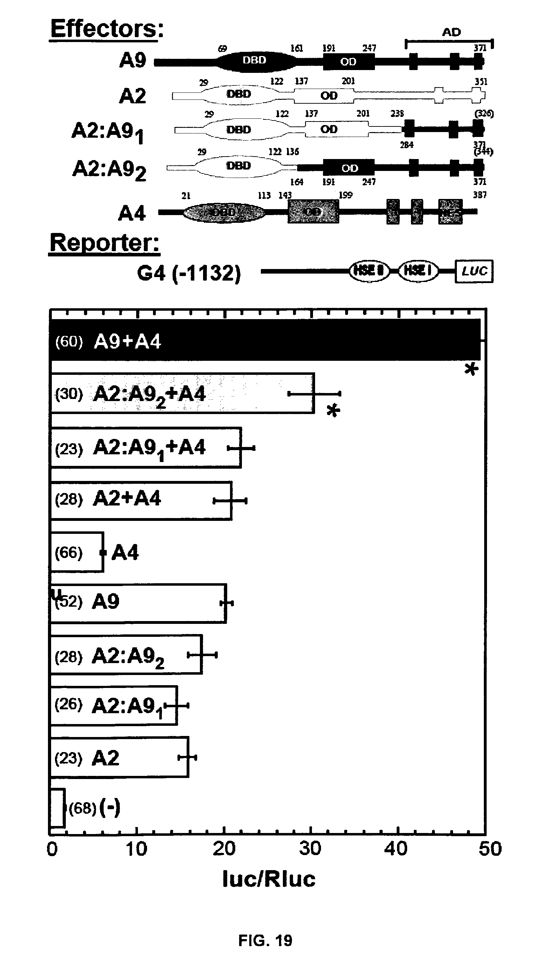

**Figure 19****. Requirements for the synergistic interaction between HaHSFA4 and HaHSFA9.** Sunflower leaves were bombarded with the - *1132(G4):LUC[G4* (-1132)], reporter gene and the indicated effector plasmid(s), used individually or in combination: none (-), LpHSFA2 (A2), A2:A9₁ hybrid HSF (A2:A9₁),A2:A9₂ hybrid HSF (A2:A9₂), HaHSFA9 (A9), HaHSFA4 (A4), LpHSFA2+HaHSFA4 (A2+A4), A2:A9₁ hybrid HSF + HaHSFA4 (A2:A9₁ +A4), A2:A9₂ hybrid HSF + HaHSFA4 (A2:A9₂ +A4), and HaHSFA9+HaHSFA4 (A9+A4). Maps for the reporter gene and the effectors are shown at the top of the Figure. Mean *Photinus* luciferase (luc) activity was normalized with *Renilla* luciferase (Rluc). Asterisks denote statistically significant synergism. Rest of symbols as in Figure 17.

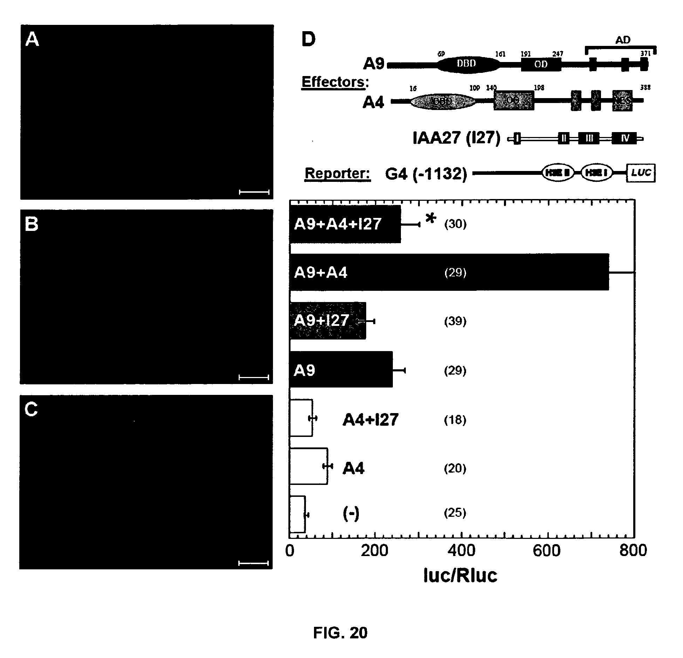

**Figure 20****. HalAA27 interacts with HaHSFA4, it hinders the interaction between HaHSFA4 and HaHSFA9, and it represses the synergistic coactivation by these HSFs in sunflower embryos. (A)** BiFC interaction between HaHSFA4 and HalAA27 in 15 dpa sunflower embryos bombarded with the HaIAA27:YFP^{N} and HaHSFA4:YFP^{C} fusion proteins. Confocal microscopy images of embryo samples bombarded with constructs that encode the following fusion proteins: **(B)** HaHSFA4:YFP^{N}+HaHSFA9:YFP^{C}. (C) HaHSFA4:YFP^{N}+HaHSFA9:YFP^{C} + HalAA27 mllab (anon-fluorescent, stabilized form of HaIAA27). Scale bars 30 µm. (D) HalAA27 fully abolished the synergistic interaction between HaHSFA4 and HaHSFA9. The embryos were bombarded with the *-1132(G4):LUC*[G4 (-1132)], reporter gene and the indicated combination of effector plasmid(s): none (-), HaHSFA4 (A4), HaHSFA4 + HalAA27 (A4 +I27), HaHSFA9 (A9), HaHSFA9 + HalAA27 (A9+I27), HaHSFA9+HaHSFA4 (A9+A4), and HaHSFA9+HaHSFA4+HaIAA27 (A9+A4+ 127). The different bar shading denotes similar Luc/Rluc activities. The asterisk indicates that the repression of the synergism was statistically significant. Rest of symbols as in Figures 17 and 19.
**Figure 21****. The combined overexpression of HaHSFA9 and HaHSFA4 in tobacco seeds enhances seed longevity beyond what achieved using only HaHSFA9.** Seed longevity was analyzed by resistance to accelerated aging treatments for 4 h at 52°C. Percent of germination observed at different times after the aging treatments were compared between seeds of double homozygous DS10:A9/A4 (A9/A4) and sibling, single homozygous DS10:A9 (A9) lines. The data correspond to 3 independent experiments. Representative pictures of seedlings taken 15 days after the aging treatment are shown to the right: one of the A9/A4 lines is compared with its sibling A9 line. Scale bars, 1 cm.

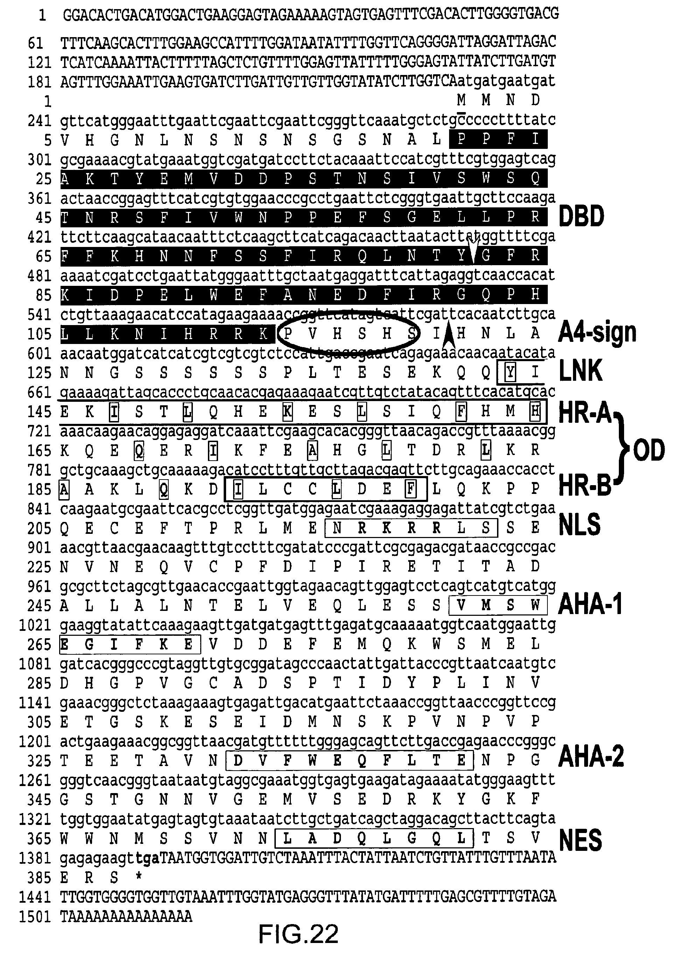

**Figure 22****. Full-length cDNA sequence (Accession# HF549285) and characteristics of the predicted HaHSFA4 protein.** Below the nucleotide sequence of *HaHSFA4,* we show the predicted amino acid sequence. An asterisk marks the stop codon. The red arrow indicates the expected position for the conserved intron present in the genomic class A HSF sequences. The black arrow marks the 5'-end of the c-DNA initially cloned by yeast two-hybrid. To the right, putative domains and functional sequences in the HaHSFA4 protein that were identified based on sequence comparisons with similar HSFs, are indicated. The DNA-binding domain (DBD) is outlined on a black background. Linker (LNK) sequences connect the DBD and the oligomerization domain (OD). These sequences include the HSF Class A4 signature sequences (A4-sign) that are marked with a red oval. The overlapping heptad repeats (HR-A, HR-B, boxed in black) of hydrophobic amino acids that conform the OD are indicated, with the hydrophobic residues outlined on a blue background. The amino acid residues of the putative nuclear localization sequences (NLS) and nuclear export sequences (NES) are outlined on yellow or red backgrounds, respectively. The two AHA motifs are indicated on a green background.

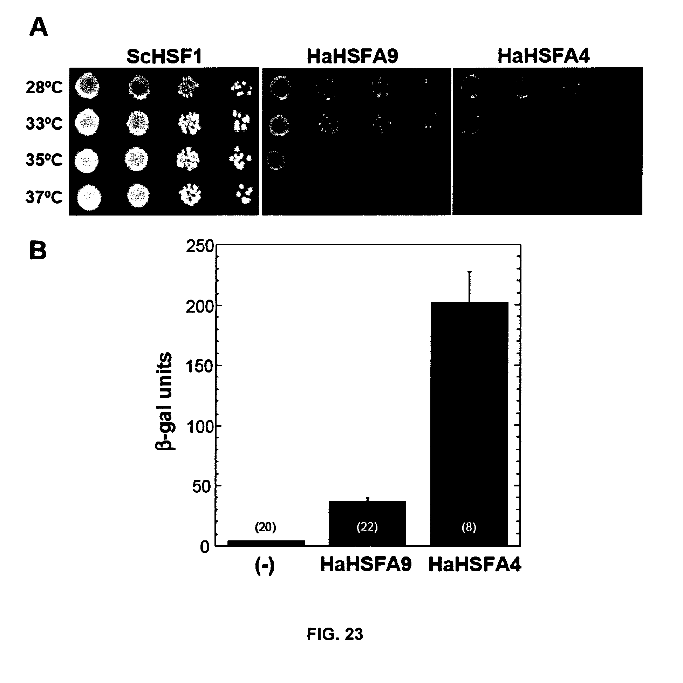

**Figure 23****. HaHSFA4 functions in yeast cells. (A)** similar functional replacement of the yeast *(Saccharomyces cerevisiae*) ScHSF1 by HaHSFA9 or HaHSFA4.The sunflower HSFs were cloned in plasmid pADΔ5 and assayed essentially as described (Almoguera *et al.,* 2002). Aliquots (2.5µl) of the indicated RSY4 yeast strain were spotted on YPD, starting from mid-logphase cultures, at decreasing cell density (1:10 dilutions steps, from left to right). Plates were incubated at the temperatures shown on the left and photographed after 2 days of growth. (B) Transcriptional activation assays in yeast using ß-galactosidase reporter plasmids containing the Gal7p promoter in the strain PJ69-4A.The sunflower HSFs were fused to the GAL4BD in pGBT9-derived plasmids. Numbers in parentheses show the number of replicates for each strain/HSF combination.

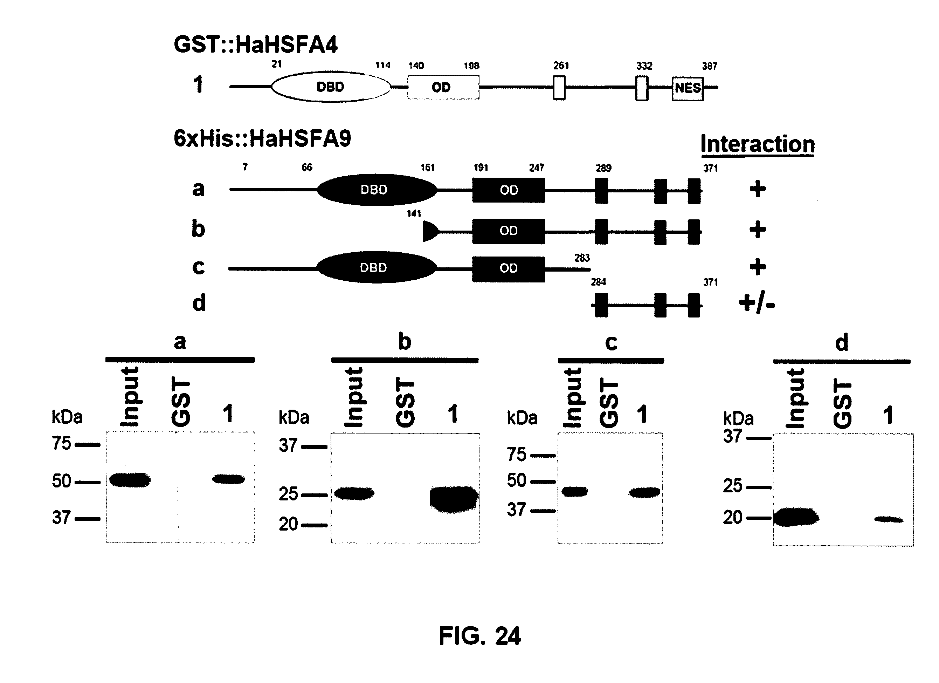

**Figure 24****. GST pull-down interaction between HaHSFA9 and HaHSFA4.A, GST and the GST:HaHSFA4 fusion protein(1) in plasmid pGEX4T-1 (Amersham Biosiences) were expressed in bacteria and bound to glutathione affinity beads. (A)** Beads were incubated with bacterial extracts containing different 6xHis:HaHSFA9 fusion proteins; a-d, which correspond to the I-IV proteins used by Díaz-Martín *et al.,* (2005). (B) The interacting proteins were eluted, and then detected by western-blot using anti-Xpress antibodies.

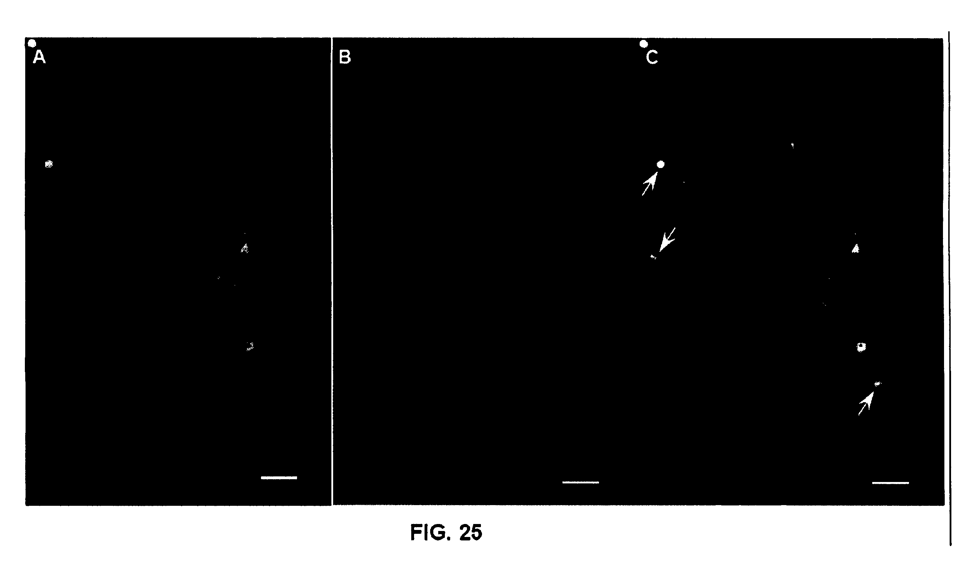

**Figure 25****. Confocal localization and co-localization of HaHSFA9 and HaHSFA4 in leaves of *Nicotiana benthamiana.*** The leaves were infiltrated with *Agrobacterium* strains that expressed two fluorescent fusion proteins: EGFP:HaHSFA4 and DsRed2:HaHSFA9. The plasmid with the EGFP:HaHSFA4 fusion is described in Material and Methods. The DsRed2:HaHSFA9 fusion was constructed in the pSAT6-DsRed2-C1 plasmid and transferred to pRCS2 (Tzfira *et al.,* 2005). **(A)** EGFP:HaHSFA4 localization (green fluorescence). **(B),** DsRed2:HaHSFA9 localization (red fluorescence). C, for co-localization, the images from the red and green channels were superimposed (EGFP:HaHSFA4+DsRed2:HaHSFA9). Arrows mark the "orange" nuclei. Scale bars, 50 µm. Disc sections were analyzed 3 days after infiltration. A confocal laser-scanning Olympus FV1000 microscope, with a UPLSAPO 20x NA:0.75 objective, was employed. Standard GFP and DsRed2 filter settings were sequentially used for image acquisition.

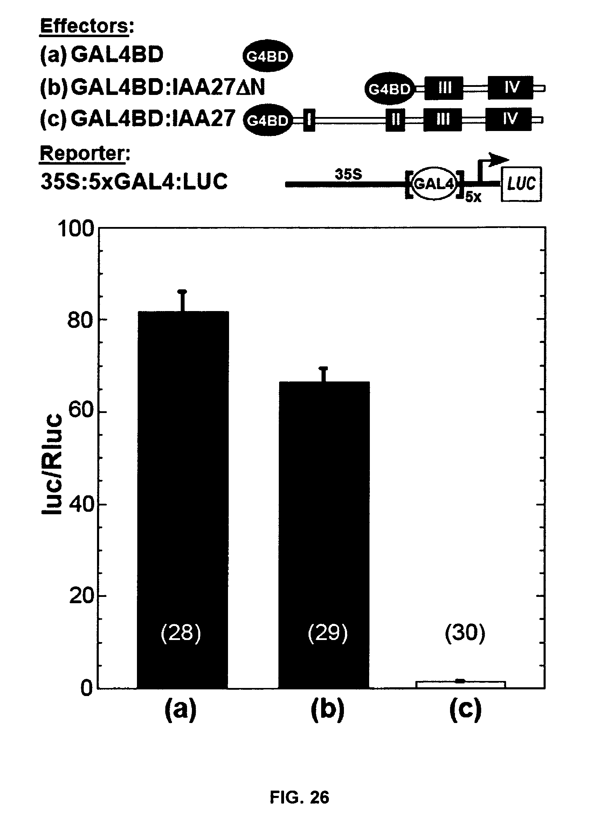

Figure **26****. Mapping active repression domains in HaIAA27.** The 35S-GAL4-TATA-LUC-NOS reporter plasmid (Hiratsu *et al.,* 2004, 35S:5xGAL4:LUC), together with effector plasmids expressing the GAL4 binding domain (GAL4BD), or GAL4BD fused in frame with the HalAA27 or the HaIAA27ΔN proteins (respectively, GAL4DB:IAA27 and GAL4DB:HaIAA27ΔN) were bombarded to 15 dpa sunflower embryos. The amounts of plasmid DNA per precipitate (5 shots) were: 2.5 µg of the reference plasmid, 5 µg of the reporter plasmid and 5 µg of the effector plasmids. Construct maps on top of the Figure. The effector plasmids derive from pBI524-GAL4DB (Després *et al.,* 2003).

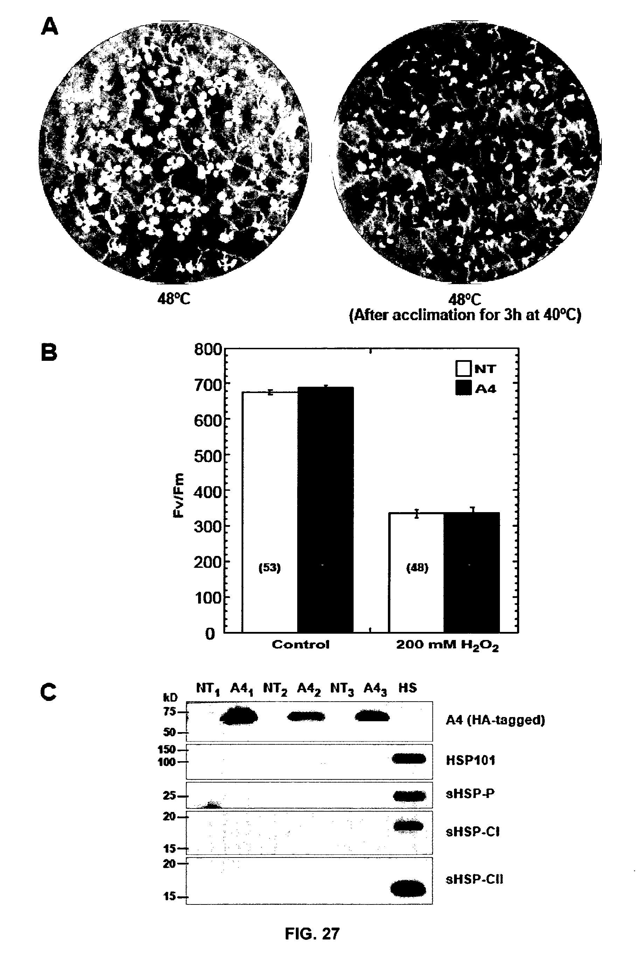

**Figure 27****.** Absence of stress resistance and lack of HSP accumulation (at normal temperatures) in the 35S:A4 seedlings.

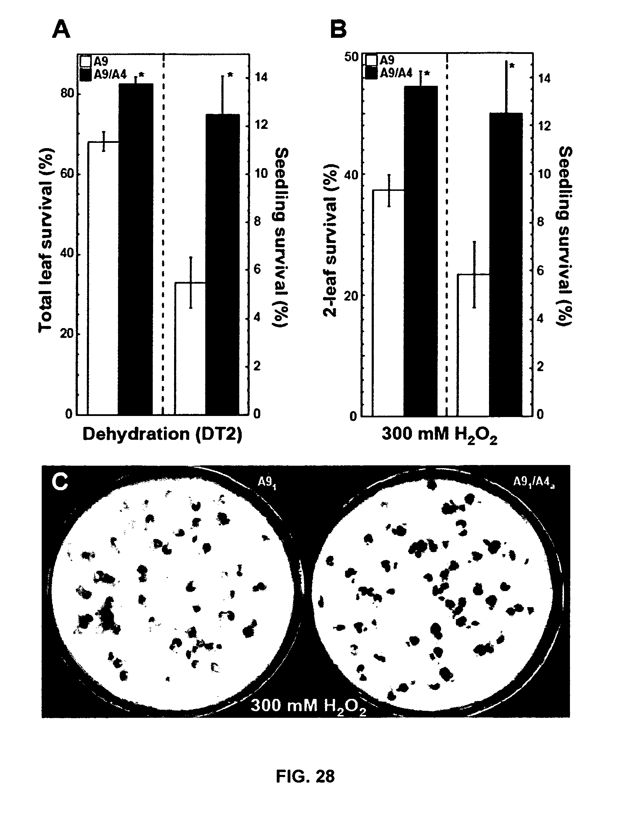

**Figure 28****.** Enhanced drastic dehydration- and oxidative-stress resistance in seedlings that combine overexpression of A9 and A4 (35S:A9/A4).

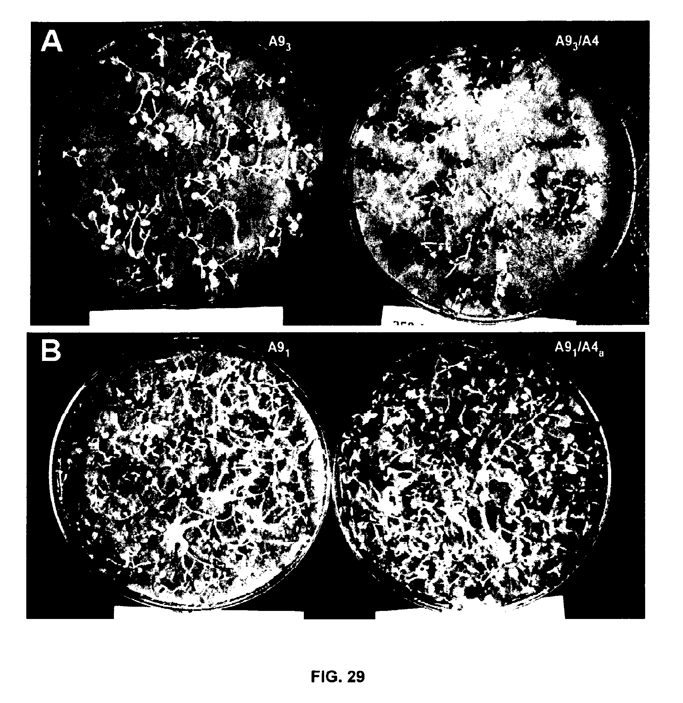

**Figure 29****.** Enhanced recovery from dark-induced senescence in seedlings that combine overexpression of A9 and A4 (35S:A9/A4).

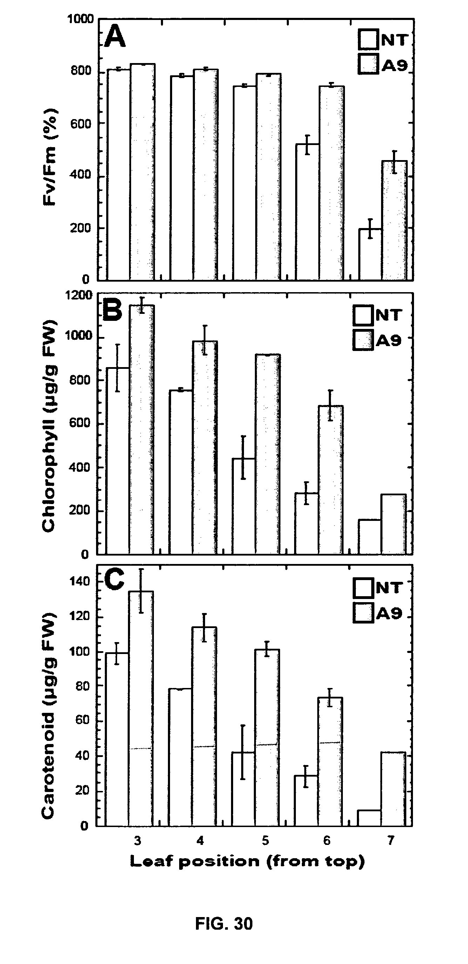

**Figure 30****.** Delayed senescence of well-watered 35S:A9 plants after 7 weeks cultureunder photoperiod illumination.

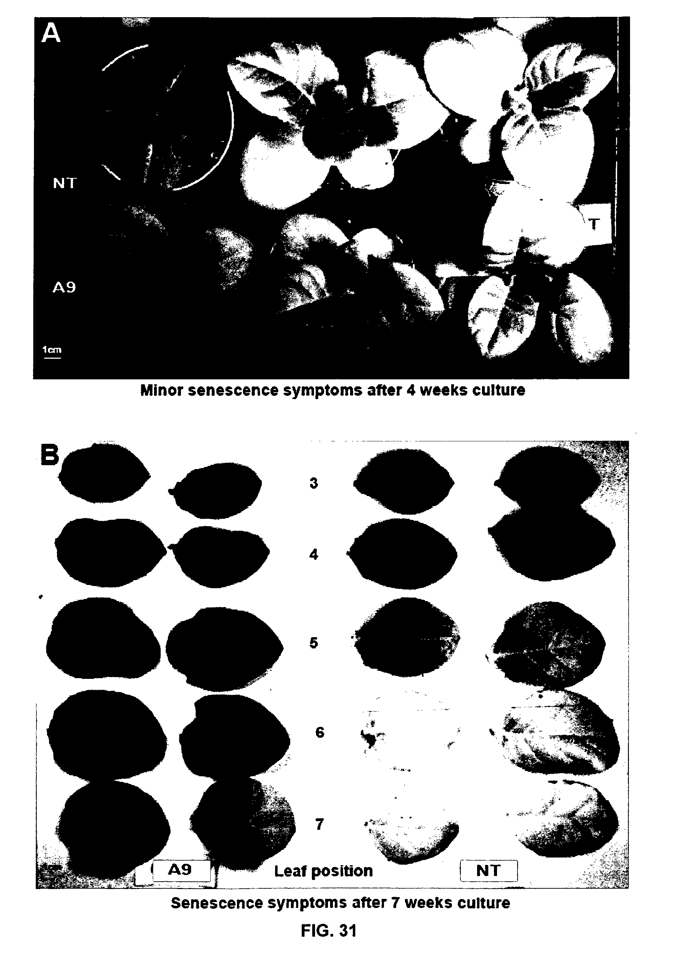

**Figure 31****.** Delayed leaf senescence of well-watered 35S:A9 plants after 7 weeks culture under photoperiod illumination.

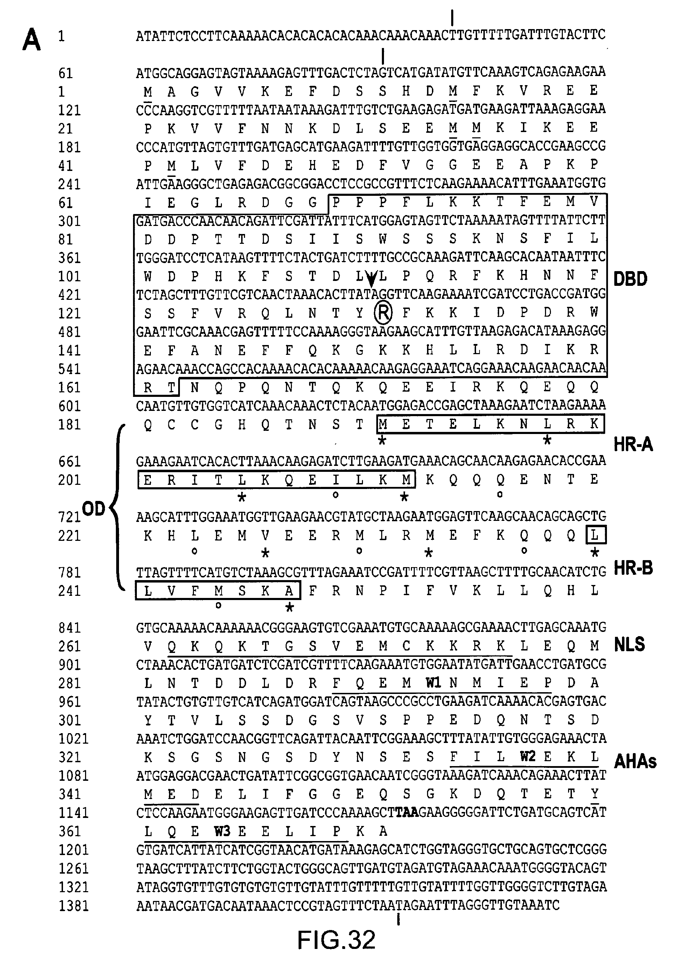

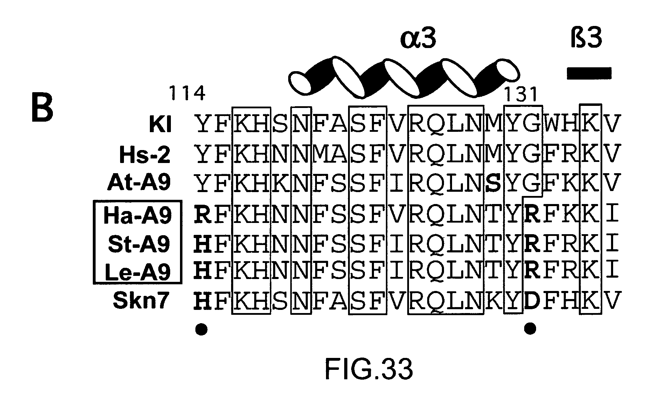

**Figure 32****. (A) -** Alignment of HSFA9 from different plants. Full-length cDNA sequence and predicted HaHSFA9 protein. We assembled the nucleotide sequences of two cDNA clones. These cDNAs showed incomplete 5'-UTRs with ends indicated by the vertical marks. Nucleotides 1-40 were added by RLM-RACE cloning. The arrow indicates the expected position for the conserved intron present in the genomic sequences. Below the nucleotide sequence, we show the conceptual translation of the HaHSFA9 protein. The stop codon is in boldface. We also indicate putative domains identified by sequence comparison with other HSFs: the DBD including the exceptional amino acid substitution (R, outlined). The oligomerization domain (OD), with overlapping heptad repeats (HR-A/B) indicated by small circles and asterisks; a putative bipartite nuclear localization sequence (NLS); and finally three putative AHA motifs (underlined) containing tryptophan residues (w1-w3). (B) - Alignment of DBDHaHSFA9 (Ha-A9) sequences (positions 114-135) in and near the DNA-recognition helix (α3). Predicted secondary structure (in the scheme on top) is based in the solved *Kluyveromyces lactis* (Kl) HSF-DBD structure. Other HSF sequences include those from *Homo sapiens* HSF2 (Hs-2), *Arabidopsis thaliana* AtHSFA9 (At-A9), and *Solanum tuberosum* (St-A9) and *Lycopersicon esculentum* (Le-A9) HSFA9. Invariant amino acid residues are boxed. The exceptional arginine residue, at position 131, in Ha-A9, St-A9 and Le-A9 is shown in bold face, as the aspartic residue that replaces glycine in the *Saccharomyces cerevisiae* protein Skn7. We similarly indicate the peculiar substitution of a conserved non-charged residue, at position 114, for a positively charged residue (H or R) in Skn7, St-A9 and LeA9. Dots at the bottom highlight the positions with these amino acid substitutions, which are numbered after the HaHSFA9 sequence.

### DETAILED DESCRIPTION OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The practice of the present invention will employ, unless otherwise indicated, conventional techniques of botany, microbiology, tissue culture, molecular biology, chemistry, biochemistry and recombinant DNA technology, bioinformatics which are within the skill of the art. Such techniques are explained fully in the literature.

In embodiments of the various aspects of the invention, embodiments that do not use recombinant technology and rely solely on traditional breeding methods are specifically excluded.

As used herein, the words "nucleic acid", "nucleic acid sequence", "nucleotide", "nucleic acid molecule" or "polynucleotide" are intended to include DNA molecules (e.g., cDNA or genomic DNA), RNA molecules (e.g., mRNA), natural occurring, mutated, synthetic DNA or RNA molecules, and analogs of the DNA or RNA generated using nucleotide analogs. It can be single-stranded or double-stranded. Such nucleic acids or polynucleotides include, but are not limited to, coding sequences of structural genes, antisense sequences, and non-coding regulatory sequences that do not encode mRNAs or protein products. These terms also encompass a gene. The term "gene" or "gene sequence" is used broadly to refer to a DNA nucleic acid associated with a biological function. Thus, genes may include introns and exons as in genomic sequence, or may comprise only a coding sequence as in cDNAs, and/or may include cDNAs in combination with regulatory sequences.

The various embodiments describe nucleic acids comprising certain SEQ ID Nos. It will be understood that unless otherwise specified, within the scope of the invention are also embodiments with nucleic acids consisting of these SEQ ID Nos.

The terms "polypeptide" and "protein" are used interchangeably herein and refer to amino acids in a polymeric form of any length, linked together by peptide bonds.

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either:
(a) the nucleic acid sequences encoding proteins useful in the methods of the invention, or
(b) genetic control sequence(s) which is operably linked with the nucleic acid sequence according to the invention, for example a promoter, or
(c) a) and b),
are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815 both incorporated by reference.

The term plant as used in all of the various aspects of the invention as described herein, including the various methods for generating transgenic plants with enhanced traits expressing HSFA9 or HSFA9 in combination with HSFA4, may be a moncot or a dicot plant.

In one embodiment, the plant is a dicot plant. A dicot plant may be selected from the families including, but not limited to *Asteraceae, Brassicaceae (eg Brassica napus), Chenopodiaceae, Cucurbitaceae, Leguminosae (Caesalpiniaceae, Aesalpiniaceae Mimosaceae, Papilionaceae or Fabaceae), Malvaceae, Rosaceae or Solanaceae.* For example, the plant may be selected from lettuce, sunflower, *Arabidopsis,* broccoli, spinach, water melon, squash, cabbage, tomato, potato, yam, capsicum, tobacco, cotton, okra, apple, rose, strawberry, alfalfa, bean, soybean, field (fava) bean, pea, lentil, peanut, chickpea, apricots, pears, peach, grape vine or citrus species. In one embodiment, the plant is oilseed rape.

Also included are biofuel and bioenergy crops such as rape/canola, sugar cane, sweet sorghum, Panicum virgatum (switchgrass), linseed, lupin and willow, poplar, poplar hybrids, Miscanthus or gymnosperms, such as loblolly pine. Also included are crops for silage (maize), grazing or fodder (grasses, clover, sanfoin, alfalfa), fibres (e.g. cotton, flax), building materials (e.g. pine, oak), pulping (e.g. poplar), feeder stocks for the chemical industry (e.g. high erucic acid oil seed rape, linseed) and for amenity purposes (e.g. turf grasses for golf courses), ornamentals for public and private gardens (e.g. snapdragon, petunia, roses, geranium, Nicotiana sp.) and plants and cut flowers for the home (African violets, Begonias, chrysanthemums, geraniums, Coleus spider plants, Dracaena, rubber plant).

In one embodiment, the plant is a monocot plant. A monocot plant may, for example, be selected from the families *Arecaceae, Amaryllidaceae* or *Poaceae.* For example, the plant may be a cereal crop, such as wheat, rice, barley, maize, oat, sorghum, rye, millet, buckwheat, turf grass, Italian rye grass, sugarcane or Festuca species, or a crop such as onion, leek, yam or banana.

Preferably, the plant is a crop plant. By crop plant is meant any plant which is grown on a commercial scale for human or animal consumption or use.

Most preferred plants are maize, wheat, rice, oilseed rape, sorghum, soybean, potato, tomato, grape, barley, pea, bean, field bean, lettuce, cotton, sugar cane, sugar beet, broccoli or other vegetable brassicas or poplar.

In one embodiment, the plant is a dicot crop plant.

In another embodiment, a nucleic acid comprising SEQ ID. No 1 or a functional variant or homolog thereof in a monocot plant as described herein is expressed in a dicot plant. In one embodiment, a nucleic acid comprising SEQ ID. No. 1 is expressed in a dicot plant.

In another embodiment, a nucleic acid comprising a functional variant or homolog of SEQ ID. No 1 from a dicot plant is expressed in a monocot plant.

Unless otherwise specified, the term "plant" as used herein encompasses whole plants, ancestors and progeny of the plants and plant parts, including seeds, fruit, shoots, stems, leaves, roots (including tubers), flowers, and tissues and organs, wherein each of the aforementioned comprise the gene/nucleic acid of interest unless otherwise specified. The term "plant" also encompasses plant cells, suspension cultures, callus tissue, embryos, meristematic regions, gametophytes, sporophytes, pollen and microspores, again wherein each of the aforementioned comprises the gene/nucleic acid of interest unless otherwise specified.

In some aspects of the invention as described herein, including the various methods for generating transgenic plants with enhanced traits expressing HSFA9 or HSFA9 in combination with HSFA4, the term plant explicitly excludes seeds and refers to any plant tissue or a plant part that is not a seed, including a whole germinated plant at any stage of development beyond the seed stage.

In some aspects of the invention as described herein, including the various methods for generating transgenic plants with enhanced traits expressing HSFA9 or HSFA9 in combination with HSFA4, the term plant explicitly excludes vegetative tissue.

The term vegetative tissue of a plant refers to tissue that is not reproductive. This term explicitly excludes a plant seed. Reproductive tissue includes flowers, stamens, gametophyte, sporophytes, pollen and microspores carpels, endosperm and the plant embryo (seed).

The terms "increase", "improve" or "enhance" as used according to the various aspects of the invention are interchangeable. An increase in a specific trait is compared to a control plant and is at least a 3%, 4%, 5%, 6%, 7%, 8%, 9% or 10%, preferably at least 15% or 20%, more preferably 25%, 30%, 35%, 40% or 50%. The term "delay" according to the various as aspects of the invention designates a delay in a trait compared to a control plant, for example by 1 to about 30 days or 1 to 6 months.

The inventors have found that overexpression of the seed-specific transcription factor HSFA9 from sunflower (HaHSFA9) is sufficient to confer delayed senescence to germinated tissue of transgenic tobacco outside the developing seed tissue, that is in a germinated plant.

The nucleic acid of HaHSFA9 is defined in SEQ ID No. 1. The amino acid of the peptide encoded by SEQ ID No.1 is defined in SEQ ID No. 2.

Therefore, in a first aspect, the invention relates to a method for delaying senescence of a plant or part thereof said method comprising introducing and expressing a nucleic acid comprising or consisting of SEQ ID No. 1 or a functional homolog, variant or part thereof in a plant wherein said plant or part thereof is not a seed.

Thus, this aspect explicitly excludes seeds and refers to any plant tissue or plant part that is not a seed or seed tissue. In other words, the method refers to plant tissue that is a germinated plant or seedling at any stage of development beyond the seed stage or any part or tissue of a germinated plant or seedling.

In one embodiment of this method, the plant or part thereof is vegetative tissue.

According to the various aspects of the invention, a nucleic acid comprising or consisting of SEQ ID No. 1 can be expressed in a plant. Preferred plants are listed elsewhere herein and include crop plants, such as maize, wheat, rice, oilseed rape, sorghum, soybean, potato, tomato, grape, barley, pea, bean, field bean, lettuce, cotton, sugar cane, sugar beet, broccoli or other vegetable brassicas or poplar. A skilled person would also understand that the various aspects of the invention are not limited to using a nucleic acid comprising SEQ ID No. 1. According to the various aspects of the invention, a functional homolog, functional variant of SEQ ID No. 1 or part of SEQ ID No. 1 may also be used provided that said functional homolog, part or variant of SEQ ID No. 1 is biologically active in a plant in the same way as SEQ ID No. 1.

The term "functional part or functional variant of a nucleic acid sequence" as used herein with reference to SEQ ID No. 1-4 refers to a variant gene sequence or part of the gene sequence which retains the biological function of the full non-variant sequence, for example confers delayed senescence when expressed in a transgenic plant. A functional variant also comprises a variant of the gene of interest which has sequence alterations that do not affect function, for example in non- conserved residues. Also encompassed is a variant that is substantially identical, i.e. has only some sequence variations, for example in non- conserved residues, to the wild type sequences as shown herein and is biologically active. A functional variant may a chimeric construct as described herein.

Thus, it is understood, as those skilled in the art will appreciate, that the aspects of the invention, including the methods and uses, encompasses not only a nucleic acid sequence comprising or consisting or SEQ ID No. 1, but also functional variants of SEQ ID No. 1 that do not affect the biological activity and function of the resulting protein. Alterations in a nucleic acid sequence which result in the production of a different amino acid at a given site that do however not affect the functional properties of the encoded polypeptide, are well known in the art. For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the polypeptide molecule would also not be expected to alter the activity of the polypeptide. Each of the proposed modifications is well within the routine skill in the art, as is determination of retention of biological activity of the encoded products.

A functional homolog of SEQ ID No. 1 is a nucleic acid encoding a HSFA9 peptide which is biologically active in the same way as SEQ ID No. 1, in other words, for example it confers delayed senescence when expressed in a plant. The term functional homolog includes HaHSFA9 orthologues in other plant species.

For example, according to the various aspects of the invention, a nucleic acid encoding an endogenous HSFA9 peptide may be expressed in a plant by recombinant methods.

For example, a tomato HSFA9 may be expressed in tomato, a wheat HSFA9 may be expressed in wheat, a rice HSFA9 may be expressed in rice. In another embodiment, a nucleic acid encoding a plant HSFA9 that is endogenous to another plant species may be expressed in a plant. Plants and their endogenous HSFA9 may be selected from any plant, such as from one of the families or species listed herein.

Preferably, the functional homologue or orthologue is a class A homologue or orthologue.

According to the various aspects of the invention, a functional homologue or orthologue of HaHSFA9 as defined in SEQ ID No. 2 has, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID No: 2. Preferably, overall sequence identity is at least 90%, 91%, 92%, 93%, 94%, 95%, 96%, 97%, 98%, or 99%. In another embodiment, the HSFA9 homolog or orthologue nucleic acid sequence has, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the nucleic acid represented by SEQ ID NO: 1. Preferably, overall sequence identity is at least 90%, 91%, 92%, 93%, 94%, 95%, 96%, 97%, 98%, or 99%.

HSFA9 belongs to Class A, a family of plant HSFs with 15 members in Arabidopsis and with a similar family size in other plants such as rice, tomato, soybean and alfalfa (Nover et al. 2001). HaHSFA9 contains a DBD, Class A OD with HR-AB, NLS and AHA motifs in the AD as HaHSFA4. It does not contain NES and A4-signature sequences (see figure 32). Thus, preferred homologues and orthologues comprise a DBD, Class A OD with HR-AB, NLS and AHA motifs in the AD.

Sequences for these domains are shown below:
SEQ ID No. 5 (DBD of HaHSFA9): 
P P P F L K K T F E M V D D P T T D S I I S W S S S K N S F I L W D P H K
F S T D L L P Q R F K H N N F S S F V R Q L N T Y R F K K I D P D R W E
F A N E F F Q K G K K H L L R D I K R R T
Motifs in the OD of HaHSFA9
SEQ ID No. 6 HR-A
   METELKNLRKERITLKQEILKM
SEQ ID No. 7 HR-B
   LLVFMSKA
SEQ ID No. 8 bipartite NLS
   Q K Q K T G S V E M C K K R K X₁₋₁₂ F Q E M W1 N M I E P
Wherein X is any amino acid
SEQ ID No. 9 AHA1
   F I L W E K L M E D
SEQ ID No. 10 AHA2
   LQEWEELIP

Thus, according to the different aspects of the invention, a HSFA9 peptide comprises a DBD as defined in SEQ ID. No. 5 or which has at least 90%, 91%, 92%, 93%, 94%, 95%, 96%, 97%, 98% or 99% homology SEQ ID. No. 5, a OD domain with the elements as defined in SEQ ID. No. and 7 or which has at least 90%, 91%, 92%, 93%, 94%, 95%, 96%, 97%, 98% or 99% homology to SEQ ID. No. 6 and 7, a NLS as defined in SEQ ID. No. 78 or which has at least 90%, 91%, 92%, 93%, 94%, 95%, 96%, 97%, 98% or 99% homology to SEQ ID. No. 8, an AHA1 domain as defined in SEQ ID. No. 9 or which has at least 90%, 91 %, 92%, 93%, 94%, 95%, 96%, 97%, 98% or 99% homology to SEQ ID. No. 9, an AHA2 domain as defined in SEQ ID. No.10 or which has at least 90%, 91%, 92%, 93%, 94%, 95%, 96%, 97%, 98% or 99% homology to SEQ ID. No. 10.

Sequences C-terminal (CT) of the OD include the activation domain (AD) of Class A HSFs. In HaHSFA9, these CTAD sequences comprise 124 amino acids, from positions 248 to 371 (SEQ ID NO. 18-21).

Thus, in addition to the motifs listed above, a HSFA9 peptide comprises a DBD as defined in SEQ ID. No.18 or which has at least 90%, 91%, 92%, 93%, 94%, 95%, 96%, 97%, 98% or 99% homology to SEQ ID. No. 5. Preferably, SEQ ID. No. 18 comprises SEQ ID No. 19, 20 and 21.

Preferred orthologues of HaHSFA9 are HSFs9 from crop plants, such as maize, wheat, rice, oilseed rape, sorghum, soybean, potato, tomato, grape, barley, pea, bean, field bean, lettuce, cotton, sugar cane, sugar beet, broccoli or other vegetable brassicas or poplar.

The overall sequence identity is determined using a global alignment algorithm known in the art, such as the Needleman Wunsch algorithm in the program GAP (GCG Wisconsin Package, Accelrys).

Suitable homologues or orthologues can be identified by sequence comparisons and identifications of conserved domains. The function of the homologue or orthologue can be identified as described herein and a skilled person would thus be able to confirm the function when expressed in a plant.

In another aspect, the invention relates to the use of a nucleic acid comprising SEQ ID No. 1, a functional homolog, variant or part thereof for delaying senescence of a plant or part thereof wherein said plant or part thereof is not a seed. In one embodiment, the plant or part thereof is vegetative tissue.

In another aspect, the invention relates to a method for generating a transgenic plant with delayed senescence comprising introducing and expressing a nucleic acid comprising SEQ ID No. 1, a functional homolog, variant or part thereof in a plant wherein said plant or part thereof is not a seed. In other words, the method relates to a method for generating a transgenic plant with delayed senescence in non-seed tissue as described above.

The method further comprises regenerating a transgenic plant from the plant or plant cell that expresses a nucleic acid comprising SEQ ID No. 1, a functional homolog, variant or part thereof in a plant wherein said plant or part thereof is not a seed wherein the transgenic plant comprises in its genome SEQ ID No. 1 or a functional homolog, variant or part thereof operably linked to a regulatory sequence and obtaining a progeny plant derived from the transgenic plant or plant cell wherein said progeny plant exhibits delayed senescence.

The improved traits according to the various aspects of the invention are compared to a control plant. For example, delayed senescence is delayed compared to senescence in a control plant. A control plant is a plant which has not been transformed with a nucleic acid construct comprising SEQ ID No. 1, a functional homolog, variant or part thereof, preferably a wild type plant. The control plant is preferably of the same species. Furthermore, the control plant may comprise genetic modifications, including expression of transgenes other than HSFA9.

Delayed senescence can be measured as shown in the examples by measuring chlorophyll content in leaves, but other molecular markers can be used for measurement as known in the art (Buchanan-Wollaston *et al.,* 2003).

The reduction in chlorophyll content of a transgenic plant is less than in a control plant. In other words, transgenic plants retain significantly more chlorophyll during the senescence process than control plants. When compared to a control plant, transgenic plants retain at least 5-50% more chlorophyll, for example at least 5%, 10%, 15% or 20%.

A transgenic plant for the purposes of the various aspects of the invention is understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the different embodiments of the invention are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place.

According to the various aspects of the invention, a nucleic acid comprising SEQ ID No. 1 or a functional homolog, variant or part may be operably linked to a regulatory sequence. Thus, a nucleic acid comprising SEQ ID No. 1 or a functional homolog, variant or part may be part of a nucleic acid construct, for example a vector.

The terms "regulatory sequence", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or microorganisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern. The term "operably linked" as used herein refers to a functional linkage between the promoter sequence and the gene of interest, such that the promoter sequence is able to initiate transcription of the gene of interest.

A "constitutive promoter" refers to a promoter that is transcriptionally active during most, but not necessarily all, phases of growth and development and under most environmental conditions, in at least one cell, tissue or organ. Examples of constitutive promoters include but are not limited to actin, HMGP, CaMV19S, GOS2, rice cyclophilin, maize H3 histone, alfalfa H3 histone, 34S FMV, rubisco small subunit, OCS, SAD1, SAD2, nos, V-ATPase, super promoter, G-box proteins and synthetic promoters.

A "strong promoter" refers to a promoter leads to increased or overexpression of the gene. Examples of constitutive promoters include, but are not limited to, CaMV-35S, CaMV-35Somega, Arabidopsis ubiquitin UBQ1, rice ubiquitin, actin, or Maize alcohol dehydrogenase 1 promoter (Adh-1).

In a preferred embodiment, the promoter is a constitutive promoters that is a strong promoter and directs overexpression of the gene. Preferred promoters are CaMV-35S, CaMV-35Somega, Arabidopsis ubiquitin UBQ1.

The term "increased expression" or "overexpression" as used herein means any form of expression that is additional to the control, for example wild-type, expression level.

The nucleic acid construct may also comprise a selectable marker which facilitates the selection of transformants, such as a marker that confers resistance to antibiotics, for example kanamycin.

Thus, according to the various aspects of the invention, SEQ ID No. 1 is introduced into a plant and expressed as a transgene. The nucleic acid sequence is introduced into said plant through a process called transformation. The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art. In some aspects of the invention, embryos are excluded.

The transfer of foreign genes into the genome of a plant is called transformation. Transformation of plants is now a routine technique in many species. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts, electroporation of protoplasts, microinjection into plant material, DNA or RNA-coated particle bombardment, infection with (non-integrative) viruses and the like. Transgenic plants, including transgenic crop plants, are preferably produced via *Agrobacterium tumefaciens* mediated transformation.

To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The inventors have also shown that overexpression of the seed specific transcription factor HSFA9 from sunflower (HaHSFA9) is sufficient to confer increased tolerance to oxidative stress to non-seed tissue of transgenic tobacco, that is to a plant outside the developing seed context.

Thus, in another aspect, the invention relates to a method for improving tolerance to oxidative stress in a plant or part thereof said method comprising introducing and expressing a nucleic acid comprising SEQ ID No. 1, a functional homolog, variant or part thereof or a functional homolog thereof in a plant wherein said plant or part thereof is not a seed.

Thus, this aspect explicitly excludes seeds and refers to any plant tissue or plant part that is not a seed or seed tissue. In other words, the method refers to plant tissue that is a germinated plant or seedling at any stage of development beyond the seed stage or any part or tissue of a germinated plant or seedling.

In one embodiment of the various aspects of the invention, the plant or part thereof is vegetative tissue.

In another aspect, the invention relates to the use of a nucleic acid comprising SEQ ID No. 1, a functional homolog, variant or part thereof for improving tolerance to oxidative stress in a plant or part thereof wherein said plant or part thereof is not a seed. In one, the plant or part thereof is vegetative tissue.

In another aspect, the invention relates to generating a transgenic plant with improved tolerance to oxidative stress comprising introducing and expressing a nucleic acid comprising SEQ ID No. 1, a functional homolog, variant or part thereof in a plant wherein said plant or part thereof is not a seed. In other words the method relates to generating a transgenic plant with improved tolerance to oxidative stress in non-seed tissue as described above. In one embodiment, the plant or part thereof is vegetative tissue.

The method further comprises regenerating a transgenic plant from the plant or plant cell after the step of introducing and expressing a nucleic acid comprising SEQ ID No. 1, a functional homolog, variant or part thereof in a plant wherein said plant or part thereof is not a seed wherein the transgenic plant comprises in its genome SEQ ID No. 1 or a functional homolog, variant or part thereof operably linked to a regulatory sequence and obtaining a progeny plant derived from the transgenic plant wherein said progeny plant exhibits delayed senescence.

Improved tolerance to oxidative stress in non-seed tissue can be measured as shown in example 1, for example by measuring degradation of the D1 protein. In transgenic plants, both the D1 protein and its plastidial synthesis are more resistant to oxidative stress than control plants.

The production and scavenging of chemically reactive species, such as ROS/RNS, are central to a broad range of biotic and abiotic stress and physiological responses in plants. Oxidative stress can be induced by various environmental and biological factors such as hyperoxia, light, drought, high salinity, cold, metal ions, pollutants, xenobiotics, toxins, reoxygenation after anoxia, experimental manipulations, pathogen infection and aging of plant organs.

Thus, the invention relates in particular to methods for increasing or enhancing plant response to oxidative stress as described herein, caused for example by UV light, irradiation, high salinity, cold, metal ions, pollutants, toxins, or pathogen infection by bacteria, viruses or fungi or a combination thereof.

Plants according to these aspects of the invention are described elsewhere. Most preferred plants are maize, wheat, rice, oilseed rape, sorghum, soybean, potato, tomato, grape, barley, pea, bean, field bean, lettuce, cotton, sugar cane, sugar beet, broccoli or other vegetable brassicas or poplar.

We have also shown that co-expression of HaHSFA9 and HaHSFA4 results in enhanced advantageous traits. Co-expression of HaHSFA9 and HaHSFA4 synergistically activates expression of target promoters. Moreover, nuclear retention of HSFA4 is enhanced. Also, co-expression of HaHSFA9 and HaHSFA4 results in beneficial effects in tissue of the plant outside the seed tissue and enhances the effect of delayed senescence compared to expression of HaHSFA9 alone. Moreover, co-expression of HaHSFA9 and HaHSFA4 also enhanced tolerance to oxidative stress in plants.

Therefore, in another aspect, the invention relates to a method for improving tolerance to oxidative stress and/or improving tolerance to dehydration in a plant said method comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant.

In one embodiment, the method relates to improving tolerance to oxidative stress in a plant, including seeds. In another embodiment, the method relates to improving tolerance to oxidative stress in a plant, excluding in seeds. For example, tolerance is improved in vegetative tissue of a plant.

In one embodiment, the method relates to improving tolerance to dehydration in a plant, including seeds. In another embodiment, the method relates to improving tolerance to dehydration in a plant, excluding in seeds. For example, tolerance is improved in vegetative tissue of a plant.

In another aspect, the invention relates to a method for delaying senescence of a plant or part thereof said method comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant wherein said plant or part thereof is not a seed.

Thus, this aspect explicitly excludes seeds and refers to any plant tissue or plant part that is not a seed or seed tissue. In other words, the method refers to plant tissue that is a germinated plant or seedling at any stage of development beyond the seed stage or any part or tissue of a germinated plant or seedling.

In one embodiment of the various aspects of the invention, the plant or part thereof is vegetative tissue.

In another aspect, the invention relates to a method for generating a transgenic plant with delayed senescence comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant wherein said plant is not a seed.

In another aspect, the invention relates to a method for generating a transgenic plant with improved tolerance to oxidative stress and/or improved tolerance to dehydration comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant.

The methods for generating a transgenic plant further comprises regenerating a transgenic plant from the plant or plant cell after the step described above wherein the transgenic plant comprises in its genome SEQ ID No. 1 or a functional homolog, variant or part thereof and SEQ ID. No. 3 or a functional homolog, variant or part thereof operably linked to a regulatory sequence and obtaining a progeny plant derived from the transgenic plant wherein said progeny plant exhibits delayed senescence, improved tolerance to oxidative stress and/or improved tolerance to dehydration.

The invention also relates to the use of a nucleic acid comprising SEQ ID No. 1, a functional homolog, variant or part thereof in combination with a nucleic acid comprising SEQ ID No. 3, a functional homolog, variant or part thereof for improving tolerance to oxidative stress and /or improving tolerance to dehydration in a plant. In one embodiment, the plant is not a seed.

The invention also relates to the use of a nucleic acid comprising SEQ ID No. 1, a functional homolog, variant or part thereof in combination with a nucleic acid comprising SEQ ID No. 3, a functional homolog, variant or part thereof for delaying senescence in a plant.

The invention also relates to the use of a nucleic acid comprising SEQ ID No. 1, a functional homolog, variant or part thereof in combination with a nucleic acid comprising SEQ ID No. 3, a functional homolog, variant or part thereof for delaying senescence of non-seed tissue of a plant. For example, the tissue is vegetative tissue.

The terms a nucleic acid comprising SEQ ID No. 1, a functional homolog, variant or part thereof according to the various aspects of the invention is described elsewhere. According to the invention, a chimeric construct that comprises the LNK, OD and AD domains of HSFA9 fused to N-terminal residues of a different HSF, for example HSFA2, such as Ls HSFA2, which comprise a DNA binding domain may also be used.

Preferably, the functional homologue or orthologue is a class A homologue or orthologue.

According to the various aspects of the invention that use HSFA9 and HSFA4 in combination, a functional homologue or orthologue of HaHSFA4 as defined in SEQ ID No. 3 (nucleic acid sequence) and SEQ ID No. 4 (peptide sequence) has, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by SEQ ID NO: 4. Preferably, overall sequence identity is at least 90%, 91%, 92%, 93%, 94%, 95%, 96%, 97%, 98%, or 99%. In another embodiment, the HaHSFA9 homolog or orthologue nucleic acid sequence has, in increasing order of preference, at least 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91 %, 92%, 93%, 94%, 95%, 96%, 97%, 98%, or 99% overall sequence identity to the nucleic acid represented by SEQ ID NO: 3. Preferably, overall sequence identity is at least 90%, 91%, 92%, 93%, 94%, 95%, 96%, 97%, 98%, or 99%.

The overall sequence identity is determined using a global alignment algorithm known in the art, such as the Needleman Wunsch algorithm in the program GAP (GCG Wisconsin Package, Accelrys).

Suitable homologues or orthologues can be identified by sequence comparisons and identifications of conserved domains. The function of the homologue or orthologue can be identified as described herein and a skilled person would thus be able to confirm the function when expressed in a in plants.

The term functional homolog includes HaHSFA4 orthologues in other plant species. For example, according to the various aspects of the invention a nucleic acid encoding an endogenous HSFA4 peptide may be expressed in a plant by recombinant methods. Thus, endogenous HSFA4 and HSF9 peptide may be co-expressed in a plant by recombinant methods.

In another embodiment, exogenous HSFA4 and HSF9 peptide may be co-expressed in a plant by recombinant methods. In another embodiment, endogenous HSFA4 and exogenous HSF9 may be co-expressed in a plant by recombinant methods. In yet another embodiment, endogenous HSFA9 and exogenous HSF4 may be co-expressed in a plant by recombinant methods.

Preferred plants are listed elsewhere herein and include crop plants, such as maize, wheat, rice, oilseed rape, sorghum, soybean, potato, tomato, grape, barley, pea, bean, field bean, lettuce, cotton, sugar cane, sugar beet, broccoli or other vegetable brassicas or poplar.

In another aspect, the invention relates to increasing nuclear retention of HSFA4 in a plant said method comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant. In one embodiment, the plant is not a seed.

In another aspect, the invention relates to methods for increasing the effects of expressing HSFA9 in a transgenic plant said method comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant. In one embodiment, the plant is not a seed.

As shown herein and according to the methods and uses described above, co-expression of HSFA9 and HSFA4 significantly enhances the effects observed when HSFA9 alone is expressed. In one embodiment of the methods of the invention, the combination acts synergistically and the effects observed are greater than the sums of expression of HSFA9 and HSFA4.

Among the different groups of class A HSFs in plants, HaHSFA4 clearly belongs to the A4 family. The A4 HSFs are characterized -among other properties- by the presence of conserved signature sequences (PVHSHS) located immediately after the DBD, and by the presence of nuclear export sequences (NES motif) in the carboxyl-terminal region (see for example Friedberg *et al.,* 2006 and references reviewed by Scharf *et al.,* 2012). The positions where these sequences are found in HaHSFA4 are shown in Figure 15A and Figure 22 (see amino acids 114-119, and 374-381, respectively). The carboxyl-terminal region of HaHSFA4 also contains the AD with two AHA motifs involved in transcriptional activation by different class A HSFs. These motifs, with acidic, hydrophobic and aromatic residues, match the FWxxF/LF/I/L consensus (von Koskull-Döring *et al.,* 2007). However, HaHSFA4 differs from the two HSFA4 factors of Arabidopsis. Such difference involves amino-acid residues (i.e., located in the DBD and OD regions) that are conserved in some HSFA4 factors from plants of families as *Fabaceae* and *Solanaceae,* which are more similar to sunflower than to Arabidopsis (Figure 15B).

Sequences for these domains are shown below.
1) HaHSFA4 DBD (SEQ ID NO. 11) (aa 21-113): 
PPFIAKTYEMVDDPSTNSIVSWSQTNRSFIVWNPPEFSGELLPRFFKHNNF
SSFIRQLNTYSFRKIDPELWEFANEDFIRGQPHLLKNIHRRK
2) HaHSFA4 OD (SEQ ID NO.12) (aa 143-199): 
YIEKISTLQHEKESLSIQFHMHKQEQERIKFEAHGLTDRLKRAAKLQKDILCC
LDEF
3) HaHSFA4 NLS (SEQ ID NO. 13) (aa 216-222):
   NRKRRLS
4) HaHSFA4 AHA1 (SEQ ID NO. 14) (aa 261-270):
   VMSWEGIFKE
5) HaHSFA4 AHA2 (SEQ ID NO. 15) (aa 332-341):
   DVFWEQFLTE
6) HaHSFA4 NES (SEQ ID NO. 16) (aa 374-381):
   LADQLGQL

Thus, according to the different aspects of the invention, a HSF4 peptide comprises a DBD as defined in SEQ ID No. 11 or which has at least 90%, 91%, 92%, 93%, 94%, 95%, 96%, 97%, 98% or 99% homology to SEQ ID No. 11, a OD domain as defined in SEQ ID No. 12 or which has at least 90%, 91%, 92%, 93%, 94%, 95%, 96%, 97%, 98% or 99% homology to SEQ ID No. 12, a NLS as defined in SEQ ID No. 13 or which has at least 90%, 91 %, 92%, 93%, 94%, 95%, 96%, 97%, 98% or 99% homology to SEQ ID No. 13, an AHA1 domain as defined in SEQ ID No. 14 or which has at least 90%, 91%, 92%, 93%, 94%, 95%, 96%, 97%, 98% or 99% homology to SEQ ID No. 14, an AHA2 domain as defined in SEQ ID No. 15 or which has at least 90%, 91%, 92%, 93%, 94%, 95%, 96%, 97%, 98% or 99% homology to SEQ ID No. 15 and an NES domain as defined in SEQ ID No. 16 or which has at least 90%, 91%, 92%, 93%, 94%, 95%, 96%, 97%, 98% or 99% homology to SEQ ID No. 16.

Motifs that characterise a HSFA9 peptide are described herein and peptides comprising one or more of these motifs as described herein are explicitly included in the aspects of the invention.

A nucleic acid sequence comprising SEQ ID No. 1 and/or 3, a functional homolog, variant or part thereof, a functional homolog, variant or part thereof may be under the control of a regulatory sequence. Suitable regulatory sequences are explicitly defined elsewhere herein. Other suitable promoters include embryo specific promoters, for example G1 or G4. According to the various aspects of the invention described herein, a nucleic acid sequence comprising SEQ ID No. 1 is co-expressed with nucleic acid sequence SEQ ID No. 3. In one embodiment, nucleic acid sequences comprising SEQ ID No. 1 and 3 may be included in separate vectors for expression. In another embodiment SEQ ID No. 1 and 3 may be included in the single vector.

As detailed above, in one embodiment of the methods of the invention, a single construct is used directing the co-expression of HSFA9 and HSFA4 encoding nucleic acid sequences.

In another embodiment, the method for producing a plant with enhanced stress tolerance comprises:
a) expressing a nucleic acid construct in a plant said construct comprising a sequence encoding a HSFA9 polypeptide,
b) expressing a nucleic acid construct comprising a sequence encoding a HSFA4 polypeptide as described herein,
c) crossing the first and second plant and
d) generating a plant homozygous for and expressing both HSFA9 and HSFA4.

Thus, the stable transformants of the first kind are crossed with stable transformants of the second kind to generate a stable homozygous progeny plant expressing both, HSFA9 and HSFA4. As a skilled person will know, crossing a HSFA9 plant and a HSFA4 plant will result in a "hybrid" that is hemizygous for each gene. The resulting plant has to be selfed and then the progeny selected to find double homozygotes - i.e. plants that are homozygous for both transgenes. A skilled person would also know that polyploids require more than one step of "selfing". Thus, the step of generating a plant homozygous for and expressing both HSFA4 and HSFA9 includes generating progeny of the plants obtained through step d) and selecting a plant that is homozygous for both transgenes.

In another embodiment, the method for producing a plant with enhanced stress tolerance comprises
a) expressing a nucleic acid construct in a plant said construct comprising a sequence encoding a HSFA4 or a HSFA9 polypeptide in a plant,
b) transforming said plant with a nucleic acid construct comprising a sequence encoding a HSFA4 polypeptide or a HSFA9 polypeptide respectively to generate a stable homozygous plant expressing HSFA4 and HSFA9.

According to this embodiment, a single transformant is created and the single transformant is transformed again with a nucleic acid construct comprising the second gene to generate a stable homozygous plant expressing HSFA9 and HSFA4. Stable homozygous plants are then selected.

The invention also relates to chimeric nucleic acid constructs that comprise HSFA9 sequences, for example HaHSFA9 sequences or sequences from a functional variant or homolog thereof.

The inventors have characterised HSFA9 and compared its structure to homologous sequences. Using chimeric constructs, the inventors have also found that it is the C-terminus of the HSFA9 protein that is important in conferring HSFA9 function.

Thus, the invention relates to a chimeric gene construct that comprises the LNK (SEQ ID No. 17 or a sequence which has at least 90%, 91 %, 92%, 93%, 94%, 95%, 96%, 97%, 98% or 99% homology to SEQ ID No. 17), OD (the elements as defined in SEQ ID No. 6 and 7 or which has at least 90%, 91 %, 92%, 93%, 94%, 95%, 96%, 97%, 98% or 99% homology to SEQ ID No. 6 and 7) and AD domains (SEQ ID No. 18 or a sequence which has at least 90%, 91%, 92%, 93%, 94%, 95%, 96%, 97%, 98% or 99% homology to SEQ ID No. 18) of HSFA9 fused to N-terminal residues of a different HSF, for example HSFA2 which comprise a DNA binding domain. Preferably, said different HSF is a class A HSF. The HaHSFA9 may be HaHSFA9 or functional variant or homolog thereof.

The oligomerization domain (OD or HR-A/B region) of Class A HSFs is connected to the DNA-binding domain (DBD) by a flexible linker (LNK) of variable length: 15 to 80 amino acid residues in total. LNK sequences are very little conserved besides their flexibility and are not considered as a domain.

In HaHSFA9 the LNK sequences comprise 28 amino acids, from positions 163 to 190: NQPQNTQKQEEIRKQEQQQCCGHQTNST (SEQ ID No. 17).

Sequences C-terminal (CT) of the OD include the activation domain (AD) of Class A HSFs. In HaHSFA9, these CTAD sequences comprise 124 amino acids, from positions 248 to 371 (SEQ ID No. 18):

FRNPIFVKLLQHLVQKQKTGSVEMCKKRKLEQMLNTDDLDRFQEMWNMIE
PDAYTVLSSDGSVSPPEDQNTSDKSGSNGSDYNSESFILWEKLMEDELIFG
GEQSGKDQTETYLQEWEELIPKA.

The CTAD sequences of HaHSFA9 include the three underlined AHA motifs, which are involved in transcriptional activation. These are SEQ ID No. 19: FQEMWNMIEP, SEQ ID No. 20: FILWEKLMED, SEQ ID No. 21: QEWEELIP. Mutation of these three AHA motifs abolished transcriptional activity of HaHSFA9, as determined by assays performed with yeast and bombarded sunflower leaves (Tejedor-Cano *et al.,* 2010)".

The invention also relates to a polypeptide encoded by this chimeric gene construct. The polypeptide is capable of conferring the same traits as HSFA9 when expressed in a transgenic plant.

The invention also relates to a vector comprising the chimeric gene construct. In one embodiment, the vector may also comprise a nucleic acid comprising SEQ ID No. 3, a functional homolog, variant or part thereof.

The invention also relates to an isolated cell expressing the chimeric gene construct. The cell may be a bacterial or plant cell. Also within the scope of the invention is a culture medium comprising a cell expressing the chimeric gene construct. The invention also relates to a culture medium comprising said isolated cell.

The invention also relates to a transgenic plant cell expressing the chimeric construct or vector described herein.

The invention also extends to harvestable parts of the transgenic plant expressing the chimeric construct or vector described herein such as, but not limited to seeds, leaves, fruits, flowers, stems, roots, rhizomes, tubers, and bulbs, which harvestable parts comprise a recombinant nucleic acid encoding a PIF polypeptide as described herein. The invention furthermore relates to products derived, preferably directly derived, from a harvestable part of such a plant, such as dry pellets or powders, oil, fat and fatty acids, starch or proteins.

The seeds of the invention in one embodiment comprise the constructs of the invention or the vector of the invention. In a further embodiment, the seeds of the invention are true-breeding for the nucleic acids, construct or the vector described herein. The invention also includes a product derived from the plant of the invention.

The invention also relates to plants obtained or obtainable by the methods described herein.

The invention also includes methods for the production of a product comprising
a) growing the plants of the invention and
b) producing said product from or by the plants of the invention or parts, including seeds, of these plants.

In a further embodiment the methods comprises steps
a) growing the plants of the invention,
b) removing the harvestable parts as defined above from the plants and
c) producing said product from or by the harvestable parts of the invention.

In one embodiment the products produced by said methods of the invention are plant products such as, but not limited to, a foodstuff, feedstuff, a food supplement, feed supplement, fibre, cosmetic or pharmaceutical. Foodstuffs are regarded as compositions used for nutrition or for supplementing nutrition. Animal feedstuffs and animal feed supplements, in particular, are regarded as foodstuffs. In another embodiment the inventive methods for the production are used to make agricultural products such as, but not limited to, plant extracts, proteins, amino acids, carbohydrates, fats, oils, polymers, vitamins, and the like. It is possible that a plant product consists of one ore more agricultural products to a large extent.

The invention also relates to an isolated nucleic acid comprising SEQ ID No. 3 or functional variant thereof. Also within the scope of the invention is a vector comprising SEQ ID No. 3 or functional variant thereof, an isolated cell expressing SEQ ID No. 3 or functional variant thereof or said vector.

The invention also relates to a transgenic plant co-expressing a nucleic acid comprising SEQ ID No. 3 and a nucleic acid sequence comprising SEQ ID No. 1.

All references mentioned herein are incorporated by reference.

### EXAMPLES

The invention is further described in the following non-limiting examples.

### Example 1 - Overexpression of HSFA9 leads to protection of PSII and PSI against severe dehydration and oxidative stress in the 35S:A9 seedlings.

### 1.1 Materials and Methods

### 1.1.1 Overexpression of HaHSFA9 in Transgenic Plants

Overexpression of HaHSFA9 in Transgenic Plants was carried out as described [1].For constitutive overexpression of HaHSFA9 in transgenic plants (35S:A9 lines), the cDNA containing the complete open reading frame and the untranslated 5'- and 3'-flanking sequences was obtained from plasmid p35S:HSFA9. A fragment containing the cDNA and the CaMV35S sequences was subcloned into the plasmid pBI101.1 by replacing the GUS coding region between the Sphl and Sacl sites. For seed-specific overexpression of HaHSFA9 (DS10:A9 lines), the complete cDNA was obtained from the plasmid pSKHSFA9-F.

First, the cDNA was inserted into the EcoRI site of pSKDS10EC1. Subsequently, a fragment containing the DS10-5':HaHSFA9:DS10-3' sequence was inserted between the Sall and Sacl sites of the binary vector pBIN19. Constructs with transgenes were transferred into the Agrobacterium tumefaciens strain LBA4404 and used for leaf disc transformation of tobacco *(Nicotiana tabacum* cv Xanthi). The T0 plants were regenerated and maintained as described previously. Transgenic plants were selected on medium with kanamycin (300 mg mL21) and different lines containing single transgene integration events in heterozygosis were obtained. This was confirmed by the 3:1 segregation observed for kanamycin resistance. Homozygous and sibling seeds without the transgene were obtained in the subsequent generation. Homozygosis was confirmed by the lack of segregation of antibiotic resistance and the absence of the transgene was inferred by sensitivity to kanamycin. Standard PCR was used to detect the presence of HaHSFA9, linked to the different promoter sequences, in leafsamples from the regenerated transgenic plants or their progeny. For PCR analyses of the 35S:A9 lines, the primers used were 5'-CATCTCTTCAGACAAAT- 3' (HaHSFA9) and 5'-ACTATCCTTCGCAAGACCCTTCC-3' (CaMV35S). Annealing was at 51"C for 1 min. The DS10:A9 lines were analyzed using primers 5'-CATCTCTTCAGACAAAT-3' and 5'-CCACCACGTCATCATACCAC- 3', which amplify a DNA fragment of 326 bp. The PCR conditions were as follows: 30 cycles of 1 min at 94"C, 1 min at 50"C, and 1 min at 72"C, plus a final step at 72"C for 5 min.

### 1.1.2 Plant growth conditions and stress treatments

The tobacco *(N. tabacum* L. var. Xanthi) T and NT seedlings, aged 3-4 weeks (with two pairs of true leaves), were grown on filter paper placed on Petri dishes with solid MS medium, as described [2].

In the modified dehydration treatment (DT) protocol, 3 to 4 T seedlings were placed on a glass 76 x 26 mm microscope slide, and the same number of the corresponding NT seedlings on a second slide. Both slides were placed alongside within the same closed glass container with 5 g silica gel and allowed to dry in the dark for 3-4 h, essentially as described for the DT2 assays [2]. This new protocol allowed using the dehydrated seedlings for immediate measurement of PSII fluorescence as described below.

For oxidative stress treatments, the filter paper with the seedlings (c.a., 60 per dish at a time) was removed and pre-washed in the solution used for the subsequent treatment: deionized water (for controls), or H₂O₂ (200 mM for the standard stress treatment). The H₂O₂ solution was prepared by diluting in deionized water the 30% w/w (8.82 M) H₂O₂ stock (Sigma #H1009). The final concentration was verified by measuring A₂₄₀ (A₂₄₀ = 1.31 for 30 mM H₂O₂). These solutions also included 0.1% Tween-20 to facilitate seedling penetration. After removing the excess of liquid, the seedlings were put back inside Petri dishes; the seedlings placed on 6 circles of filter paper wetted with the respective treatment solution. The Petri dishes were incubated at 20-25°C in the dark for 24 h. After the treatment, the seedlings were washed with deionized water. When the stress treatment was followed by a treatment with lincomycin, the seedlings were washed in 1 mM lincomycin and placed on filter paper soaked with 1 mM lincomycin within sealed Petri dishes; the seedlings were first left for 1 h in the dark to allow lincomycin penetration, and then put under normal light conditions (daylight lamps, ≈ 6,000 lux) for 6 h. For assaying survival of seedlings after the H₂O₂ treatment and washing with deionized water, the seedlings were placed again on wet filter paper within sealed Petri dishes. The seedlings were photographed after 1 week inside growth-cabinets with photoperiod.

### 1.1.3 Chlorophyll fluorescence

Chlorophyll fluorescence was measured with a mini-PAM Photosynthesis Yield Analyzer (Heinz Walz, Effeltrich, Germany). Immediately after the stress (or respective control) treatments, each seedling was placed between glass microscope slides (76x26 mm, bottom, and 60x24 mm, cover); the slides were fastened using DLC-8 clips. After a subsequent dark adaptation period of 25 min, minimum fluorescence (Fₒ) was determined by a weak red light. Maximum fluorescence of dark-adapted state (Fₘ) was measured during a subsequent saturating light pulse (14,000 µmol m⁻² s⁻¹ for 0.8 s). The maximal quantum efficiency of PSII (Fᵥ/Fₘ) was determined as the ratio of variable fluorescence (Fᵥ) to Fₘ. Measurements for six different seedlings per condition (e.g., control or stress treatment) were averaged in each independent experiment. Fᵥ/Fₘ values after the different stress treatments are represented as percent values of the respective control Fᵥ/Fₘ values. The seedlings were chosen to represent different locations within the Petri dish. The number of experimental repeats is indicated in the Figure legends.

### 1.1.4 Isolation of thylakoid membranes

Thylakoid membranes were prepared essentially as described [27]. Whole-seedling material was frozen and reduced to a fine powder using liquid N₂ in a cooled mortar; then it was homogenized under weak ambient light in ice-cold isolation buffer (330 mM sucrose, 25 mM HEPES-KOH, pH 7.4, 10 mM MgCl₂, and 10 mM NaF) supplemented with 1 mM Pefablock (Roche). Approximately 1 mL of buffer was used per 200 mg of seedling powder. Extracts were collected after filtration through Miracloth (Calbiochem). The filtrate was then centrifuged at 4°C for 5 min at 6,000*g*; pellets with the membranes were gently re-suspended in isolation buffer without sucrose, and washed in this buffer by centrifugation and re-suspension in the same conditions. The washed thylakoid membranes were re-suspended in Pefablock-supplemented isolation buffer (using 1 µL buffer per mg of starting frozen material), and stored at -80°C in aliquots of ≈ 50 µL. The protein content of the thylakoid membranes was quantified using the modification of the Lowry procedure by Markwell *et al.* [28].

### 1.1.5 Protein analyses

The procedures for the analyses performed with seedling total protein extracts were as described [1, 2]. 1D-PAGE with thylakoid protein extracts was performed similarly, only that the 12.5% polyacrylamide gels and sample buffer contained 4 M Urea. Immunoblot procedures using PVDF membranes were essentially as described [1, 2]. The following primary antibodies - obtained from Agrisera, Sweden- were used in this study, at dilutions indicated in each case in the corresponding Figures: Arabidopsis HSP21 (AS08285); D1 protein (PsbA, anti-C-terminal and anti-DE-loop, respectively AS05084, AS10704); PsaB (AS10695).

### 1.1.6 BN-PAGE

1D-BN-PAGE was performed as described [29] with slight modifications. Samples of washed thylakoids (100 µg total protein) were re-suspended in 10 µL 20% (w/v) glycerol, 25 mM BisTris-HCl, pH 7.0. An equal volume of re-suspension buffer containing 2% β-dodecyl-maltoside was gently mixed within a micro-pipet tip. Samples then were incubated for 30 min at 4°C, the insoluble material removed by centrifugation; the solubilized material was combined with Serva-Blue G and loaded into 0.75-mm-thick 5-13.5% acrylamide gradient gels [29]. These gels were run for 4-5 h at constant voltage (150 V) at 4-8°C. The cathode buffer was exchanged with buffer lacking dye after 2 h electrophoresis; the electrophoresis was stopped when most of the front-moving dye exited the gel. BN-Gels were then directly photographed, stained with colloidal Coomassie Brilliant Blue G-250 [30], or transferred to PVDF membranes. The immunoblot analyses after BN-PAGE differ from those previously described [1, 2] in that: 1. - Blocking with TBST was prolonged overnight to facilitate removal of the bound Serva-Blue G dye from the membrane. 2. - Hybridization with the primary antibodies was for 4 h at 25°C.

### 1.1.7 Statistical analyses

Differences between T and NT groups of sibling seedlings were tested by analysis of variance (ANOVA). The statistical analyses were performed as described in detail [1, 2].

### 1.2 Results

### 1.2.1 The seed HSFA9 program includes plastidial sHSPs that ectopically accumulate at high levels in the 35S:A9 seedlings.

We first investigated whether genes encoding plastidial sHSPs are activated in the HSFA9 program. To this end, commercial antibodies raised against HSP21 -a chloroplast-localized sHSP from Arabidopsis- were used. The accumulation of polypeptides detected by these antibodies was determined in transgenic lines that up-regulate (DS10:A9) or down-regulate (DS10:A9M3) the HSFA9 program in seed [1, 3]. The observed patterns were then compared with the accumulation of these polypeptides in vegetative organs of heat-acclimated non-transgenic lines, and in non-stressed 35S:A9 lines. Each transgenic line (T) was compared with its sibling non-transgenic (NT) control line obtained by Mendelian segregation. The T lines represent different single integration events of the respective *HaHSFA9* transgenes in homozygosis. Different sibling T/NT pair lines were used for the experiments described in Figure 1 (see the legend). The same three pairs of sibling 35S:A9 lines [2] were also used for the rest of the experiments in this report. Mature seeds of the DS10:A9 lines, which display gain of function of the HSFA9 program, showed consistently higher accumulation levels of HSP21-like polypeptides compared to the NT control lines (Figure 1, top). Conversely, loss of function of the HSFA9 program in the seeds of DS10:A9M3 lines abolished accumulation of these polypeptides compared to the respective NT control lines (Figure 1, middle). The specificity of the anti-HSP21 antibodies is evident, as the molecular mass of the detected polypeptides is consistent with that for processed plastidial sHSPs (mature forms without the chloroplast transit peptide). Furthermore, we did not detect the smaller polypeptides recognized by anti-Cl- and anti-CII-sHSP antibodies: compare Figure 1 with data in our previous publications [1-3]. Thus, genes encoding plastidial sHSPs are activated during seed maturation as part of the HSFA9 program, as previously shown for different sHSPs. In control (non-stressed) NT seedlings we could not detect accumulation of HSP21-like polypeptides; in contrast seedlings of the 35S:A9 (T) lines accumulated these plastidial sHSPs. The accumulation level of plastidial sHSPs in the 35S:A9 seedlings was consistently much higher than observed with heat-acclimated NT seedlings, where treatments for 3 h at 40°C induced plastidial sHSPs as expected (Figure 1, bottom). We also showed partial association to thylakoid membranes, under unstressed growth conditions, of the plastidial sHSPs overexpressed in the T lines. These plastidial sHSPs showed a thylakoid-membrane association at least as strong as that of the PsbP protein of PSII (Figure 8).

### 1.2.2 Protection of PSII and PSI against severe dehydration and oxidative stress in the 35S:A9 seedlings.

We previously showed that 3-week-old 35S:A9 (T) seedlings survive drastic dehydration; whereas 100% NT siblings die, ≈ 80% of the T seedlings recover from dehydration treatments (DT) up to ψ ≈ -20 MPa; the root is lost and only leaves and green tissue of the T seedlings survive to different extents after DT up to ψ ≈ -40/-50 MPa. Seedling survival was optimized by performing DT in the dark [2]. Here, the described DT2 protocol [2] was modified to allow performing DT using seedlings placed on glass microscope slide during the stress treatment (see "Material and Methods"). Severe dehydration was thus achieved to contents of 0.5 - 1.0 g water per gram of dry weight (corresponding to ψ≈ -20 MPa and lower), enabling measurement of Fᵥ/Fₘ of PSII immediately after DT (see "Materials and Methods" for details). Figure 2A shows that the maximal efficiency of PSII declined after DT, albeit to levels with respect to the control treatment values that were significantly lower for the NT seedlings compared to T seedlings. These results indicate protection of PSII in seedlings of the 35S:A9 plants. Damage after severe dehydration involves the dismantling of membranes. Dehydration damage also leads to the oxidation of cellular components by ROS (see [16] and references therein). Therefore, we also investigated whether the 35S:A9 seedlings can survive treatments with H₂O₂ in the dark, and if the PSII is indeed protected from oxidative damage. In initial experiments we found that the PSII of T seedlings was protected to different extent after treatments for 24 h with 50 to 500 mM H₂O₂. Treatment with 200 mM H₂O₂ was selected as the standard experimental condition, because it was the highest H₂O₂ concentration that allowed survival of ≈ 100% of the T seedlings. The photosynthetic organs -except cotyledons- conserved their green color. In contrast, the NT sibling bleached and did not survive treatments with 200 mM H₂O₂ (see representative results in Figure 3A). In Figure 3B, we also show that immediately after the 200 mM H₂O₂ treatments, the Fᵥ/Fₘ values declined to levels with respect to the control treatment values that were significantly lower for the NT than for the T seedlings. The results of Figures 2 and 3 demonstrate protection of vegetative organs and PSII integrity in the 35S:A9 plants.

Thylakoid chlorophyll-protein complexes were also analyzed using Blue-Native gels. These complexes were separated after mild solubilization using β-dodecyl-n-maltoside. We could detect -for example- monomeric PSII, dimeric PSII, and super-complexes with dimeric PSII associated to light-harvesting proteins. These complexes (Figure 2B), which in this gel system are not separated from PSI complexes, were assigned based on comparison of their mobility with those of thylakoid chlorophyll-protein complexes resolved by BN-PAGE reported in the literature (see e.g., [17-19]). Under control conditions, the detected complexes were similar for the NT and T seedlings (Figure 3C). Both the dehydration and H₂O₂ treatments destroyed the plastid membrane complexes in the NT seedlings (Figures 2B and 3C). In contrast, some complexes resisted the stress treatments in the T seedlings. This stress-protection was perhaps more evident for PSII-containing complexes. Notably the protection of PSII super-complexes, which are regarded as the functional photosynthetic units, was higher (Figures 2B and 3C). Interestingly, immediately after DT all complexes apparently resisted dehydration and appeared similar in both NT and T seedlings (Figure 2B, DT samples 0 h). However, following rehydration for only 1 h after DT most NT complexes disappeared, whereas T seedling complexes resisted (Figure 2B, DT samples 1 h). These results contrast with what observed for the H₂O₂ treatments, where protection of the complexes in the T seedlings could be observed immediately after treatment (Figure 3C). Most likely the rapid and drastic dehydration conditions used for DT do not allow the immediate disassembly and degradation of the complexes in the NT seedlings. Indeed, we reported that upon DT2 the water content is reduced below 2.5 g water/g dry weight after only 3 h dehydration [2]. Thus, at least the complexes of PSII in thylakoid membranes of 35S:A9 plants are indeed protected against the oxidative damage imposed by the severe DT and H₂O₂ treatments.

Protection of PSII and PSI complexes was further analyzed by immunoblot detection in Blue-Native gels of complexes that incorporate either the D1, or the PsaB protein, respectively (Figure 4). We detected not only the dimeric PSII complexes and PSII super-complexes, but also the partially assembled CV (PSII monomer) and CVII (CP43-less PSII monomer) complexes ([17], see Figure 4A, top). Using anti-PsaB antibodies, the monomeric PSI complex and the PSI-LHCI super-complex were also detected (Figure 4A, bottom). Under control conditions the complexes containing PSII or PSI were similar in samples from the T and NT seedlings. The H₂O₂ treatments destroyed most PSII and PSI complexes of the NT seedlings, and only some monomeric PSI, and CVII complexes of PSII persisted (Figure 4A). In contrast in the T seedlings, fully assembled PSII complexes endured the H₂O₂ treatment. In addition, partially assembled D1-containing complexes -in particular the CVII complex- accumulated to higher levels in stressed T than in NT seedlings (Figure 4A, top, H₂O₂ samples). The PSI-LHCI super-complex also tolerated the H₂O₂ treatment in the T seedlings (Figure 4A, bottom). Protection of the D1-containing PSII complexes and super-complexes was confirmed in the T seedlings after treatments of severe dehydration. These complexes persisted in the T and NT seedlings immediately after dehydration [Figure 4B, samples DT (0 h)]. However, 16 h following rehydration after the dehydration treatment, the PSII complexes of T seedlings resisted the treatment better than the NT seedlings. In this case, the CV complex is the partially disassembled D1-containing complex that accumulated to higher level in the T seedlings after the stress treatment [Figure 4B, samples DT (16 h)]. Thus, we confirmed structural protection of the two photosystems.

### 1.2.3 Protection of the D1 protein from damage and shielding of PSII repair following oxidative stress and dehydration in the 35S:A9 seedlings. Protection of PSI.

We analyzed if the D1 protein of PSII is protected under the drastic H₂O₂ and dehydration stress conditions used above to study the 35S:A9 seedlings. The preliminary characterization of the seedlings revealed that in our experimental control conditions the T seedlings had higher content of D1 protein than the sibling NT seedlings (Figure 9). In the following analyses, the NT and T protein samples were compared using immunoblots that were normalized attempting to show equal initial (control) levels of the D1 protein signals. To this end, higher amounts of total thylakoid-membrane protein were applied for NT samples. In that way the initial amount of detected D1 protein was similar for each pair of the NT and T samples. This facilitated visual comparison of the abundance of the different D1 forms detected in immunoblot analyses after the stress treatments: e.g., the intact protein, D1-protein adducts, and D1-degradation products. In Figure 5A we show representative results of samples from two pairs of sibling T and NT lines analyzed immediately after 200 mM H₂O₂ treatments performed in the dark. Consistent with the requirement of light for the degradation of the D1 protein, the initial accumulation level of the D1 protein detected in the NT and T samples before treatment did not substantially decrease during the incubation with H₂O₂ in darkness. A slight retardation in the mobility of the major D1 band was observed for both the NT and T samples after H₂O₂ treatments. However, only the NT samples showed bands that migrated above (≈ 44.4 kD) and below (≈ 20 and 19 kD) the major D1 band. These bands, which accumulated at low levels in the dark, most likely represent cross-linked adducts of D1 with closely located thylakoid proteins, and initial degradation products of the D1 protein, respectively. The observation for the treated NT samples would thus be similar to previous results describing damage of the D1 protein after *in vitro* exposure to H₂O₂ in the dark [20]. In contrast, D1 appears to be protected from oxidative damage in the treated T samples, as no cross-linked D1-bands or degradation products were detected (Figure 5A). In Figure 5B we depict the results of a similar analysis of samples from NT/T lines after severe dehydration (DT) treatment. There, immediately after DT no D1-degradation products were detected; however, the NT samples clearly show higher accumulation of a different D1-crosslinked band of ≈ 36 kD (Figure 5B, samples 0 h). Only following rehydration for 16 h after DT additional changes and differences between the NT and T samples were observed (Figure 5B, samples 16 h). These differences were similar to those found as consequences of the H₂O₂ treatments (compare Figures 5A and 5B). The results summarized in Figure 5 clearly show that the D1 protein of the PSII reaction center of the 35S:A9 plants was protected against the oxidative stress that occurs as a result of the severe dehydration and H₂O₂ treatments *in vivo.*

The synthesis of plastid-encoded components of PSII -as the D1 protein- has been shown to be rapidly inactivated by different forms of stress that cause oxidative damage (see for example [21] and references therein). Because the T seedlings survive DT [2] and drastic H₂O₂ treatments (Figure 3A) that involve ROS-induced damage, protection and/or reversible inactivation of PSII repair must also occur in the 35S:A9 plants. To test this, we analyzed the effects of lincomycin, a specific inhibitor of plastidial protein synthesis. Following 24 h treatments of seedlings in the dark in the absence or presence of 200 mM H₂O₂, the control and the stressed seedlings were transferred to normal illumination conditions for 6 h with or without the addition of lincomycin. In Figure 6A we depict the effect of lincomycin on D1 protein accumulation (the initial NT/T D1 protein levels were normalized as in Figure 5). Apparently under control conditions, normal illumination in the presence of the inhibitor can damage the D1 protein. Thus, the D1 protein of control NT seedlings showed a net degradation only in the presence of lincomycin (Figure 6A, C samples). This damage is clearly much lower in the control T seedlings, which showed higher levels of D1 protein in the presence of lincomycin. Therefore, we infer that protection of the D1 protein in T seedlings delays D1 degradation compared to NT seedlings even under control conditions. After H₂O₂ stress conditions protection and delayed degradation of the D1 protein in the T seedlings was even more evident; furthermore, it occurred to the same extent both in the absence and presence of lincomycin (Figure 6A, H₂O₂ samples). The accumulation level of the D1 protein in the H₂O₂-treated T seedlings was very similar to that of control T seedlings. In contrast, we observed substantial degradation of the D1 protein in the NT seedlings after exposure to H₂O₂ stress conditions, as demonstrated by a drastic reduction of D1 accumulation levels. That reduction also occurred both with and without the lincomycin treatment. We thus confirmed protection and delayed degradation of D1 in the stressed T seedlings. That lincomycin treatment did not have an additional effect on D1 accumulation after the stress treatments would fit the expected, H₂O₂-induced, inhibition of the protein synthesis [21] that is involved in replacement of the damaged D1 protein.

The exposure to H₂O₂ may inhibit the synthesis of the D1 protein in the NT seedlings to a similar extent as with the lincomycin treatment. To support this notion, the seedlings used for the protein analyses of Figure 6A were characterized in parallel for the indirect effects of lincomycin on the maximal quantum efficiency of PSII (Figure 6B). Lincomycin treatment significantly reduced the Fᵥ/Fₘ values of control NT seedlings, and a similar reduction, although lower, was also significant for control T seedlings (Figure 6B, compare C and C+L values). H₂O₂ treatment of the NT seedlings drastically decreased the Fᵥ/Fₘ values to very low levels. In contrast, the stressed T seedlings showed significantly higher Fᵥ/Fₘ values. These results confirm protection of PSII in the T seedlings, both under control and H₂O₂ stress conditions. Lincomycin treatment did not further decrease the marginal Fᵥ/Fₘ value observed for the H₂O₂-treated NT seedlings. However, for the H₂O₂-treated T seedlings a significant decrease of Fᵥ/Fₘ was observed upon lincomycin treatment (Figure 6B, compare H₂O₂ and H₂O₂+L values). This result would indicate both the protection from oxidative stress and the partial recovery of the plastidial protein synthesis involved in the repair of PSII in the 35S:A9 plants. In contrast, the PSII and the plastidial protein synthesis of the sibling NT plants would be irreversibly damaged, which contributes to the observed eventual death of these seedlings (see Figure 3A). In Figure 10, we present similar results using 35S:A9 plants subjected to DT. An indication of the recovery of plastidial protein synthesis was also observed only for the T plants. Sixteen hours after DT lincomycin significantly further reduced the Fᵥ/Fₘ values (Figure 10, difference indicated by the asterisk, F= 103.6, P= 0.0001). Thus, the recovery after DT appears to be delayed in comparison to what occurs after H₂O₂ stress.

The effects of the H₂O₂ treatments on the accumulation of the PSI core protein PsaB were also analyzed. Using the same control and H₂O₂-treated samples as in Figure 6A (the samples without subsequent treatment with lincomycin), complete degradation of PsaB in the NT seedlings was observed. In contrast, PsaB was still detected in the H₂O₂-treated T seedlings (Figure 11). Protection of the PsaB protein thus further confirmed the structural protection of PSI observed in these seedlings (Figure 4A, bottom).

### 1.2.4 The heat-acclimation response does not confer resistance to the drastic oxidative stress conditions tolerated by the 35S:A9 seedlings.

The HSFA9 program includes the seed expression of genes that encode HSPs of different kinds. Some of these HSPs also accumulated in seedlings in response to the sub-lethal heat stress acclimation conditions (3 h at 40°C) used to induce acquired thermo-tolerance [2]. It was previously shown [2] that heat-acclimated NT seedlings withstand treatments of 2.5 h at 50°C but are killed by the severe dehydration conditions (the DT2 treatments). In contrast, sibling T (35S:A9) seedlings withstand DT2 but are killed by the 50°C treatment unless previously heat-acclimated [2]. Here it was investigated if the same observation applies to resistance to the unusually high levels of oxidative stress imposed by the harsh H₂O₂ treatments used in this study (Figure 7). The effects of the H₂O₂ treatments as in Figure 3B (compare the C and H₂O₂ samples) were analyzed using also seedlings that were first heat-acclimated during 3 h at 40°C [2]. Heat-acclimation did not prevent the decrease of Fᵥ/Fₘ in the stressed NT seedlings; neither did it enhance the protection observed in the stressed T seedlings (Figure 7A). The heat-acclimated NT seedlings did not survive treatments for 24 h with 200 mM H₂O₂ in the dark (Figure 7B). In addition, the heat-acclimated T seedlings survived these treatments to the same extent as observed with non-acclimated T seedlings (Figures 7C and 7D). The genetic program(s) activated in vegetative organs by heat are thus insufficient for conferring resistance to the oxidative stress conditions used in the present study. Such conditions, however, are tolerated by the 35S:A9 seedlings, which overexpress the seed HSFA9 program.

### 1.3 Discussion

The ectopic activation of the HSFA9 program in vegetative tissues of transgenic tobacco led to phenotypes of tolerance to drastic dehydration [2]. Here, we found that these phenotypes include tolerance to very severe oxidative stress and protection of the photosynthetic apparatus (Figures 2-7). The HSFA9 program is seed-specific and thus our results suggest that such program is involved in the protection of seed non-photosynthetic plastids and proplastids, since these and other organelles must survive developmental desiccation.

The HSFA9 program conferred protection against severe dehydration and oxidative damage of photosynthetic membranes, PSII and core components of PSII as the D1 protein, as well as protection of PSI and its core protein PsaB. The protection appears to occur at different structural levels. We observed that both the D1 protein and its plastidial synthesis were resistant to damage (Figures 5 and 6). The integrity of supra-molecular membrane-protein complexes was also preserved to some extent; for example that of super-complexes with dimeric PSII, or PSI-LHCI (Figures 2B, 3C and 4). The *de novo* synthesis required for replacement of damaged proteins in the reaction center of PSII -in particular that of the D1 protein- affects the stability of PSII complexes in thylakoids; such complexes collapse under different experimental conditions where the synthesis is blocked (reviewed in [15, 22]). This suggests that the protection of the D1 protein and its plastidial synthesis might contribute to explaining the observed higher stability of PSII-containing complexes and super-complexes in the stressed 35S:A9 plants. In fact, we get converse effects on PSII as compared to mutants where a decreased stability of the PSII complexes was observed in coincidence with lower D1 protein accumulation/stability and lower D1-synthesis rates than in the WT. This includes mutants lacking PAM68 [18], or LQY1 [23]. Both PAM68 and LQY1 are thylakoid-associated proteins that would be involved in PSII-complex assembly by enhancing the turnover and biogenesis of the D1 protein. Therefore, the protection of other thylakoid-associated proteins, such as assembly factors, might also be required to explain the complex stress resistance phenotypes of the 35S:A9 seedlings. In conclusion, protection against oxidative damage of the D1 protein and that of its repair synthesis in the plastid could in part explain the drastic resistance to dehydration of PSII complexes in the 35S:A9 seedlings. It is worth noting that in some resurrection plants, but not in normal plants, the PsaB and D1 proteins, as well as PSI and PSII complexes, appeared to be similarly protected from dark dehydration treatments similar to those used here (e.g., [12, 13]). In these homoiochlorophyllous resurrection plants, which do not dismantle photosynthetic membranes when desiccated, this protection involves limiting the structural damage to levels that are reversible (reviewed in [16]). That would be similar to what we observed in Figures 2B, 3C and 4. The severe oxidative/dehydration stress conditions used in this work would irreversibly damage not only PSII but also PSI in the NT seedlings, which do not survive the stress treatments. PSI function in vegetative tissues strictly depends on protection mechanisms that are still not well characterized. These mechanisms would suffice to cope only with normal stress levels in most plants [11]. The severe stress conditions used here would overcome the normal protection of PSI/PSII. Only the activation of seed-specific protection mechanism(s) in the 35S:A9 seedlings would allow subsistence (PSI/PSII) and repair (PSII) of the photosystems and survival of the plantlets. In resurrection plants, genetic programs that are seed-specific in normal plants operate in vegetative organs; thus, the photosynthetic machinery would be protected from drastic dehydration in a similar way as proposed here [16].

We previously discussed the dehydration levels tolerated by whole 35S:A9 seedlings and by vegetative organs, such as leaves, in comparison to seeds, resurrection plants and mosses [2]. What was achieved in these seedlings is not a "canonical" desiccation tolerance as found in resurrection plants and seeds. However, the tolerated dehydration in 35S:A9 green organs is still far beyond what has been reported in any other study of non-resurrection plants. This includes genetic attempts to enhance their native tolerance (see [2] and references therein). The results presented in Figure 7 revealed that the oxidative stress conditions used in this report are as harsh as the drastic dehydration treatments (Figure 2 and [2]). Indeed, the H₂O₂ treatments damaged components of PSII, and even of PSI, which is considered to be resistant at least to moderate levels of stress [11]. Resistance to either very severe dehydration, or to the drastic oxidative stress conditions used in this report, was not observed after standard heat acclimation treatments (Figure 7). These acclimation treatments [2] induce a complex genetic response that in vegetative organs suffices only for coping with moderate levels of stress. In contrast, the unusual stress tolerance reported here required the activation of the seed HSFA9 program in vegetative organs of the 35S:A9 seedlings (Figure 7). Comparisons of the patterns of HSP and sHSP accumulation indicate that a partial overlap of common target genes exists between the HSFA9 program and the programs of vegetative heat-acclimation ([2]; Figure 1). We previously reasoned that the unusual dehydration-stress resistance of the 35S:A9 seedlings is explained by expression at normal growth of seed genes (HSFA9 targets) that differ from those activated by heat acclimation [2]. The present study extends that proposal. Here, we infer that only the specific target genes of the HSFA9 program can induce resistance of the photosynthetic machinery to both the severe dehydration, and to the drastic oxidative stress conditions used in our studies. The results in the present study also point to further differences in gene expression that involve plastidial sHSPs (as presented in Figure 1). Thus, the ectopic expression of different seed HSPs, including plastidial sHSPs, correlated with the observed protection in seedlings. However, we believe that it is unlikely that any single gene activated by HaHSFA9 suffices for this protection. The anti-oxidant functions demonstrated for plastidial sHSPs of higher plants [7-9, 24] might in part explain the observed protection of the D1 protein from oxidative damage. Thus, the 35S-driven overexpression of Arabidopsis HSP21 generated a higher abundance of the plastidial protein in unstressed leaves than induced by heat acclimation temperatures [25]. This is similar to what we observe for the HSP21-like polypeptides induced by HaHSFA9 in Figure 1 (bottom). The overexpression of HSP21 has been reported to only confer very moderate protection from oxidative damage under combined heat and high-light stress. Only subtle differences between the Fᵥ/Fₘ of transgenic and NT plants were observed [24]. Therefore, the overexpression of a single plastidial sHSP would not be sufficient for explaining the complex protection to extreme stress conditions that the 35S:A9 seedlings show. The association of different sHSPs with membranes has been found to decrease their fluidity, and in some cases to enhance thylakoid stability. The latter includes results obtained for sHSPs from cyanobacteria that are similar to sHSPs Cl (reviewed in [26]). In summary, seed sHSPs that include plastidial sHSPs could be in part responsible for the complex protection effects observed. Any other potential target gene of HSFA9 that is involved in the protection effects reported in the present study would also not be part of the vegetative programs of thermo-tolerance.

In conclusion, the seed HSFA9 program conferred protection of photosynthetic membranes in seedlings, from damage caused *in vivo* by very harsh dehydration and oxidation. We identified some of the protected components in the two photosystems. Protection of the photosynthetic apparatus from the extreme stress conditions used in this report would be unprecedented for normal plants. The results presented here might facilitate the future engineering of stress-tolerant photosynthetic membranes, green organs, and even whole plants.

### Example 2 - Overexpression of HSFA9 delays senescence in seedlings

### 2.1 The 35S:A9 seedlings show resistance to lethal conditions of, dark-induced, leaf senescence.

The ectopic overexpression of A9 in the 35S:A9 tobacco seedlings protected the photosynthetic apparatus from very harsh dehydration and oxidative stress treatments, which were performed in the dark. In addition, in our Petri dish culture conditions (under photoperiod, in absence of other stress), the 35S:A9 tobacco seedlings showed statistically significant higher Fv/Fm values than for non-transgenic sibling lines. The dark-adapted, maximal, PSII efficiency (Fᵥ/Fₘ) can be used as a measure of PSII damage. The unstressed T seedlings showed higher Fᵥ/Fₘ values than NT siblings. The respective average values from all Fᵥ/Fₘ determinations in each case were: 0.792 ± 0.002 (T, n= 288) and 0.697 ± 0.004 (NT, n= 299). Thus, the T value was≈ 13.6% above that for NT. This difference showed high statistical significance (F= 405.05, P= 0.0001). This would be consistent with an enhanced protection of PSII against cumulative stress damage inferred under normal growth conditions in the T seedlings. Also fitting with this possibility, we observed higher levels of D1 protein accumulation in the unstressed T seedlings compared to the NT siblings (between 1.7 and 4-fold higher). The D1 protein is perhaps the most labile protein component of the PSII reaction center.

Furthermore, the accumulation of plastidial-encoded proteins of PSII, as that of the D1 (PsbA) protein was higher in the 35S:A9 tobacco seedlings. Finally, as discussed above, the resistance of PSII, PSI, and of specific components of the two photosystems to drastic stress was only comparable to what described for resurrection plants (Dinakar *et al.,* 2012). Some resurrection plants withstand dark for very long periods that induce senescence and death of normal plants (Denev *et al.,* 2012). These precedents led us to investigate whether the 35S:A9 tobacco seedlings resist dark-induced senescence, and to what extent.

The results of Figure 12 summarize two independent experiments performed with 4-week old seedlings grown under photoperiod on filter paper (d= 50g/m²) placed on MS medium in Petri dishes. In each experiment, we used 3 pairs of different transgenic (T, 35S:A9) and sibling non-transgenic (NT, syngenistic) lines: the previously described T₁/NT₁, T₂/NT₂, and T₃/NT₃ pairs (Almoguera *et al.,* 2012). We included 4 dishes per line (NT or T) and experiment (total of 24 Petri dishes). For the prolonged dark incubation treatment, each filter paper disk with the 4-week old seedlings was transferred to new Petri dishes, each containing 4 filter paper disks (d= 73 g/m²) wetted with 4 ml MilliQ water. On top of the seedlings, 2 additional filter paper disks (d= 73 g/m²) wetted with 2 ml MilliQ water were placed. The Petri dishes were sealed with Parafilm"M"®, wrapped in aluminum foil, and placed in the dark at 24°C within SANYO MLR-350H growth cabinets for the indicated time (from 1 to 5 weeks). Total chlorophyll and the maximum quantum yield of PSII (Fv/Fm) was measured immediately before the dark treatment and at the indicated dark treatment times using the seedlings of one Petri dish per experimental case and time: Fv/Fm values were obtained from 9 seedlings and the chlorophyll extracted from 5 seedlings in 1 ml 80% acetone. The remaining seedlings (approximately 50 per dish) were used for the survival assays performed after the dark treatments. The filter paper disk with these seedlings was placed on MS medium Petri dishes under photoperiod and photographed ten days after.

It was observed that after 1 week of dark treatment the total chlorophyll content of the seedlings decreased, although to a lower level in the non-transgenic (NT) than in the 35:A9 (T) seedlings: to ≈51% and ≈68% of the initial content, respectively (Figure 12A). Such decrease is expected, as chlorophyll degradation is the first visible symptom of leaf senescence (Buchanan-Wollaston *et al.,* 2003). The lower decrease of chlorophyll content in the 35:A9 seedlings indicates that A9 alleviates the senescence symptoms. After 3 weeks of dark treatment the A9 effect on senescence was still evident as the chlorophyll content of the 35S:A9 seedlings was reduced to ≈47% compared to reduction to ≈34% in the NT seedlings. However, the A9 effect on senescence was not observed after 5 weeks of dark treatment. At this time a similar drastic reduction of the chlorophyll content (to less than ≈30% of the initial content) was observed for both the NT and the 35S:A9 seedlings (Figure 12A).

A similar conclusion was inferred from the observation of protection of the Fv/Fm in the dark-treated 35S:A9 seedlings, compared to NT seedlings (Figure 12B). It is noteworthy the very deep decrease of Fv/Fm observed in the NT seedlings after only 1 week of dark treatment (to ≈22% of the initial value, compared to ≈63% for the 35S:A9 seedlings). Such result showed A9-mediated protection of the PSII from damage induced by dark, a damage that, judging from the Fv/Fm values appears to be more severe than that caused by oxidative damage after treatment of seedlings with 200mM H₂O₂ for 24h in the dark (see figures 3 and 12). PSII Fv/Fm protection was still evident after 3 weeks under dark conditions (≈15% for NT versus ≈28% for T), but it disappeared after 5 weeks of dark treatment as observed for chlorophyll reduction (compare Figures 12B and 13A).

The dark-induced senesce was not lethal after the 1-week treatment, as both the NT and 35S:A9 seedlings similarly recovered after transferring them to normal grown conditions with photoperiod illumination. In contrast, after 3 weeks treatments, dark induced senescence was lethal the NT seedlings (0%-5% survival observed); however the 35S:A9 seedlings recovered (80-100%), as illustrated by Figure 12C.

We conclude that A9 enhances leaf longevity by protection against dark-induced senescence. The protection was observed under very harsh stress conditions that are lethal for non-transgenic seedlings.

### 2.2 The enhanced resistance to dark-induced leaf senescence persists in 4-week old 35S:A9 plants grown on soil.

We have investigated whether the A9-induced resistance to dark induced senescence observed with small seedlings (≈ 0.8 cm broad, with 2 pairs of true leaves, see Figure 1) persists in larger and older plants grown on soil. For the experiments in Figure 13, the 35S:A9 and NT seeds (a single seed/plant per pot) were germinated in Florabella® potting soil mixed with perlite (4F:1 P), within 200ml pots (7cm top diameter, 4cm bottom diameter, 9 cm length). After 4 weeks of growth under photoperiod illumination, the plants (≈ 10 cm broad) had 8 pairs of true leaves. These plants were watered with 70ml tap water 1 day before the treatment, which was performed by placing the uncovered plants within a dark growth chamber at 22-24°C and 60-65% RH. Figure 13 includes the results of two independent experiments, performed with the 3 pairs of different transgenic (T, 35S:A9) and sibling non-transgenic (NT, syngenic) lines: For experiment 1 (Figure 13A) we used 4 single plants (pots) per each NT or T line. For experiment 2 (Figure 13B), we used 3 plants (pots) per line. In Figures 2A-B, we show average values of Fv/Fm measures in individual leaves at different positions within the plants. The Fv/Fm measurements were performed as essentially described (Almoguera *et al.,* 2012), after 22 days (Figure 13A) or 27 days (Figure 13B) of dark treatment. Each Fv/Fm determination was repeated twice on each individual leaf. We depict the Fv/Fm values from the third top leaf (younger) to the sixth leaf (older, near the bottom). The leaves were numbered just before transferring the plants to the dark. In the dark the plants elongate and develop additional leaves that are visible in Figures 14C and 14D Total Fv/Fm determinations per individual leave and case (T or NT) were: n=24 (Figure 13A), and n=18 (Figure 13B).

In the employed experimental conditions, the absolute Fv/Fm of the leaves of 35S:A9 was higher than that of sibling NT plants after dark-induced senescence. The tobacco plants grown in pots resisted quite well 22 days under dark conditions (experiment 1, Figure 13A); there, only in the older 5th and 6th leaves senescence damage of PSII (i.e., lower Fv/Fm values) was evident in the NT lines compared to the T lines. In experiment 2, the longer dark incubation (for 27 days under dark) might explain the higher damage in the NT lines. In this case protection in the T lines was more evident in the younger 3rd and 4th leaves than in the older leaves (Figure 13B). For experiment 2, we also include pictures of plants, which after the dark treatment were transferred to normal photoperiod illumination conditions for 2 days (Figures 14C and D, scale bars = 1cm). Leaf recovery from symptoms of induced senescence and leaf survival was clearly improved in the T plants compared to the sibling NT plants. This is shown with representative pictures of the T₁/NT₁ (Figure 13C) and T₂/NT₂ (Figure 13D) pairs.

These results indicate that the protection from dark-induce senescence persists in 35S:A9 plants grown on soil during vegetative stages well beyond germination.

### 2.3 The persistence of resistance to senescence in the 35S:A9 plants correlates with described molecular effects of HaHSFA9 (A9).

In young seedlings, grown on MS medium, the effects of A9 on tolerance to severe dehydration and oxidative stress, and on protection from dark-induced senescence (Figure 12), correlate in all cases with the accumulation in these seedlings of different HSPs (Prieto-Dapena *et al.,* 2008). Of these HSPs, some as HSP101 have high molecular weight. Other small HSPs (sHSPs) of lower molecular weight are induced by A9; among them the cytosolic Cl (sHSP-Cl) and plastidial (sHSP-P) types might have some relevance for protection of the photosynthetic apparatus from oxidative and other senescence damage. Regarding sHSP-P, we discussed their possible contribution to protection from oxidative and severe dehydration damage (Almoguera *et al.,* 2012). The sHSP-Cl considered as cytosolic/nuclear proteins, also associate with chloroplasts,and they are involved in targeting membrane proteins to plastid outer membranes (Kim *et al.,* 2011).

We investigated if the previously reported correlation of the molecular effects of A9 also holds for the phenotypes of the 35S:A9 plants grown on soil (Figure 13). In Figure 14, we analyzed HSP accumulation in these plants grown between 2 and 5 weeks on soil. The accumulation levels at normal growth temperature of sHSP-P, sHSP-Cl, and HSP101 were determined by western blot analyses using antibodies specific for each HSP type. The HSP accumulation levels were compared with that of 35S:A9 seedlings grown for 4 weeks on MS (MS). The accumulation of sHSP-P persisted at high levels from 3 to 5 weeks; those of sHSP-Cl only slightly decreased; HSP101 persisted until at least 4 weeks (not determined at 5 weeks). Thus, the previously described molecular effects of A9 also correlate with the resistance to dark-induced senescence observed for the 35:A9 plants in Figure 13.

### 2.4 Diminished leaf senescence symptoms in, well-watered, 35S:A9 plants grown under normal photoperiod illumination conditions for 7 weeks.

In these experiments, we used NT and sibling 35S:A9 plants grown on Florabella® potting soil mixed with perlite (4F:1 P), within ≈ 200 ml pots (same initial conditions as for the experiment of Figure 2). The plants are kept well irrigated under normal photoperiod illumination culture conditions (Prieto-Dapena *et al.,* 2008) for a total of 7 weeks. The plants were photographed after 4 weeks culture. After 7 weeks culture, we evaluated the Fv/Fm, total chlorophyll and total carotenoid content of individual leaves located at positions 3, 4, 5, 6 and 7 from the top of the plants. Immediately thereafter Fv/Fm determination, leaves at each position, from two plants per analyzed line, were detached and photographed on filter paper. Two independent experiments were performed using plants from six lines (NT₁, NT₂, NT₃, A9₁, A9₂ and A9₃) from the 3 sibling pairs. In experiment 1, two A9 plants per line (n= 6) and 3 NT lines (n= 9) were used. For experiment 2, we used 4-5 plants per A9 or NT line (n= 12-15 in both cases). Pigment content was determined from ≈ 350 mg, pooled,fresh-sliced material from the same positioned leaves of 6 plants (2 plants per each of 3 NT or A9 lines). Chlorophyll content for the leaves at position 7 was determined only in Experiment 2.

In Figure 30 we grouped the results of the quantitative determinations from the experiments 1 and 2. After 7 weeks culture, the maximal photochemical efficiency of PSII levels (Fv/Fm, Figure 7A), the chlorophyll (Figure 30B) and the carotenoid content (Figure 7C) decreased more in the NT leaves than in the A9 leaves. Such differences were clearer in the older leaves (positioned near the bottom, as leaves numbered 6 or 7). After 4-week culture the plants (NT or A9) showed little if any visual symptoms of senescence damage (Figure 31A, representative results shown for the NT₂/A9₂ line pair). However, after 7-week culture, the leaves of NT plants showed more senescence symptoms (yellowish color, dead tissue) than the leaves of A9 plants. Moreover these senescence symptoms appeared in "younger" leaves (placed near the top) in the NT lines than in the A9 lines.

Our results are consistent with protection from senescence damage of PSII in the A9 lines. This senescence damage might be caused by micronutrient and/or macronutrient deprivation after prolonged culture in the small pots used in our experimental conditions (see for example, Thomas and De Villiers, 1996; Wingler *et al.,* 2005; reviewed by Roberts *et al.,* 2012). We note that when we cultured the same NT and A9 lines in larger pots (of= 1700 ml volume) under similar watering and soil conditions for longer periods (up to seed production) no evident visual symptoms of senescence were observed (Prieto-Dapena *et al.,* 2008 and unpublished observations). We also note, that different from the experiments of Figure 2, here the plants were well-watered during the whole culture duration and were not exposed to dark conditions for the 3 last weeks. Therefore, the 35S:A9 plants resisted different types of stress-induced senescence.

### Example 3 - Synergistic transcriptional activation and repression of a genetic program of seed longevity.

In sunflower (*Helianthus annuus*) a genetic program involved in seed longevity is activated by Heat Stress Factor A9 (HaHSFA9). Here we cloned and characterized HaHSFA4, a peculiar HSF that interacted with HaHSFA9 in plant cells using BiFC assays. HaHSFA4 showed cytosolic and nuclear localization; however, interaction with HaHSFA9 resulted in nuclear retention of HaHSFA4. HaHSFA9 and HaHSFA4 could synergistically co-activate transcription from promoters of putative target genes of HaHSFA9, as revealed by transient assays performed in sunflower. Using hybrid HSFs containing sequences from HaHSFA9 and from LpHSFA2, a class A HSF unable to synergize with HaHSFA4, we determined that only the HSF-interactions that caused nuclear retention of HaHSFA4 resulted in synergistic coactivation. Furthermore HaHSFA4 and HalAA27 showed BiFC interaction in immature sunflower embryos. In addition HalAA27 could repress the synergistic co-activation by HaHSFA4 and HaHSFA9. HalAA27 impaired interaction of HaHSFA4 with HaHSFA9 in sunflower embryos; auxin, by destabilizing HaIAA27, would facilitate the synergistic interaction of HaHSFA4 with HaHSFA9. We propose that in sunflower HaHSFA4 and HaHSFA9 co-activate the same genetic program of seed longevity. The inferred co-activation involves a novel functional combination of class A HSFs. This proposal was confirmed in transgenic tobacco by observing an enhancement of seed longevity after the combined overexpression of the two HSFs.

### 3.1 Materials and methods

### 3.1.1 Cloning of the full-length HaHSFA4 cDNA.

The complete HaHSFA4 cDNA sequences were assembled in plasmid pUC-HaHSFA4 from two DNA fragments of 921 bp and 610 bp. The 916 bp fragment contained the coding sequence of HaHSFA4 downstream of the *BsaB*I site (position 608) and the 3'-UTR to position 1516. This fragment was derived, by digestion with *BsaB*I and Bg/II, from one of the plasmids with a clone obtained by two-hybrid screening of the sunflower embryo cDNA library (Almoguera *et al.* 2002), pGAD424#33. The rest of coding sequences and the 5'-UTR sequences of HaHSFA4 were obtained by RNA Ligase-mediated amplification (RLM-RACE, see Almoguera *et al.,* 2002 and other details listed below). The sequence of the two strands of the HaHSFA4 cDNA in pGAD424#33 plasmid (positions 588-1716) and those of all PCR-amplified fragments was obtained and verified. The same applies for the rest of PCR-amplified sequences for the rest of plasmid constructs in this report.

### 3.1.2 Two-hybrid cloning and interaction assays is yeast.

Conditions for cDNA library (Almoguera *et al.,* 2002) screening, and for the interaction assays were essentially as described (Carranco *et al.,* 2010). The positive clones obtained were verified, after retransformation in the PJ69-4A yeast strain (James *et al.,* 1996), by their ability to grow in selective medium without leucine, tryptophan and histidine, supplemented with 50mM 3-AT. The pGBT9-derived HaHSFA9 plasmids numbered 1 and 2 in Figure 15C have been previously described, respectively as pGBT9-HaHSFA9mAD and pGBT9-HaHSFA9ΔDBD (Carranco *et al.,* 2010). Construction of other plasmids used in Figure 15C is listed below.

### 3.1.3 In planta BiFC interaction and protein localization assays.

For the assays in leaves of *N. benthamiana,* 3 to 4 week-old plants were grown and infiltrated with mixtures of *Agrobacterium* strains, which contained the required plasmid combinations, as described (Carrancoet *al.,* 2010). 48 h after infiltration, disc sections from the infiltrated leaves were analyzed.

For the experiments in Figure 16, BiFC was studied under a Leica TCS-SP2 confocal laser-scanningmicroscope with a HC PL FLUOTAR 20x/0.50 objective. We used standard YFP excitation and detection settings. Image analysis was carried out with Leica LCS software and Adobe Photoshop 7.0. For BiFC assays in sunflower embryos (Figures 20, 21A and 21 B), 15 dpa embryos were bombarded and analyzed using the Olympus FV1000 microscope as described (Carranco *et al.,* 2010). Plasmid constructs for BiFC in embryos were derived from the SPYCE(M) and SPYNE173 vectors (Waadt *et al.,* 2008). The HaHSFA9, HaHSFA4, and hybrid-HSF sequences fused to the non-fluorescent YFP-halves in these constructs were PCR-amplified from the respective effector plasmids used for the transient activation assays (see below). The interference of HalAA27 on the BiFC between HaHSFA4 and HaHSFA9 in embryos (see Figure 21A and 21 B) was analyzed by co-bombardment of 5 µg of a plasmid encoding the HalAA27 stabilized mutant mIIab (pBI221-HaIAA27mIIab, see Carranco *et al.,* 2010). The same amount of pBI221 plasmid was used as a negative control.For BiFC and protein localization in leaves, we used the SPYCE (YFP^{C}) and SPYNE (YFP^{N}) fusions transferred to the corresponding binary vectors: kanII-SPYCE(M) and kanII- SPYNE173 (Waadt *et al.,* 2008).

For the protein localization assays of Figure 18, the infiltrated leaf sections were analyzed with a confocal laser-scanning Olympus FV1000 microscope. In this case we used UPLSAPO 20x NA:0.75 objective and standard GFP filter settings. Image analysis was performed with FV10-ASW 1.7 and Adobe Photoshop 7.0 software. Image acquisition conditions were adjusted for each sample, to avoid saturation in most of the pictured area. The GFP-fusion constructs used for the localization assays in leaves are described below. The strain with the WT HaHSFA4: GFP fusion was also co-infiltrated with strains containing non-fluorescent YFP^{C} fused to HaHSFA9, or to the A2:A9₁ andA2:A9₂hybrid proteins. The A9:YFP^{C} fusion (Carranco *et al.,* 2010) and the A2:A9₁hybrid protein (HSFA2-A9C in Díaz-Martín *et al.,* 2005) have been previously described. Construction of the A2:A9₂hybrid protein is described below (see plasmids for transient expression assays).

### 3.1.4 Transient activation and repression assays in sunflower.

The assays using 20 dpa sunflower embryos (Figure 17A) were performed essentially as described (Díaz-Martín *et al.,* 2005). The amounts of plasmid DNA included per precipitate(5 shots), as indicated, were: 1 µg pBI221-RIuc, 5 µg of -289(G1):LUC reporter, 2.5 µg pBI221-HaHSFA4, and 125 ng pBI221-HaHSFA9. For the assays of Figure 20D, 15 dpa sunflower embryos were used to insure that the WT form of HalAA27 is stabilized (Carranco *et al.,* 2010). Bombardment was as for 20 dpa embryos, except for the reporter plasmid used [-1132(G4):LUC] and for the amounts of pBI221-HaHSFA9 and pBI221-HaIAA27 effectors (40 ng and 5 µg , respectively). The total amount of plasmid DNA in Figures 17 and 20D was adjusted with pBI221 to 8.5 µg or 13.5 µg, respectively.

Bombardment of sunflower leaves was performed essentially as described (Tejedor-Cano *et al.,* 2010). For the assays in Figure 17B, the amounts of plasmid DNA were as in Figure 17A. For the assays in Figure 19 the amounts of plasmid DNA were as in Figure 6D except for the amounts of effector plasmids: 125 ng of pRT-LpHSFA2 (Rojas *et al.,* 2002), and 20 ng of pBI221-HaHSFA9, pRT-A2:A9₁ or pRT-A2:A9₂. The total amount of plasmid DNA was adjusted with pBI221 to 8.5 µg.

We have previously described the internal reference plasmid (pBI221-RIuc; Díaz-Martín *et al.,* 2005) and most of the reporter and effector plasmids used for transient assays and as a source for the fusion proteins employed in this report: -289(G1):LUC (Díaz-Martín *et al.,* 2005), -1132(G4):LUC (Tejedor-Cano *et al.,* 2010), pBI221-HaHSFA9 (Almoguera *et al.,* 2002), pB1221-HalAA27 (Carranco *et al.,* 2010), pRT-LpHSFA2 (Rojas *et al.,* 2002), and pRT-A2:A9₁ (pRT-HSFA2-A9C in Díaz-Martín *et al.,* 2005). The rest of plasmids are described below.

### 3.1.5 Generation and analyses of tobacco double-transgenic lines.

Two homozygous transgenic lines that overexpress HaHSFA9 from DS10 (a seed-specific promoter) were transformed with a similar transgene that overexpresses HaHSFA4 (see below). The parental transgenic lines were previously described as DS10:A9#6-7 and DS10A9#14-5 (Prieto-Dapena *et al.,* 2006). In Prieto-Dapena *et al.,* (2006), we also described procedures for tobacco transformation and for the selection of transgenic lines. We first obtained heterozygous DS10:A4 lines (with single integration events) in the two homozygous DS10:A9 backgrounds. The double-homozygous DS10:A9/A4 lines were obtained by segregation in the subsequent generation. We also selected for the sibling DS10:A9 lines, which were used as the proper, single-transgenic control, lines. These procedures were described in detail for a similar analysis of the effects of HaDREB2 on the DS10:A9 lines (Almoguera *et al.,* 2009). Seed sterilization, germination, and seedling growth under controlled conditions were as described (Prieto-Dapena *et al.,* 2006). Germination of seeds after accelerated aging treatments was performed as described (Prieto-Dapena *et al.,* 2006), except that the BTA treatments were for 4 hours at 52°C.

A 3xHA-tagged form of HaHSFA4 was integrated in a binary plasmid derived from pSK-ds10EC1 (Rousselin *et al.,* 2002) and pBIB-Hyg (Becker, 1990). This binary plasmid, used for transformation of the DS10A9 lines, was named pBIB-DS10:3xHA:HaHSFA4:DS10 (for plasmid construction details, see below).

### 3.1.6 Statistical analyses.

The differences between reporter gene activity in transient expression, as well as the differences between transgenic groups of germinating seeds, were tested using ANOVA. Germination after accelerated aging was compared using repeated measures ANOVA. Statistical analyses have been previously described in depth (Prieto-Dapena *et al.,* 2006 & 2008).

**3.1.7 Details for RLM-RACE and cloning of the HaHSFA4 full-length cDNA**:for RLM-RACE (see Almoguera *et al.,* 2002), we used an oligonucleotide specific for HaHSFA4. This HSFA4-RACE primer (5'-GGAGACGACGACGATGATGATCCATT-3') spans from positions 629 to 604 in the non-coding strand. The PCR-amplified sequences were cloned in plasmid pCR^{R}4-TOPO^{R} (Invitrogene). We obtained plasmid pCR^{R}4-TOPO^{R} - HaHSFA4-5'#35, which was used to obtain the 610 bp fragment by PCR amplification followed by *Xbal* digestion. The amplification was with 5'-aaacgaattcgcctctaGACACTGACATGGACTGA-3', which contains a *Xbal* site placed next to position 1 of HaHSFA4, and with 5'-TGATCCATTGTTTGCAAGATT-3', starting with the *BsaB*I half-site at position 612; both sites are respectively underlined. The 921 bp and 610 bp fragments were combined by their ligation between the *Xbal* and *BamH*I sites of the pUC19 vector, which originated pUC-HaHSFA4.

**3.1.8 Plasmid constructs used in the yeast two-hybrid assays:**pGBT9-HaHSFA9Δ166 (numbered 3 in Figure 15C) contains a PCR-amplified fragment of 533 bp, with amino-acid residues 1 to 166 from HaHSFA9 inserted in frame at the *Sma*I site of pGBT9. pGBT9-HaHSFA9Δ284 (numbered 4 in Figure 15C) was constructed by a similar insertion at this site of a 867 bp fragment with amino-acid residues 1 to 283 from HaHSFA9. The HaHSFA4 prey plasmid, pGAD424-HaHSFA4, contains the complete coding sequence of HaHSFA4 (amino acids 1-387) cloned in frame between the Ncol and *Pst*I sites of the pGAD424 vector (Clontech). The required restriction sites, at the ends of the PCR-amplified fragment of 1333 bp, were introduced with the oligonucleotides 5'-GGTATATCTTGGTCcATGgTGAATGATGTTCATGGGAATTTG-3' and 5'-GAGCTCGGTACCCGGGGATCTGCAG-3'. The respective sites are underlined.

**3.1.9 GFP-fusion constructs used in the experiments of** **Figure 4**:These fusions contained the, PCR-amplified, coding region of HaHSFA4 inserted between the *Sal*I and *Sma*I sites of the pSAT6-EGFP-C1 plasmid (Tzfira *et al.,* 2005). The HaHSFA4:GFP fusions were transferred from the pSAT6-EGFP-C1 plasmid to the pRCS2-*nptII* binary vector (Tzfira *et al.,* 2005), and used in *N. benthamiana.* The WT and NESmut HaHSFA4 fusions differ in six nucleotide substitutions, which were introduced by a, two-step, megaprimer PCR-mutagenesis (see Chen and Przybyla, 1994; Carranco *et al.,* 2010). We used the pSAT6-EGFP:HaHSFA4 plasmid as the amplification template and 5'-TGTAAATAATgcTGCTGATCAGgcAGGACAGgcTACTTCAGTAGAGAGA-3' as the mutagenic primer. This resulted in the three amino acid substitutions, within the NES, described in Results.

**3.1.10 Plasmids for transient-expression assays:** The HaHSFA4 effector plasmid, pBI221-HaHSFA4, was constructed from pUC-HaHSFA4. The 1514 bp fragment (containing 228 bp of 5'-UTR, 1163 bp of coding sequences, and 123 bp of 5'-UTR) obtained by digestion of pUC-HaHSFA4 with *Xbal* and *Sac***I** was introduced between these restriction sites in pBI221 (Acc AF502128). The A2:A9₂ hybrid HSF contains the LpHSFA2 sequences from amino acids 1 to 136, followed by HaHSFA9 sequences from amino acids 164 to 371. The A2:A9₂ protein was obtained by PCR-based fusion (Hobert, 2002; Díaz-Martín *et al.,* 2005). PCR1 (with an annealing temperature of 57°C) was performed with the oligonucleotides 5'-ACTATCCTTCGCAAGACCCTTCC-3' (OliA, Díaz-Martín *et al.,* 2005) and 5'-gtttttgtgtgttttgtggctgGCAAGCACCAGATCCTTGTTGATT-3' (OliB), using pRT-LpHSFA2 (Rojas *et al.,* 2002) as the template for amplification. The lowercase sequences in OliB are a 22-nucleotide overhang that encodes HaHSFA9 amino acids from position 164. The uppercase sequences encodeLpHSFA2 amino acids ending at position 136. PCR2 (with an annealing temperature of 51°C) was performed with the oligonucleotides 5'-CAGCCACAAAACACACAAAAAC-3' (OliC) and 5'-TCGGAATTAACCCTCACTAAAG-3',(OliD, T3 sequences), using pSKHSFA9-F (Almogueraet *al.,* 2002) as the amplification template. The OliC sequences are the reverse complement of the 22-nucleotide overhang contained in OliB. The fusion PCR (with an annealing temperature of 47°C) was performed with OliA and 5'-TCTAGAACTAGTGGATC-3' (OliD'). OliD' contains, nested, SK sequences that are located 59 nucleotides upstream of OliD. We used the mixed, unpurified, products of PCR1 (501 bp) and PCR2 (786 bp) as the amplification template (approximately 10 ng DNA each). The product of fusion PCR (1198 bp) was digested with *Xho*I and *Xba*I*.* The resulting DNA fragment (1160 bp) was used to replace the *Xho*I*-Xba*I fragment of pRT-LpHSFA2 (1335 bp), thus originating the effector plasmid pRT-A2:A9₂.

### 3.1.11 Construction of the pBIB-DS10:3xHA:HaHSFA4:DS10 plasmid.

The HaHSFA4 cDNA was amplified by PCR from pBI221::HaHSFA4. In this step, an *Xba*I site (located 3 bp before the ATG) and a *Sal*I site (located 5 bp after the STOP codon) were introduced with the oligonucleotides 5'-GTTGTTGGTATATCTAGATCAATGATGAATGATGTTCATGG-3' and 5'-GTAAATTTAGACAGTCGACCATTATCAACTTCTCTCTACTG-3' (with an annealing temperature of 67°C). The amplified DNA (1215 bp) was digested with *Xbal* and *Sal*I*.* The resulting fragment (1178 bp) was introduced between the *Xbal* and *Sal*I sites of the pUC19-35S:HA vector (Tejedor *et al.,* 2010), thus generating pUC- 35S-3xHA:HaHSFA4. The 3xHA:HaHSFA4 cassette was amplified by PCR from this plasmid with the oligonucleotides 5'-TCTAGTAAAAATGGCATACC-3' and 5'-TTATCAACTTCTCTCTACTG-3' (with an annealing temperature of 45°C). The amplified 1339 bp fragment was introduced in the Klenow-filled *EcoR*I site of pSK-ds10EC1 (Rousselin *et al.,* 2002), thus originating the pSK-ds10EC1:3xHA:HaHSFA4 plasmid. This plasmid was digested with *Sal*I and *Xbal,* and the resulting 4967 bp fragment was cloned between the corresponding sites of pBIB-Hyg (Becker, 1990).

### 3.2 Results

### 3.2.1 Interaction-cloning in yeast of HaHSFA4: a peculiar class A HSF that is co-expressed with HaHSFA9 in sunflower seeds.

We have used an immature-embryo sunflower cDNA library, first to clone HaHSFA9 by interaction with a promoter DNA fragment (Almoguera *et al.,* 2002), then to clone the HalAA27 repressor by protein interaction with HaHSFA9 (Carranco *et al.,* 2010). The same library and yeast two-hybrid cloning strategy was employed here to clone a different HSF also expressed in immature seeds that we named HaHSFA4. As bait we used a mutagenized form of HaHSFA9, which contains 3 amino-acid residue substitutions in the AD that render it unable to activate transcription (HaHSFA9mutAD, Figure 1C, Carranco *et al.,* 2010). We confirmed 5 positive clones that interacted with the bait after screening 1.4 library equivalents (1.16 x 10⁶ cDNAs). Two of them encoded the same HaHSFA4 cDNA. The cloned HaHSFA4 cDNA was incomplete, as it lacked the DBD and other coding and non-coding 5'-flanking sequences. The missing sequences were cloned by 5'-RACE and a full cDNA that contained the complete protein (387 amino acids) and 228 nucleotides of 5'-UTR sequences was obtained (Figure 15A and 22). The complete protein encoded by the HaHSFA4 cDNA could substitute the yeast sole HSF in functional complementation assays, as previously shown for HaHSFA9 (Almoguera *et al.,* 2002). Thus, HaHSFA4 allowed the growth of transformed yeast strains at temperatures up to 33°C. Using a GAL4_{DBD}-HaHSFA4 fusion protein, we could activate transcription, in one-hybrid experiments, about five-fold higher than using a similar HaHSFA9 protein (Figure 23). We conclude that the complete HaHSFA4 encodes a transcriptional activator HSF that is functional in yeast.

Among the different groups of class A HSFs in plants, HaHSFA4 clearly belongs to the A4 family. The A4 HSFs are characterized -among other properties- by the presence of conserved signature sequences (PVHSHS) located immediately after the DBD, and by the presence of nuclear export sequences (NES motif) in the carboxyl-terminal region (see for example Friedberg *et al.,* 2006 and references reviewed by Scharf *et al.,* 2012). The positions where these sequences are found in HaHSFA4 are shown in Figure 15A and Figure 22 (see amino acids 114-119, and 374-381, respectively). The carboxyl-terminal region of HaHSFA4 also contains the AD with two AHA motifs involved in transcriptional activation by different class A HSFs. These motifs, with Acidic, Hydrophobic and Aromatic residues, match the FWxxF/LF/I/L consensus (von Koskull-Döring *et al.,* 2007). However, HaHSFA4 differs from the two HSFA4 factors of Arabidopsis. Such difference involves amino-acid residues (i.e., located in the DBD and OD regions) that are conserved in some HSFA4 factors from plants of families as *Fabaceae* and *Solanaceae,* which are more similar to sunflower than to Arabidopsis (Figure 15B).

HaHSFA4 was the only HSF expressed in immature sunflower embryos that was cloned by two-hybrid interaction in yeast. We note that HaHSFA9 was not cloned here, nor in a previous two-hybrid screening of the same library (Carranco *et al.,* 2010), despite the high abundance of the HaHSFA9 cDNA in the embryo library (Almoguera *et al.,* 2002). This result indicates that HaHSFA9 preferentially interacts with HaHSFA4 rather than with HaHSFA9.

We determined the domains of HaHSFA9 involved in two-hybrid interaction with HaHSFA4. These include the OD, which is normally required for homo- or hetero-oligomerization of HSFs. Thus for example, the DBD and the N-terminal extension of HaHSFA9 without the OD did not allow interaction with HaHSFA4 (see bait construct 3, Figure 1 C). This contrast with the interaction that showed HaHSFA9 forms that contained the OD and sequences (LNK) that link the OD and DBD, but did not contain the DBD or the C-terminal sequences with the AD (see bait constructs 2 and 4, Figure 15C). Therefore neither the DBD of the C-terminal AD are required for two-hybrid interaction.

### 3.2.2 In planta interaction between HaHSFA9 and HaHSFA4.

We first confirmed *in vitro* a direct physical interaction between HaHSFA9 and HaHSFA4. Thus, GST pull-down experiments showed that the interaction between HaHSFA4 and recombinant forms of HaHSFA9 that contained the OD was much more efficient than the interaction of HaHSFA4 with a protein that only contains the C-terminal region of HaHSFA9 with the AD (Figure 24).

Using BiFC assays, we have confirmed that the interaction between HaHSFA4 and HaHSFA9 occurs in plant nuclei. In Figure 16A, we show results of assays performed in *N. benthamiana* leaves, see further below for additional assays performed in sunflower embryos. We also could detect BiFC interaction between HaHSFA4 and HaHSFA4, this time occurring both in the cytosol and in the nucleus (Figure 16B). The *in planta* BiFC interaction between HaHSFA9 and HaHSFA9 is nuclear (Figure 16C). However, and in contrast to the efficient and reproducible HaHSFA4-HaHSFA4 and HaHSFA4-HaHSFA9 interactions, we could observe the HaHSFA9-HaHSFA9 interaction only in one of the many experimental repetitions that we made. HaHSFA9 mightthus preferentially interact with HaHSFA4 *in planta.* That possibility is consistent with the specificity indicated by the cloning results of HaHSFA4 in yeast. The bipartite localization of the HaHSFA4-HaHSFA4 interaction (Figure 16B) suggested thatHaHSFA4 is exported from the nucleus to the cytosol; thus the NES motif in HaHSFA4 would be functional. We also investigated the localization of HaHSFA9 and HaHSFA4 in plant cells by confocal microscopy of fusion proteins, which have intrinsic fluorescence in green (EGFP:HaHSFA4) or red (DsRed2:HaHSFA9) light. The results of these experiments (Figure 24) are consistent with those of BiFC assays (Figure 16). Thus we observed nuclear and cytosolic localization of HaHSFA4. We also observed exclusive nuclear localization of HaHSFA9 and only nuclear co-localization of HaHSFA4 and HaHSFA9. Mutagenesis of the NES motif sequences in HaHSFA4 confirmed the relevant role of these sequences for nuclear export of HaHSFA4 (see further below).

### 3.2.3 Synergistic interaction of HaHSFA4 and HaHSFA9 in the transcriptional activation of the seed-specific G1 promoter.

The observed interaction between HaHSFA9 and HaHSFA4 (e.g., Figures 15C and 16) suggested that HaHSFA4 might help HaHSFA9 in the transcriptional activation of sHSP gene promoters in the HSFA9 seed genetic program. We first tested such possibility by transient activation assays of the *Hahsp17.6 G1* (G1) promoter in sunflower. The G1 promoter is strictly embryo-specific and it contains imperfect cis-elements (Heat Stress Elements, HSEs) recognized by HaHSFA9 (Carranco *et al.,* 1997; Almoguera *et al.,* 2002). The results summarized in Figure 17A demonstrate that HaHSFA9 and HaHSFA4 synergistically activate the G1 promoter in bombarded sunflower embryos. Upon co-bombardment of the two HSFs transcriptional activation was higher than the sum of the activations observed with each HSF. Such difference was statistically significant. HaHSFA4 could not activate the G1 promoter in sunflower embryos or leaves. This was surprising, considering that HaHSFA4 showed higher transcriptional activity than HaHSFA9 in yeast (compare Figure 23 and Figure 17). However in leaves, and as observed in embryos, a statistically significant synergisticst transcriptional effect on the G1 promoter was observed upon co-bombardment of HaHSFA9 and HaHSFA4 (Figure 17B). In Table I, we include the statistical analyses for the results of Figure 17.

### 3.2.4 The subcellular localization of HaHSFA4 is changed by co-expression with HaHSFA9 in planta.

The results in Figure 16B and Figure 25 showed that HaHSFA4 was localized both in the cytosol and in the nucleus. The NES sequences in the carboxyl-terminal region of HaHSFA4 would thus be functional and mediate its export from the nucleus to the cytosol. HaHSFA4 seems therefore to differ from other class A4 plant HSFs (as AtHSFA4c), which show exclusive nuclear localization, as their NES is not active (Baniwal *et al.,* 2007). We first confirmed the function of the NES motif in HaHSFA4 by analyzing the effect of 3 amino acid substitutions by alanine of leucine residues at positions 374, 378 and 381, which are crucial for NES activity in similar HSFs (Scharf *et al.,* 2012). These substitutions caused the exclusive nuclear localization of a HaHSFA4 (mutNES):GFP fusion in leaves of *Nicotiana benthamiana* (Figure 18A). In tomato, LpHSFA2, as HaHSFA4, has a functional NES motif that determines nuclear export and a cytosolic-nuclear bipartite localization of the HSF. The interaction and hetero-oligomerization of LpHSFA2 with LpHSFA1 has been shown to change the localization of the former in tomato cells. Furthermore, the synergistic transcriptional activation by these tomato class A HSFs is explained in part by the mentioned subcellular localization change of LpHSFA2 (Chan-Schaminet *et al.,* 2009). Therefore, we next investigated whether the nuclear localized HaHSFA9 (see Figure 16 and Figure 25) could also impair the nuclear export of HaHSFA4. A HaHSFA4:GFP fusion was localized mostly in the cytosol in leaves of *Nicotiana benthamiana* (see Figure 25A). A similar cytosolic localization was observed when the HaHSFA4:GFP fusion was co-expressed with the non-fluorescent YFP^{C} fragment encoded by plasmid pGPTV-II kanII-SPYCE(M) (Waadt *et al.,* 2008; data not shown). In contrast, co-expression of HaHSFA4:GFP with HaHSFA9 caused a substantial increase of the nuclear localization of the former at the expense of a drastic reduction of the GFP signal in the cytosol (Figure 18B). This effect was similar to but not as strong as the localization change of HaHSFA4 caused by mutation of the NES motif (compare Figures 4A and 4B). The change of subcellular localization of LpHSFA2 caused by LpHSFA1 has been shown to require their interaction, which involves LpHSFA1 sequences that include the OD (Chan-Schaminet *et al.,* 2009).

Therefore, we finally explored a similar requirement of HaHSFA9 sequences for the change of subcellular localization of HaHSFA4. We compared the localization of the HaHSFA4:GFP fusion when co-expressed with two non-fluorescent, hybrid LpHSFA2-HaHSFA9 proteins. One such hybrid -A2:A9₁-contains mostly LpHSFA2 sequences (amino acids 1-238, including the DBD, LNK and OD regions) and only the carboxyl-terminal region of HaHSFA9 (amino acids 284-371, including the AD).The A2:A9₁ hybrid has been previously described as HSFA2-A9C (Díaz-Martín *et al.,* 2005). The second hybrid protein -A2:A9₂- contains the DBD of LpHSFA2 (amino acids 1-136) and most of HaHSFA9 (amino acids 164-371, including the LNK, OD and AD regions). We note that the two A2:A9 hybrid proteins lack a NES motif. Our results clearly showed that the hybrid protein A2:A9₂, but not A2:A9₁, caused enhanced nuclear retention of HaHSFA4 to the same extent as observed with the complete HaHSFA9 protein (compare Figures 18B, C and D). We conclude that the interaction between HaHSFA9 and HaHSFA4 causes nuclear retention of HaHSFA4 in a similar way as previously reported for the interaction between LpHSFA1 and LpHSFA2.

### 3.2.5 The synergistic interaction between HaHSFA4 and HaHSFA9 requires the OD of HaHSFA9.

Using LpHSFA2, HaHSFA9, and the two A2:A9 hybrid HFSs employed in Figure 4, we also determined their capacity to synergistically interact in transcriptional assays with HaHSFA4. We could thus compare effects of these HSFs on HaHSFA4 localization (Figure 18) with their synergistic transcriptional effect. The results of this comparison are summarized in Figure 19, and in the statistical analyses included in Supplementary Table 1. The transcriptional effects were analyzed using the *Hahsp17.7 G4* (G4) promoter in transient assays performed with sunflower leaves. The G4 promoter is active in seeds and in heat- or drought-stressed vegetative organs; furthermore, it has a more complex and wider array of imperfect HSEs than the G1 promoter (Almoguera *et al.,* 1998). That characteristic better allows binding and transcriptional activation of the G4 promoter by different HSFs, the G1 promoter being more restrictive than G4 (Rojas *et al.,* 2002). This is indeed consisting with the Results of Figure 19, as HaHSFA9, LpHSFA2, and the two hybrid A2:A9 HSFs could transactivate the G4 promoter to a similar extent. Only the transcription activation by HaHSFA4 was lower than that by the rest of HSFs (see Figure 19). The transcriptional activation by HaHSFA4 added to that observed with each HSF in separate was compared to the results observed after co-bombardment of the respective, different, HSF combination. Difference(s) between the compared values that show statistical significance would denote a synergistic transcriptional effect. That synergistic effect was not observed with LpHSFA2 or with the A2:A9₁hybrid HSF. In contrast, HaHSFA9 and the A2:A9₂ hybrid HSF could clearly synergize with HaHSFA4 for transcriptional activation of the G4 promoter (Figure 19 and Table 1). We conclude that the synergistic interaction shows some specificity for the analyzed HSFs. Furthermore, the impairment of nuclear export of HaHSFA4 by some of these HSFs (Figure 18) parallels their capacity for synergistic transcriptional interaction (Figure 19). There was not such synergistic interaction observed with HSFs unable to impair the nuclear export of HaHSFA4 (for example with LpHSFA2 and the A2:A9₁hybrid HSF). However, the synergistic interaction was observed when the HSFs could impair the nuclear export of HaHSFA4 (for example with the complete HaHSFA9 and with the A2:A9₂ hybrid protein).

### 3.2.6 HaIAA27 interacts with HaHSFA4 and hinders the interaction between HaHSFA4 and HaHSFA9 in sunflower embryos, leading to repression of their synergistic coactivation.

The results of functional interaction (Figures 17 and 19) presented here so far would support the identification of HaHSFA4 as one the additional class A HSF(s), which cooperate with HaHSFA9 in activating the same genetic program of seed longevity. A second characteristic that HaHSFA9 meets - and these additional HSF(s) are expected to meet- is a physical interaction with the HalAA27 protein, which leads to repression of the mentioned genetic program (Carranco *et al.,* 2010). The results presented in Figure 20A confirm that HaHSFA4 showed BiFC interaction with theHalAA27 protein in bombarded immature embryos of sunflower. This is similar to what previously published for HaHSFA9 and HaIAA27; the only difference would be that the interaction with HaHSFA4 occurred both in the cytosol and in the nucleus, whereas the interaction of HalAA27 with HaHSFA9 was strictly nuclear (Carranco *et al.,* 2010). That difference would fit the different subcellular localization of HaHSFA9 (strictly nuclear) and HaHSFA4 (cytosolic and nuclear, see Figure 16 and Figure 25).

The interaction of HalAA27 with HaHSFA9 (or with HaHSFA4) might have effects on their synergistic interaction, besides direct repression effects on each HSF. The hetero-oligomerization of HSFs (Scharf *et al.,* 2012), as well as the interaction of HalAA27 with HaHSFA9 (Carranco *et al.,* 2010) involve the HSF OD. Thus, a possible effect of HalAA27 on the synergism would be that it impairs the interaction between HaHSFA9 and HaHSFA4. Such possibility was analyzed by testing in bombarded sunflower embryos the effect of a stabilized form of HalAA27 (HaIAA27 mIIab, Carranco *et al.,* 2010) on the BiFC interaction between HaHSFA9 and HaHSFA4. The interaction between the two HSFs was detected exclusively in the nucleus, as previously shown in leaves of *N. benthamiana* (compare Figures 16A and 6B). When the HalAA27 mIIab form was co-bombarded with HaHSFA4 and HaHSFA9, the BiFC interaction between the two HSFs was substantially reduced in repeated experiments (Figure 20C).

That HalAA27 represses the synergistic coactivation by HaHSFA9 and HaHSFA4 was tested by transient expression. In these experiments, performed with bombarded sunflower embryos, we used the reporter gene with the G4 promoter. HaHSFA4 could activate the G4 promoter, although to a lower level than observed with HaHSFA9. A very strong synergistic effect of HaHSFA9 and HaHSFA4 was observed. The strong synergistic coactivation observed by co-bombardment of HaHSFA9 and HaHSFA4 was fully abolished by HaIAA27. Indeed, in presence of HaIAA27, HaHSFA9 and HaHSFA4 activated the G4 promoter to the same levels as observed with only HaHSFA9 in absence of the repressor (F= 0.19, P= 0.66). Such drastic effect contrasted with the much moderate reduction of the transcriptional activation by either HaHSFA9or HaHSFA4 that was observed when the effect of HalAA27 was analyzed in separate(respectively, F= 4.35, P= 0.038, and F= 8.9, P= 0.003, see Figure 20D).

The results in Figure 20 (panels A-C) are consistent with the notion that HalAA27 represses the synergism by a passive mechanism: i.e., by steric obstruction, rather than by the involvement of active repression domains in HaIAA27. A passive repression mechanism would also fit other previously published results from our lab. Thus, we reported that overexpression of HalAA27 ΔN, another stabilized form of HalAA27 that lacks domain I -the only known active repression domain of Aux/IAA proteins- caused loss-of-function of the HSFA9 program in transgenic seeds (Carrancoet *al.,* 2010). The repression by the HalAA27 ΔN form has been further analyzed in transient assays performed with bombarded embryos (Supplementary Figure 19). We employed a transient assay system used in the literature for detection and mapping of active repression domains in plant proteins (Hiratsu, *et al.,* 2004; Ikeda and Ohme-Takagi, 2009). The results in Figure 25 indicated that in these assays repression by the HalAA27 ΔN form was negligible compared to that of the intact HalAA27 protein (containing domain I). Both forms were assayed as GALDB fusions; reporter gene activity using the HalAA27 ΔN form fusion was only slightly lower than that with the un-fused GALDB protein (Figure 26). Therefore it is unlikely that the HalAA27 ΔN form, which *in planta* represses the HSFA9 program as efficiently as the intact HalAA27 protein, contains an unmapped active repression domain.

### 3.2.7 Functional validation of HaHSFA4 in transgenic tobacco plants: enhanced seed longevity in plants that over-express HaHSFA9 and HaHSFA4.

From homozygous lines that over-express HaHSFA9 specifically in tobacco seeds, the DS10:A9 lines (Prieto-Dapena *et al.,* 2006), we have obtained lines that combine seed-specific over-expression of HaHSFA9 and HaHSFA4: the DS10:A9/A4 lines. Taking advantage of Mendelian segregation (see Methods) we obtained 7different sibling pairs of DS10:A9 (single-homozygous) and DS10:A9/A4 (double-homozygous) lines. These sibling pairs represent different single-integration events of the DS10:A9 and DS10:A4 transgenes; in each case the DS10:A9/A4 line differs from it sibling DS10:A9 only by the presence of the DS10:A4 transgene in homozygosis (in a different genetic background for each pair). We investigated whether the over-expression of HaHSFA4 in the DS10:A9/A4 lines enhances resistance to accelerated aging, a measure of seed longevity. We compared seeds from the different DS10:A9/A4 lines with seeds from the respective sibling DS10:A9 lines. We performed accelerated aging procedures similar to that used in our earlier studies, except that the aging temperature was increased from 50°C to 52°C. The results of the experiments summarized in Figure 21, clearly show a statistically significant increase of the resistance to accelerated aging of the DS10:A9/A4 lines compared to the sibling DS10:A9 lines (F= 32.95, P< 0.0001, 1 and 831 df). We thus demonstrate in transgenic plants that the combined overexpression of HaHSFA4 and HaHSFA9 enhanced seed-longevity beyond what reported for the overexpression of HaHSFA9 in separate (Prieto-Dapena *et al.,* 2006). These results confirm that HaHSFA4 and HaHSFA9 coactivate the same genetic program of seed longevity.

### 3.3 Discussion

### 3.3.1 The synergistic interaction of a pair of class A HSFs, HaHSFA9 and HaHSFA4, enhances seed longevity.

In this report we found that HaHSFA4, a class A HSF expressed in immature embryos of sunflower seeds interacts with HaHSFA9, leading to synergistic transcriptional activation of seed sHSP gene promoters. HaHSFA9 is the seed-specific class A HSF (Almoguera *et al.,* 2002) that we previously characterized as involved in transcriptional activation of a genetic program of seed longevity (Prieto-Dapena *et al.,* 2006) and desiccation tolerance (Prieto-Dapena *et al.,* 2008). Additional work in our lab indicated the existence of potentially other HSFs necessary for the activation of the HaHSFA9 program (Tejedor-Cano *et al.,* 2010; Carranco *et al.,* 2010). HaHSFA4, cloned by protein-interaction with HaHSFA9 in yeast (Figure 15) would be one such additional HSF. The *in planta* nuclear interaction with HaHSFA9 (Figures 16A and 16B) and, moreover, the statistically significant synergistic transcriptional effects observed with HaHSFA9 on the sHSP promoters, in particular in sunflower embryos and on the seed-specific G1 promoter, meet for HaHSFA4 the expected requirements (Figures 17 and 20D). Furthermore, a functional *in planta* verification of HaHSFA4, as a co-activator of the HaHSFA9 program, was obtained in this report. We thus could show that the combined overexpression of HaHSFA9 and HaHSFA4 in tobacco seeds further enhanced longevity, in comparison with the effects of HaHSFA9 alone (Figure 21). HaHSFA9 and HaHSFA4 therefore synergistically co-activate an embryonic program of seed longevity. This resembles results from studies in animal systems. In these systems, pairs of different HSFs, as HSF1 and HSF2, showed analogous protein and functional interactions that are relevant for the control of developmental gene expression during embryogenesis (Ostling *et al.,* 2007; Abane and Mezger, 2010). Interestingly these interactions also occur on promoters, which as the G1 and G4 promoters analyzed in our study contain gapped and imperfect HSE cis-elements (Abane and Mezger, 2010).

In plant systems at least three pairs of Class A HSFs have been found to specifically interact: LpHSFA1-LpHSFA2 (Chan-Schaminet *et al.,* 2009), LpHSFA4b-LpHSFA5 (Baniwal *et al.,* 2007), and AtHSFA1a-AtHSFA1b (Li *et al.,* 2010). Some of these interactions had distinct transcriptional effects, either leading to synergistic transcriptional activation (Chan-Schaminet *et al.,* 2009) or to repression (Baniwal *et al.,* 2007). All the previously analyzed interactions involve "vegetative HSFs" (constitutive or heat-induced) and thus would be relevant mostly for heat-, and different, stress responses in organs other than seeds. These HSF interactions involve protein domains and activation mechanisms that are compatible with our observations for HaHSFA4 and HaHSFA9. For example, it has been shown that the OD of Class A HSFs is necessary to determine pairwise interactions of these transcription factors (Chan-Schaminet *et al.,* 2009; Li *et al.,* 2010), and that the OD defines the specificity of the synergism between LpHSFA1-LpHSFA2 (Chan-Schaminet *et al.,* 2009). In agreement with this, we found that the OD of HaHSFA9 is required for the synergistic activation obtained with HaHSFA4 and HaHSFA9 in transient expression in sunflower. Thus, the synergism was observed only with HaHSFA9 (e.g., not LpHSFA2) and the LpHSFA2:HaHSFA9 hybrid HSF (A2:A9₂) that contains the OD of HaHSFA9 (Figure 19). It has been proposed that combination of distinct ADs (with dissimilar AHA motif number and sequences) from different HSFs in part explains the synergistic activation by LpHSFA1 and LpHSFA2 (Chan-Schaminet *et al.,* 2009). That might also apply to the synergism between HaHSFA9 and HaHSFA4. On the other hand a change in the subcellular localization of LpHSFA2 is a component of the mechanism of the synergistic activation by LpHSFA1 and LpHSFA2. Thus, when LpHSFA1 interacts with LpHSFA2 in the nucleus somehow it impairs the nuclear export of LpHSFA2, most likely by directly or indirectly hindering the NES of LpHSFA2 (Chan-Schaminet *et al.,* 2009). The results shown in Figures 18 and 19 would support a similar mechanistic explanation for the synergism between HaHSFA9 and HaHSFA4. Only HaHSFA9 and the LpHSFA2:HaHSFA9, hybrid HSF, A2:A9₂, when co-expressed with HaHSFA4, caused an enhanced nuclear localization of HaHSFA4 (Figure 18) that parallels the observation of synergistic transcription coactivation using the mentioned HSFs (Figure 19).

In summary, the novel HaHSFA4-HaHSFA9 synergistic interaction occurs by mechanisms similar to those described for other Class A HSFs and it is relevant for seed functions, as longevity.

### 3.3.2 Auxin could enhance the HaHSFA9 program by alleviating the repression by HaIAA27, which in turn would facilitate the synergism between HaHSFA9 and HaHSFA4.

HaHSFA4 differs from other plant HaHSFA4s in its little transcriptional activity, at least in planta (Figures 17, 19). It is surprising that in the, heterologous, yeast system HaHSFA4 showed even a much higher activity than HaHSFA9 (Figure 23). That difference would be consistent with negative regulation of the HaHSFA4 transcriptional activity. This would occur by mechanisms that are plant-specific, for example by repression involving Aux/IAA proteins. The loss of function of the HaHSFA9 program was also possible using stabilized forms of the Aux/IAA protein HalAA27 (Carranco *et al.,* 2010). From that result we inferred that HalAA27 would repress not only HaHSFA9, but also the additional HSF(s) involved in transcriptional activation of the HaHSFA9 program. The results reported here show that HaHSFA4 interacts with HalAA27 in sunflower embryos (Figure 20A); HalAA27 also repressed HaHSFA4, furthermore it abolished the synergistic transcriptional effect of HaHSFA4 and HaHSFAA9 on the G4 promoter (Figure 20D). These results further confirmed that HaHSFA4 is an additional HSF that, together with HaHSFA9, activates the same genetic program of seed longevity. The interactions of HaHSFA9 with either HaHSFA4 (e.g., Figure 15C, and Figure 17) or with HalAA27 (Carranco *et al.,* 2010) required in both cases the OD of HaHSFA9. Therefore, HaIAA27, by interacting with HaHSFA9 (or with HaHSFA4) it could hinder the interaction between HaHSFA4 and HaHSFA9 and thus impair the synergistic transcriptional activation by the two HSFs. Negative effects of HaIAA27, on both the interaction and the synergistic activation between these HSFs, were indeed observed in assays performed with bombarded sunflower embryos (Figure 20). The hindering of that interaction by HalAA27 (Figures 20B and C) and the efficient repression of the HSFA9 program by HalAA27 ΔN (Carranco *et al.,* 2010), which lacks an active repression domain (Figure 26), indicate that the effects of HalAA27 mainly involve a passive repression mechanism. As auxin induces the destabilization of HalAA27 (Carranco *et al.,* 2010), we propose that auxin would relieve the HalAA27-mediated repression of the synergism between HaHSFA9 and HaHSA4.

### 33.4 Conclusion

We conclude that in sunflower a genetic program of seed longevity is synergistically co-activated by HaHSFA9 and HaHSFA4. A novel synergistic interaction of two different Class A HSFs has thus relevance for seed functions, including longevity and desiccation tolerance. This novel transcriptional synergistic interaction occurs in seed embryos by mechanisms that require the OD of HaHSFA9; these mechanisms involve a HaHSFA9-induced nuclear retention of HaHSFA4. HalAA27 obstructs the interaction and synergistic coactivation that involves HaHSFA9 and HaHSFA4. Auxin, by destabilizing HalAA27 might relieve that passive repression and in turn might facilitate the synergistic interaction of the two HSFs in embryos.

### 3.4.1 Constitutive over-expression of HaHSFA4 in transgenic tobacco (alone or combined with HaHSFA9).

A 3xHA-tagged form of HaHSFA4 was integrated in a binary plasmid derived from the pBIB-Hyg vector (Becker, 1990) and the pUC-35S-3xHA:HaHSFA4 plasmid described herein. A 2284 pb, Hind*III-*Kpn*I*, DNA fragment excised from pUC-35S-3xHA:HaHSFA4 was cloned between the Hind*III* andKpn/sites of pBIB-Hyg. As a result, we obtained pBIB-Hyg-35S-3xHA:HaHSFA4, the tagged 35S:A4 binary plasmid.

### 3.4.2 Generation of the tobacco single-transgenic lines 35S:A4.

We transformed tobacco (*Nicotiana tabacum* L. var. Xanthi) with the tagged 35S:A4 binary plasmid. Using the procedures described for the selection of 35S:A9 line pairs (Prieto-Dapena *et al.,* 2006), we similarly obtained 35S:A4₁, NT₁; 35S:A4₂, NT₂, and 35S:A4₃, NT₃. These pairs include three transgenic lines with, different, single integration events of the A4 transgene in homozygosis (A4₁, A4₂, and A4₃) and the corresponding syngenic (non-transgenic) lines: NT₁, NT₂ and NT₃, obtained by segregation from the parental heterozygous lines.

### 3.4.3 Generation of the tobacco double-transgenic lines 35S:A9/A4.

Three homozygous transgenic lines that overexpress HaHSFA9 from 35S CaMV sequences were transformed with the 35S:A4 binary plasmid. The parental transgenic lines were previously described as 35S:A9#2-18, 35S:A9#17-8 and 35S:A9#12-4 (Prieto-Dapena *et al.,* 2006). In Prieto-Dapena *et al.,* (2006), we also described procedures for tobacco transformation and for the selection of transgenic lines. We first obtained heterozygous 35S:A4 lines (with single integration events) in the three homozygous 35S:A9 backgrounds. The double-homozygous 35S:A9/A4 lines were obtained by segregation in the subsequent generation. We also selected for the sibling 35S:A9 lines, which were used as the proper, single-transgenic control, lines. These procedures are described in a draft manuscript, where we analyzed the combined A9/A4 effects on seed longevity (Tejedor-Canoet *al.,* in preparation). We thus obtained 4 pairs of sibling lines (each with a different A9 background): 35S:A9₁/A4ₐ, 35S:A9₁; 35S:A9₁/A4_{b}, 35S:A9₁; 35S:A9₂/A4, 35S:A9₂, and 35S:A9₃/A4, 35S:A9₃. The A9₁, A9₂ and A9₃ backgrounds correspond to 35S:A9#2-18, 35S:A9#17-8 and 35S:A9#12-4, respectively. In the A9₁ background we obtained two different double-homozygous lines, 35S:A9₁/A4ₐ and 35S:A9₁/A4_{b}.

### 3.4.4 HaHSFA4, by itself, does induce HSP accumulation, thermotolerance, or resistance to oxidative stress.

We analyzed possible effects by A4 on stress tolerance using the 35S:A4 lines (Figure 4). These effects were studied in 3-4 week-old seedlings grown on Petri dishes with MS media. Basal thermotolerance to high temperature (48°C) and tolerance to drastic oxidative stress (200mM H₂O₂) was analyzed used procedures that have been described in detail for similar analyses of 35S:A9 lines (Prieto-Dapena *et al.,* 2008; Almoguera *et al.,* 2012). The two analyses of stress tolerance in Figure27 include data, each, from 3 independent experiments performed with samples from the 3 different transgenic/NT pairs: 35S:A4₁, NT₁; 35S:A4₂, NT₂, and 35S:A4₃, NT₃. For the thermotolerance assays (Figure 27A) we used 1 Petri dish per line (A4 or NT), condition (48°C with/without acclimation) and experiment (n= 9, n= 36 in total). For each H₂O₂ tolerance experiment, we used 1 dish per line for the control treatments, and 2 dishes per line for the 200mM H₂O₂ treatment (n= 9 for control and n= 18 for 200 mM H₂O₂, n= 54 in total).

Non-transgenic tobacco seedlings do not withstand lethal heat stress treatments for 2 h at 48°C, whereas the same treatment is resisted by 35S:A9 seedlings (Prieto-Dapena *et al.,* 2008). In contrast, Figure 27A shows that the 35S:A4 seedlings (with a representative result for the 35S:A4₁ line) are killed by the 48°C treatment. Figure 4A also shows that the 35S:A4₁ line resists 48°C treatment only after a heat-acclimation treatment for 3h at 40°C. This is similar to what observed for non-transgenic tobacco seedlings (Prieto-Dapena *et al.,* 2008). Therefore, A4 does not induce basal thermotolerance (resistance to HS).

The results in Figure 4B showed that after treatments for 24h with 200mM H₂O₂ in the dark, the Fv/Fm of the 35S:A4 seedlings reached statistically similar values (F= 0.54, P= 0.47) as those for the sibling NT seedlings. Consequently, A4 does not induce the resistance to drastic oxidative stress that we previously observed for A9 (Almoguera *et al.,* 2012).

In Figure 4C, we investigated if the 35S:A4 seedlings (3-week old) accumulate at normal growth temperatures (20-25°C) different classes of Heat Shock Proteins (HSP). The analyzed HSP are not expressed without heat stress (HS) in non-transgenic tobacco and their accumulation in 35:A9 seedlings correlates with their reported stress resistance (Prieto-Dapena *et al.,* 2008; Almoguera *et al.,* 2012). Western blot analyses, with protein-extract samples from the three 35S:A4 lines pairs and the indicated HSP class-specific antibodies were performed essentially as described (Prieto-Dapena *et al.,* 2008; Almoguera *et al.,* 2012). As a positive control we also included a sample from the heat-acclimated NT₁ line used in panel 4A.We could not detect A4-induced accumulation of any of the analyzed HSP in the transgenic 35S:A4 lines. This includes HSP101, cytosolic sHSP of classes CI and CII (sHSP-CI and sHSP-CII, respectively) and chloroplast-localized sHSP (sHSP-P). Accumulation of the HA-tagged A4 factor was detected in the different 35S:A4 samples, but not in samples from the corresponding sibling NT lines. As expected, the analyzed HSP could not be detected in the NT samples. In contrast, accumulation of all the analyzed HSP was detected in the heat-acclimated NT₁, sample (HS). Thus A4 did not induce the accumulation of the analyzed HSP in seedlings.

We note that A4 differs from A9 and from other activator Class-A Heat Stress Factors (HSF) of plants in that it does not induce the accumulation of sHSP-CI, -CII, -P, and HSP101 in seedlings at normal growth temperatures. It is also unusual the incapacity of A4 (when assayed without A9 or any other HSF) to confer any HS resistance (Figure 27A). However, these results agree with our transient expression analyses using sHSP-Cl promoters in sunflower. These analyses showed that *in planta* A4 had very little (if any at all) transcriptional activity by itself. In contrast, A4 assayed together with A9 showed a synergistic transcriptional effect not only in embryos but also in leaves (Tejedor-Cano *et al.,* in preparation).

### 3.4.5 HaHSFA4 combined with HaHSFA9 further enhances resistance to drastic dehydration and oxidative stress conditions.

We determined whether the combined overexpression of A9 and A4 in transgenic tobacco enhances the stress tolerance observed upon the single overexpression of A9 (Prieto-Dapena *et al.,* 2008). We performed severe dehydration (DT2, Prieto-Dapena *et al.,* 2008) and oxidative stress treatments with H₂O₂ in the dark for 24h, using conditions essentially as described, respectively. The H₂O₂ concentration was increased from 200 mM to 300 mM, to decrease survival of the 35S:A9 after the oxidative stress treatments. The analyses summarized in Figure 28 where performed with 3 to 4 week-old seedlings grown on MS medium in Petri dishes; these analyses involved several independent experimental repeats, each one including samples (Petri dishes, in equal numbers) from the 4 sibling pairs of single-transgenic (35S:A9) and double-transgenic (35SA9/A4) lines: 35S:A9₁/A4ₐ, 35S:A9₁; 35S:A9₁/A4_{b}, 35S:A9₁; 35S:A9₂/A4, 35S:A9₂, and 35S:A9₃/A4, 35S:A9₃. To facilitate presentation, we grouped and averaged the results obtained with the four 35S:A9 (A9) lines and the four 35S A9/A4 (A9/A4) lines in each set of experiments (in Figures 28A and 28B). The statistical analysis of differences between the grouped data was consistent with the results obtained when the differences were separately analyzed for each sibling line pair. For the included ANOVA analyses see above. For the t-Student analyses, used for non normal-distributed data, see Carranco *et al.,* (2010). The dehydration tolerance analyses of Figure 28A correspond to 18, independent, full experiments (n= 162). The H₂O₂ tolerance analyses of Figure 28B correspond to 10, independent, full experiments (n= 100).

The combined over-expression of A9 and A4 in the A9/A4 lines substantially enhanced survival of whole seedlings after either stress treatment: severe dehydration (see Figure 28A), or 300 mM H₂O₂ stress (see Figure 28B). In both cases survival of whole seedlings more than doubled compared with that of sibling A9 lines. These differences were statistically highly significant (Figure 28A, t= -3.59, P= 0.0004; Figure 5B, t= -2.59, P= 0.01. See "seedling survival"). The surviving A9/A4 whole seedlings represented slightly above 12% of the initial amount of seedlings. However, in most seedlings only some leaves resisted the stress treatments. Survival after dehydration of 1-4 leaves per seedling (see Figure 28A, "total leaf survival") was also higher for the A9/A4 lines compared to the A9 lines (t= -4.82, P< 0.0001). After the 300 mM H₂O₂ stress treatments only a maximum of 2 true leaves per seedling survived; this survival (Figure 28B, see "2-leaf survival",) was higher for the A9/A4 lines compared to the A9 lines (t= -4.87, P< 0.0001).As a representative example of the experimental results in Figure 5B, Figure 5C depicts differential survival of seedlings and leaves between to dishes with A9₁ or A9₁/A4ₐ material.

Enhanced protection of the PSII Fv/Fm in the A9/A4 lines compared with sibling A9 lines was also observed. The respective average values determined after the 300 mM H₂O₂ stress treatments were 454.73 ± 8.24 and 402.74 ± 7.8 (F= 23.21, P= 0.0001). We note that after normal 200 mM H₂O₂ treatments, there was not difference between the Fv/Fm of A9/A4 and A9 plants: 477.2 ± 11.9 and 487.77 ±9.88, respectively (F= 0.236, P= 0.63). The additional protection of Fv/Fm conferred by the A9+A4 combination is thus observed only with the stronger 300 mM H₂O₂ treatments. The data of statistical analyses (given here for each comparison in brackets) showed that the all differences between the A9/A4 and A4 lines indicated in Figure 28 or in the text are statistically significant. Therefore A4, in combination with A9, further enhances the stress resistance conferred by the single overexpression of A9. This is shown for tolerance to severe dehydration (Figure 28A) and for tolerance to drastic oxidative stress (Figure 28B).

### 3.4.6 HaHSFA4 combined with HaHSFA9 enhances recovery from dark induced senescence.

We analyzed whether the combined overexpression of A9 and A4 also enhances the protection from dark induced senescence observed upon the single overexpression of A9. For the experiments of Figure 29, we used Petri dishes with 3-week-old seedlings grown on MS media. The dark treatment procedure was exactly as described in the detail for the experiments with the single transgenic 35S:A9 lines, except that it was maintained for 5 weeks. We performed two independent experiments, each one including Petri dishes with seedlings from the 4 sibling pairs of single-transgenic (35S:A9) and double-transgenic (35SA9/A4) lines: 35S:A9₁/A4ₐ, 35S:A9₁; 35S:A9₁/A4_{b}, 35S:A9₁; 35S:A9₂/A4, 35S:A9₂, and 35S:A9₃/A4, 35S:A9₃. In experiment 1 (Figure 29A), we used one Petri dish per individual transgenic line (n= 8). In experiment 2 (Figure 29B), we used two Petri dishes per individual transgenic line (n= 16). In experiment 1, the Fv/Fm and the total chlorophyll content was measured immediately after the dark treatment (as above). Once finished the dark treatments, the Petri dishes were transferred to normal growth conditions under photoperiod illumination for two weeks and pictures were taken at the end of the experiments (Figure 6).

In both experiments we observed that the double-transgenic seedlings (A9/A4) recovered much better than the single transgenic seedlings (A9). This is illustrated with representative results for the A9₃ and A9₃/A4 seedlings in experiment 1 (Figure 29A), and with results for the A9₁ and A9₁/A4ₐ seedlings in experiment 2 (Figure 29B). The preliminary measurements of the PSII Fv/Fm and the total chlorophyll content in experiment 2 indicate that after 5 weeks under dark both the A9 and the A9/A4 are damaged to same extent. Effects of the A9+A4 on senescence damage repair, rather than on senescence damage protection thus may explain the enhanced recovery of the A9/A4 seedlings.

**Table 1**

| | **luc/Rluc+SE** | | |
|---|---|---|---|
| **TF combination** | **Co-bomb** | **Sum** | **ANOVA** |
| **A9+A4 [****Fig. 3****, Embryos]** | 262.8±19.6 | 143.1±8.82 | F=56.61, **P=0.0001** |
| **A9+A4 [****Fig. 3****, Leaves]** | 40.13±3.48 | 30.46±3.65 | F=7.811, **P=0.006** |
| **A9+A4 [****Fig. 5****]** | 49.14±2.86 | 24.42±0.66 | F=100.2, **P=0.0001** |
| **A2:A92+A4 [****Fig. 5****]** | 30.22±2.94 | 21.65±1.56 | F=6.273, **P=0.013** |
| A2:A91+A4 [Fig. 5] | 21.88±1.50 | 18.79±1.29 | F=0.682, P=0.410 |
| A2+A4 [Fig. 5] | 20.68±1.75 | 20.08±0.99 | F=0.026, P=0.871 |
| **A9+A4 [Fig. 6D]** | 741.1±69.7 | 290.2±27.7 | F=23.09, **P=0.0001** |

**Table 1**. Statistical analysis of the synergistic interactions observed by transient expression in sunflower. Data from the indicated TF combinations and Figures were analyzed following the logarithmic normalization and one-way ANOVA procedures previously described (Almoguera *et al.,* 1998; Rojas *et al.,* 2002). When indicated, we show the SE and mean reporter activity (luc/Rluc) obtained after co-bombardment (Co-bomb) and from the sums of the activities separately induced by the corresponding TFs. Note that from each sum of two individual activity values, the basal level (activity without TF) was subtracted once. Sample sizes are similar to those indicated in the corresponding Figures, except for few points removed to improve normalization. The statistic (F) and probability (P) values are shown for each comparison between the Co-bom and Sum mean activities. Statistically significant differences (P< 0.05) are indicated with bold face.

### REFERENCES

1. Prieto-Dapena P, Castaño R, Almoguera C, Jordano J (2006) Improved resistance to controlled deterioration in transgenic seeds. Plant Physiol 142: 1102-1112.
2. Prieto-Dapena P, Castaño R, Almoguera C, Jordano J (2008) The ectopic overexpression of a seed-specific transcription factor, HaHSFA9, confers tolerance to severe dehydration in vegetative organs. Plant J 54:1004-1014.
3. Tejedor-Cano J, Prieto-Dapena P, Almoguera C, Carranco R, Hiratsu K, et al. (2010) Loss of function of the HSFA9 seed longevity program. Plant Cell Environ 33:1408-1417.
4. Sugliani M, Rajjou L, Clerkx EJM, Koornneef M, Soppe WJJ (2009) Natural modifiers of seed longevity in the Arabidopsis mutants abscisic acid insensitive3-5 (abi3-5) and leafy cotyledon1-3 (lec1-3). New Phytol 184:898-908.
5. Almoguera C, Rojas A, Diaz-Martin J, Prieto-Dapena P, Carranco R, et al. (2002) A seed-specific heat-shock transcription factor involved in developmental regulation during embryogenesis in sunflower. J Biol Chem 277:43866-43872.
6. Kotak S, Vierling E, Bäumlein H, Koskull-Döring P (2007) A Novel transcriptional cascade regulating expression of heat stress proteins during seed development of Arabidopsis. Plant Cell 19:182-195.
7. Barua D, Downs CA, Heckathorn SA (2003) Variation in chloroplast small heat-shock protein function is a major determinant of variation in thermotolerance of photosynthetic electron transport among ecotypes of Chenopodium album. Funct Plant Biol 30:1071-1079.
8. Neta-Sharir I, Isaacson T, Lurie S, Weiss D (2005) Dual role for tomato heat shock protein 21: Protecting photosystem II from oxidative stress and promoting color changes during fruit maturation. Plant Cell 17:1829-1838.
9. Guo SJ, Zhou HY, Zhang XS, Li XG, Meng QW (2007) Overexpression of CaHSP26 in transgenic tobacco alleviates photoinhibition of PSII and PSI during chilling stress under low irradiance. J Plant Physiol 164:126-136.
10. Allakhverdiev SI, Kreslavski VD, Klimov VV, Los DA, Carpentier R, et al. (2008) Heat stress: an overview of molecular responses in photosynthesis. Photosynth Res 98:541-550.
11. Sonoike K (2011) Photoinhibition of photosystem I. Physiol Plant 142:56-64.
12. Deng X, Hu ZA, Wang HX, Wen XG, Kuang TY (2003) A comparison of photosynthetic apparatus of the detached leaves of the resurrection plant Boea hygrometrica with its non-tolerant relative Chirita heterotrichia in response to dehydration and rehydration. Plant Sci 165:851-861.
13. Georgieva K, Röding A, Büchel C (2009) Changes in some thylakoid membrane proteins and pigments upon desiccation of the resurrection plant Haberlea rhodopensis. J Plant Physiol 166:1520-1528.
14. Aro EM, Virgin I, Andersson B (1993) Photoinhibition of Photosystem II. Inactivation, protein damage and turnover. Biochim Biophys Acta 1143:113-134.
15. Nixon PJ, Michoux F, Yu J, Boehm M, Komenda J (2010) Recent advances in understanding the assembly and repair of photosystem II. Ann Bot (London) 106:1-16.
16. Dinakar C, Djilianov D, Bartels D (2012) Photosynthesis in desiccation tolerant plants: Energy metabolism and antioxidative stress defense. Plant Sci 182:29-41.
17. Lundin B, Nurmi M, Rojas-Stuetz M, Aro EM, Adamska I et al. (2008) Towards understanding the functional difference between the two PsbO isoforms in Arabidopsis thaliana-insights from phenotypic analyses of psbo knockout mutants. Photosynth Res 98:405-414.
18. Armbruster U, Zuhlke J, Rengstl B, Kreller R, Makarenko E, et al. (2010) The Arabidopsis thylakoid protein PAM68 is required for efficient D1 biogenesis and photosystem II assembly. Plant Cell 22:3439-3460.
19. García-Cerdán JG, Kovács L, Tóth T, Kereïche S, Aseeva E, et al. (2011) The PsbW protein stabilizes the supramolecular organization of photosystem II in higher plants. Plant J 65:368-381.
20. Miyao M, Ikeuchi M, Yamamoto N, Ono T (1995) Specific degradation of the D1 protein of photosystem II by treatment with hydrogen peroxide in darkness: implications for the mechanism of degradation of the D1 protein under illumination. Biochemistry 34:10019-10026.
21. Nishiyama Y, Allakhverdiev SI, Murata N (2011) Protein synthesis is the primary target of reactive oxygen species in the photoinhibition of photosystem II. Physiol Plant 142:35-46.
22. Aro EM, Suorsa M, Rokka A, Allahverdiyeva Y, Paakkarinen V, et al. (2005) Dynamics of photosystem II: a proteomic approach to thylakoid protein complexes. J Exp Bot 56:347-356.
23. Lu Y, Hall DA, Last RL (2011) A small zinc finger thylakoid protein plays a role in maintenance of photosystem II in Arabidopsis thaliana. Plant Cell 23:1861-1875.
24. Härndahl U, Hall RB, Osteryoung KW, Vierling E, Bornman JF, et al. (1999) The chloroplast small heat shock protein undergoes oxidation-dependent conformational changes and may protect plants from oxidative stress. Cell Stress Chaperones 4:129-138.
25. Osteryoung KW, Vierling E (1992) Genetic approaches to the function of the chloroplast low molecular weight heat shock protein. In: Murata N, editor. Research in Photosynthesis. Kluwer Academic Publisher, Vol IV, pp. 129-136.
26. Nakamoto H, Vígh L (2007) The small heat shock proteins and their clients. Cell Mol Life Sci 64:294-306.
27. Lepistö A, Kangasjärvi S, Luomala EM, Brader G, Sipari N, et al. (2009) Chloroplast NADPH-thioredoxin reductase interacts with photoperiodic development in Arabidopsis. Plant Physiol 149:1261-1276.
28. Markwell MA, Haas SM, Bieber LL, Tolbert NE (1978) A modification of the Lowry procedure to simplify protein determination in membrane and lipoprotein samples. Anal Biochem 87:206-210.
29. Cline K, Mori H (2001) Thylakoid DeltapH-dependent precursor proteins bind to a cpTatC-Hcf106 complex before Tha4-dependent transport. J Cell Biol 154:719-729.
30. Neuhoff V, Arold N, Taube D, Ehrhardt W (1988) Improved staining of proteins in polyacrylamide gels including isoelectric focusing gels with clear background at nanogram sensitivity using Coomassie Brilliant Blue G-250 and R-250. Electrophoresis 9:255-262.

Kim DH, Xu ZY, Na YJ, Yoo YJ, Lee J, Sohn EJ, Hwang I (2011) Small heat shock protein Hsp17.8 functions as an AKR2A cofactor in the targeting of chloroplast outer membrane proteins in Arabidopsis. Plant Physiol 157: 132-146
Buchanan-Wollaston V, Earl S, Harrison E, Mathas E, Navabpour S, Page T, Pink D (2003) The molecular analysis of leaf senescence-a genomics approach. Plant Biotechnol J 1: 3-22
Denev I, Stefanov D, Terashima I (2012) Preservation of integrity and activity of Haberlea rhodopensis photosynthetic apparatus during prolonged light deprivation. Physiol Plant 146: 121-128
Dinakar C, Djilianov D, Bartels D (2012) Photosynthesis in desiccation tolerant plants: Energy metabolism and antioxidative stress defense. Plant Sci 182: 29-41
Abane R, Mezger V (2010) Roles of heat shock factors in gametogenesis and development. Febs J 277: 4150-4172
Almoguera C, Prieto-Dapena P, Jordano J (1998) Dual regulation of a heat shock promoterduringembryogenesis: stage-dependent role of heat shock elements. Plant J 13: 437-446
Almoguera C, Prieto-Dapena P, Díaz-Martín J, Espinosa JM, Carranco R, Jordano J (2009) The HaDREB2 transcription factor enhances basal thermotolerance and longevity of seeds through functional interaction with HaHSFA9. BMC PlantBiol9: 75
Baniwal SK, Chan KY, Scharf KD, Nover L (2007) Role of heat stress transcription factor HsfA5 as specific repressor of HsfA4. J BiolChem282: 3605-3613
Becker D (1990) Binary vectors which allow the exchange of plant selectable markers and reporter genes. NucleicAcids Res 18: 203
Buchanan-Wollaston V, Earl S, Harrison E, Mathas E, Navabpour S, Page T, Pink D (2003) The molecular analysis of leaf senescence-a genomics approach. Plant Biotechnol J 1: 3-22
Carranco R, Almoguera C, Jordano J (1997) A plant small heat shock protein gene expressed during zygotic embryogenesis but noninducible by heat stress. J BiolChem272: 27470-27475
Carranco R, Espinosa JM, Prieto-Dapena P, Almoguera C, Jordano J (2010) Repression by an auxin/indole acetic acid protein connects auxin signaling with heat shock factor-mediated seed longevity. Proc Natl Acad Sci U S A 107: 21908-21913
Chan-Schaminet KY, Baniwal SK, Bublak D, Nover L, Scharf KD (2009) Specific interaction between tomato HsfA1 and HsfA2 creates hetero-oligomeric superactivator complexes for synergistic activation of heat stress gene expression. J Biol Chem 284: 20848-20857
Davletova S, Rizhsky L, Liang H, Shengqiang Z, Oliver DJ, Coutu J, Shulaev V, Schlauch K, Mittler R (2005) Cytosolicascorbateperoxidase 1 is a central component of the reactive oxygen gene network of Arabidopsis. PlantCell17: 268-281
Díaz-Martín J, Almoguera C, Prieto-Dapena P, Espinosa JM, Jordano J (2005) Functional Interaction between Two Transcription Factors Involved in the Developmental Regulation of a Small Heat Stress Protein Gene Promoter. Plant Physiol139: 1483-1494
Friedberg JN, Bowley SR, McKersie BD, Gurley WB, Czarnecka-Verner E (2006) Isolation and characterization of class A4 heat shock transcription factor from alfalfa. Plant Science 171: 332-344
Hiratsu K, Mitsuda N, Matsui K, Ohme-Takagi M (2004) Identification of theminimalrepressiondomain of SUPERMAN shows that the DLELRL hexapeptideisbothnecessary and sufficient for repression of transcription in Arabidopsis. BiochemBiophys Res Commun321: 172-178
Ikeda M, Ohme-Takagi M (2009) A novel group of transcriptionalrepressors in arabidopsis. Plant and CellPhysiology50: 970-975
James P, Halladay J, Craig EA (1996) Genomiclibraries and a host straindesignedforhighlyefficienttwo-hybridselection in yeast. Genetics144: 1425-1436
Kotak S, Vierling E, Bäumlein H, Koskull-Döring P (2007) A Novel Transcriptional Cascade Regulating Expression of Heat Stress Proteins during Seed Development of Arabidopsis. Plant Cell 19: 182-195
Li M, Doll J, Weckermann K, Oecking C, Berendzen KW, Schoffl F (2010) Detection of in vivo interactions between Arabidopsis class A-HSFs, using a novel BiFC fragment, and identification of novel class B-HSF interacting proteins. Eur J Cell Biol89: 126-132
Ostling P, Bjork JK, Roos-Mattjus P, Mezger V, Sistonen L (2007) Heat shock factor 2 (HSF2) contributes to inducible expression of hsp genes through interplay with HSF1. J BiolChem282: 7077-7086
Rojas A, Almoguera C, Carranco R, Scharf KD, Jordano J (2002) Selective Activation of the Developmentally Regulated Ha hsp17.6 G1 Promoter by Heat Stress Transcription Factors. Plant Physiol129: 1207-1215
Rousselin P, Molinier J, Himber C, Schontz D, Prieto-Dapena P, Jordano J, Martini N, Weber S, Horn R, Ganssmann M, Grison R, Pagniez M, Toppan A, Friedt W, Hahne G (2002) Modification of sunfloweroilqualitybyseed-specificexpression of a heterologous Δ9-stearoyl-(acylcarrierprotein) desaturase gene. PlantBreeding121: 108-116
Scharf KD, Heider H, Hohfeld I, Lyck R, Schmidt E, Nover L (1998) The tomato Hsf system: HsfA2 needs interaction with HsfA1 for efficient nuclear import and may be localized in cytoplasmic heat stress granules. Mol Cell Biol18: 2240-2251
Scharf KD, Berberich T, Ebersberger I, Nover L (2012) The plant heat stress transcription factor (Hsf) family: Structure, function and evolution. BiochimBiophysActa1819: 104-119
Tejedor-Cano J, Prieto-Dapena P, Almoguera C, Carranco R, Hiratsu K, Ohme-Takagi M, Jordano J (2010) Loss of function of the HSFA9 seed longevity program. Plant Cell Environ 33: 1408-1417
von Koskull-Döring P, Scharf KD, Nover L (2007) Thediversity of plant heat stress transcriptionfactors. TrendsPlantSci12: 452-457
Waadt R, Schmidt LK, Lohse M, Hashimoto K, Bock R, Kudla J (2008) Multicolor bimolecular fluorescence complementation reveals simultaneous formation of alternative CBL/CIPK complexes in planta. Plant J 56: 505-516
Yamanouchi U, Yano M, Lin H, Ashikari M, Yamada K (2002) A rice spottedleaf gene, Sp17, encodes a heat stress transcription factor protein. Proc Natl Acad Sci U S A 99: 7530-7535
Chen B, Przybyla AE (1994) Anefficientsite-directed mutagenesis method based on PCR. Biotechniques17: 657-659
Després C, Chubak C, Rochon A, Clark R, Bethune T, Desveaux D, Fobert PR (2003) TheArabidopsis NPR1 diseaseresistanceproteinis a novel cofactor thatconfersredoxregulation of DNA bindingactivitytothebasicdomain/leucine zipper transcription factor TGA1. PlantCell15: 2181-2191
Hobert O (2002) PCR fusion-basedapproachtocreatereporter gene constructs for expression analysis in transgenic C. elegans. Biotechniques32: 728-730
Tzfira T, Tian GW, Lacroix B, Vyas S, Li J, Leitner-Dagan Y, Krichevsky A, Taylor T, Vainstein A, Citovsky V (2005) pSATvectors: a modular series of plasmids for autofluorescent protein tagging and expression of multiple genes in plants. Plant Mol Biol 57: 503-516
Thomas et al (2009) Evolution of plant senescence BMC Evolutionary Biology, 9:163
Nover L., Bharti K., Döring P., Mishra S.K., Ganguli A. & Scharf K.D. (2001) Arabidopsis and the heat stress transcription factor world: how many heat stress transcription factors do we need? Cell Stress and Chaperones 6, 177-189.
Roberts IN, Caputo C, Criado MV, Funk C (2012) Senescence-associated proteases in plants. Physiol Plant 145: 130-139
Thomas H, De Villiers L (1996) Gene expression in leaves of Arabidopsis thaliana induced to senesce by nutrient deprivation. Journal of Experimental Botany 47: 1845-1852
Wingler A, Brownhill E, Pourtau N (2005) Mechanisms of the light-dependent induction of cell death in tobacco plants with delayed senescence. Journal of Experimental Botany 56: 2897-2905

## Claims

1. A method for delaying senescence of a plant or part thereof said method comprising introducing and expressing a nucleic acid comprising SEQ ID No. 1, a functional homolog, variant or part thereof in a plant or part thereof wherein said plant or part thereof is not a seed.

2. A method for improving tolerance to oxidative stress in a plant or part thereof said method comprising introducing and expressing a nucleic acid comprising SEQ ID No. 1, a functional homolog, variant or part thereof in a plant wherein said plant or part thereof is not a seed.

3. A method according to claim 1 or 2 wherein said plant or part thereof is vegetative tissue.

4. A method according to a preceding claim wherein said functional homolog has at least 90%, 91%, 92%, 93%, 94%, 95%, 96%, 97%, 98%, or 99% overall sequence identity with SEQ ID No. 1.

5. A method according to a preceding claim wherein said functional homolog is a class A HSF.

6. A method according to a preceding claim wherein said functional homolog comprises a DBD as defined in SEQ ID. 5 or which has at least 90%, 91%, 92%, 93%, 94%, 95%, 96%, 97%, 98% or 99% homology to SEQ ID. 5, a OD domain with the elements as defined in SEQ ID. 6 and 7 or which has at least 90%, 91%, 92%, 93%, 94%, 95%, 96%, 97%, 98% or 99% homology to SEQ ID. 6 and 7, a NLS as defined in SEQ ID. 78or which has at least 90%, 91%, 92%, 93%, 94%, 95%, 96%, 97%, 98% or 99% homology to SEQ ID. 8, an AHA1 domain as defined in SEQ ID. 9 or which has at least 90%, 91%, 92%, 93%, 94%, 95%, 96%, 97%, 98% or 99% homology to SEQ ID. 9, an AHA2 domain as defined in SEQ ID. 10 or which has at least 90%, 91%, 92%, 93%, 94%, 95%, 96%, 97%, 98% or 99% homology to SEQ ID. 10.

7. A method according to a preceding claim wherein said nucleic acid is operably linked to a regulatory sequence.

8. A method according to claim 7 wherein said regulatory sequence is a constitutive promoter.

9. A method according to claim 8 wherein said constitutive promoter is selected from CaMV-35S, CaMV-35Somega, Arabidopsis ubiquitin UBQ1.

10. A method according to preceding claim wherein said plant is a dicot plant.

11. A method according to any of claims 1 to 9 said plant is monocot plant.

12. A method according to preceding claim wherein said plant is a crop plant.

13. A method according to claim 12 wherein said crop plant is selected from maize, rice, wheat, oilseed rape, sorghum, soybean, potato, tomato, grape, barley, pea, bean, field bean, lettuce, cotton, sugar cane, sugar beet, broccoli or other vegetable brassicas or poplar.

14. A use of a nucleic acid comprising SEQ ID No. 1, a functional homolog, variant or part thereof in delaying senescence of a plant wherein said plant is not a seed.

15. A use of a nucleic acid comprising SEQ ID No. 1, a functional homolog, variant or part thereof in improving tolerance to oxidative stress of a plant or part thereof wherein said plant or part thereof is not a seed.

16. A use according to claim 14 or 15 wherein said plant or part thereof is vegetative tissue.

17. A method for generating a transgenic plant with delayed senescence comprising introducing and expressing a nucleic acid comprising SEQ ID No. 1, a functional homolog, variant or part thereof in a plant wherein said plant or part thereof is not a seed.

18. A method for generating a transgenic plant with improved tolerance to oxidative stress comprising introducing and expressing a nucleic acid comprising SEQ ID No. 1, a functional homolog, variant or part thereof in a plant wherein said plant or part thereof is not a seed.

19. A method according to claim 17 or 18 wherein said plant or part thereof is vegetative tissue.

20. A method for improving tolerance to oxidative stress and /or improving tolerance to dehydration in a plant said method comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant.

21. A method for delaying senescence of a plant or part thereof said method comprising introducing and expressing a nucleic acid comprising SEQ ID No. 1, a functional homolog, variant or part thereof and introducing and expressing a nucleic acid comprising SEQ ID No. 3, a functional homolog, variant or part thereof in a plant wherein said plant or part thereof is not a seed.

22. A method according to claim 20 or 21 wherein said plant or part thereof is vegetative tissue.

23. A method according to any of claims 17 to 22 wherein said functional homolog has at least 90%, 91%, 92%, 93%, 94%, 95%, 96%, 97%, 98%, or 99% overall sequence identity with SEQ ID No. 1 or SEQ ID No. 3.

24. A method according to any of claims 17 to 23 wherein said functional homolog is a class A HSF.

25. A method according to any of claims 17 to 24 wherein said HSF4 functional homolog comprises a DBD as defined in SEQ ID. 11 or which has at least 90%, 91%, 92%, 93%, 94%, 95%, 96%, 97%, 98% or 99% homology to SEQ ID. 11, a OD domain as defined in SEQ ID. 12 or which has at least 90%, 91%, 92%, 93%, 94%, 95%, 96%, 97%, 98% or 99% homology to SEQ ID. 12, a NLS as defined in SEQ ID. 13 or which has at least 90%, 91%, 92%, 93%, 94%, 95%, 96%, 97%, 98% or 99% homology to SEQ ID. 13, an AHA1 domain as defined in SEQ ID. 14 or which has at least 90%, 91%, 92%, 93%, 94%, 95%, 96%, 97%, 98% or 99% homology to SEQ ID. 14, an AHA2 domain as defined in SEQ ID. 15 or which has at least 90%, 91%, 92%, 93%, 94%, 95%, 96%, 97% , 98% or 99% homology to SEQ ID. 15 and an NES domain as defined in SEQ ID. 16 or which has at least 90%, 91%, 92%, 93%, 94%, 95%, 96%, 97%, 98% or 99% homology to SEQ ID. 16.

26. A use of a nucleic acid comprising SEQ ID No. 1, a functional homolog, variant or part thereof in combination with a nucleic acid comprising SEQ ID No. 3, a functional homolog, variant or part thereof for improving tolerance to oxidative stress and /or improving tolerance to dehydration in a plant.

27. A use of a nucleic acid comprising SEQ ID No. 1, a functional homolog, variant or part thereof in combination with a nucleic acid comprising SEQ ID No. 3, a functional homolog, variant or part thereof for delaying senescence of a plant or part thereof wherein said plant or part thereof is not a seed.

28. A chimeric construct comprising the LNK, OD and AD domains of SEQ ID No. 1, a functional homolog, variant or part thereof fused to N-terminal residues of a different HSF.

29. A chimeric construct according to claim 28 wherein said different HSF is a class A HSF.

30. A vector comprising the chimeric construct of claim 28 or 29.

31. An isolated cell expressing the chimeric construct of claim 28 or 29 or the vector of claim 30.

32. The cell of claim 31 wherein said cell is a bacterial or plant cell.

33. A transgenic plant cell expressing the chimeric construct of claim 28 or 29 or the vector of claim 30.

34. A product or harvestable part derived from a transgenic plant according to claim 32.

35. The harvestable part of claim 34 wherein said harvestable part is a seed.

36. An isolated nucleic acid comprising SEQ ID No. 3 or functional variant thereof.

37. A vector comprising SEQ ID No. 3 according to claim 36.

38. An isolated cell expressing SEQ ID No. 3 or functional variant thereof according to claim 33 or a vector according to claim 37.

39. A transgenic plant co-expressing a nucleic acid comprising SEQ ID No. 3 and a nucleic acid sequence comprising SEQ ID No. 1.


SEQUENCE LISTING

<110>  Consejo Superior de Investigaciones Cientificas

<120>  Stress Tolerant Plants

<130>  PC926790GB

<160>  40

<170>  PatentIn version 3.3

<210>  1
<211>  1434
<212>  DNA
<213>  Artificial

<220>
<223>  HaHSFA9 full-length cDNA (Accession number AY099451)

<400>  1
atattctcct tcaaaaacac acacacacaa acaaacaaac ttgtttttga tttgtacttc      60

atggcaggag tagtaaaaga gtttgactct agtcatgata tgttcaaagt cagagaagaa     120

cccaaggtcg tttttaataa taaagatttg tctgaagaga tgatgaagat taaagaggaa     180

cccatgttag tgtttgatga gcatgaagat tttgttggtg gtgaggaggc accgaagccg     240

attgaagggc tgagagacgg cggacctccg ccgtttctca agaaaacatt tgaaatggtg     300

gatgacccaa caacagattc gattatttca tggagtagtt ctaaaaatag ttttattctt     360

tgggatcctc ataagttttc tactgatctt ttgccgcaaa gattcaagca caataatttc     420

tctagctttg ttcgtcaact aaacacttat aggttcaaga aaatcgatcc tgaccgatgg     480

gaattcgcaa acgagttttt ccaaaagggt aagaagcatt tgttaagaga cataaagagg     540

agaacaaacc agccacaaaa cacacaaaaa caagaggaaa tcaggaaaca agaacaacaa     600

caatgttgtg gtcatcaaac aaactctaca atggagaccg agctaaagaa tctaagaaaa     660

gaaagaatca cacttaaaca agagatcttg aagatgaaac agcaacaaga gaacaccgaa     720

aagcatttgg aaatggttga agaacgtatg ctaagaatgg agttcaagca acagcagctg     780

ttagttttca tgtctaaagc gtttagaaat ccgattttcg ttaagctttt gcaacatctg     840

gtgcaaaaac aaaaaacggg aagtgtcgaa atgtgcaaaa agcgaaaact tgagcaaatg     900

ctaaacactg atgatctcga tcgttttcaa gaaatgtgga atatgattga acctgatgcg     960

tatactgtgt tgtcatcaga tggatcagta agcccgcctg aagatcaaaa cacgagtgac    1020

aaatctggat ccaacggttc agattacaat tcggaaagct ttatattgtg ggagaaacta    1080

atggaggacg aactgatatt cggcggtgaa caatcgggta aagatcaaac agaaacttat    1140

ctccaagaat gggaagagtt gatcccaaaa gcttaagaag ggggattctg atgcagtcat    1200

gtgatcatta tcatcggtaa catgataaag agcatctggt agggtgctgc agtgctcggg    1260

taagctttat cttctggtac tgggcagttg atgtagatgt agaaacaaat ggggtacagt    1320

ataggtgttt gtgtgtgtgt tgtatttgtt tttgttgtat tttggttggg gtcttgtaga    1380

aataacgatg acaataaact ccgtagtttc taatagaatt tagggttgta aatc          1434

<210>  2
<211>  371
<212>  PRT
<213>  Artificial

<220>
<223>  Predicted HaHSFA9 protein

<400>  2

Met Ala Gly Val Val Lys Glu Phe Asp Ser Ser His Asp Met Phe Lys
1               5                   10                  15

Val Arg Glu Glu Pro Lys Val Val Phe Asn Asn Lys Asp Leu Ser Glu
            20                  25                  30

Glu Met Met Lys Ile Lys Glu Glu Pro Met Leu Val Phe Asp Glu His
        35                  40                  45

Glu Asp Phe Val Gly Gly Glu Glu Ala Pro Lys Pro Ile Glu Gly Leu
    50                  55                  60

Arg Asp Gly Gly Pro Pro Pro Phe Leu Lys Lys Thr Phe Glu Met Val
65                  70                  75                  80

Asp Asp Pro Thr Thr Asp Ser Ile Ile Ser Trp Ser Ser Ser Lys Asn
                85                  90                  95

Ser Phe Ile Leu Trp Asp Pro His Lys Phe Ser Thr Asp Leu Leu Pro
            100                 105                 110

Gln Arg Phe Lys His Asn Asn Phe Ser Ser Phe Val Arg Gln Leu Asn
        115                 120                 125

Thr Tyr Arg Phe Lys Lys Ile Asp Pro Asp Arg Trp Glu Phe Ala Asn
    130                 135                 140

Glu Phe Phe Gln Lys Gly Lys Lys His Leu Leu Arg Asp Ile Lys Arg
145                 150                 155                 160

Arg Thr Asn Gln Pro Gln Asn Thr Gln Lys Gln Glu Glu Ile Arg Lys
                165                 170                 175

Gln Glu Gln Gln Gln Cys Cys Gly His Gln Thr Asn Ser Thr Met Glu
            180                 185                 190

Thr Glu Leu Lys Asn Leu Arg Lys Glu Arg Ile Thr Leu Lys Gln Glu
        195                 200                 205

Ile Leu Lys Met Lys Gln Gln Gln Glu Asn Thr Glu Lys His Leu Glu
    210                 215                 220

Met Val Glu Glu Arg Met Leu Arg Met Glu Phe Lys Gln Gln Gln Leu
225                 230                 235                 240

Leu Val Phe Met Ser Lys Ala Phe Arg Asn Pro Ile Phe Val Lys Leu
                245                 250                 255

Leu Gln His Leu Val Gln Lys Gln Lys Thr Gly Ser Val Glu Met Cys
            260                 265                 270

Lys Lys Arg Lys Leu Glu Gln Met Leu Asn Thr Asp Asp Leu Asp Arg
        275                 280                 285

Phe Gln Glu Met Trp Asn Met Ile Glu Pro Asp Ala Tyr Thr Val Leu
    290                 295                 300

Ser Ser Asp Gly Ser Val Ser Pro Pro Glu Asp Gln Asn Thr Ser Asp
305                 310                 315                 320

Lys Ser Gly Ser Asn Gly Ser Asp Tyr Asn Ser Glu Ser Phe Ile Leu
                325                 330                 335

Trp Glu Lys Leu Met Glu Asp Glu Leu Ile Phe Gly Gly Glu Gln Ser
            340                 345                 350

Gly Lys Asp Gln Thr Glu Thr Tyr Leu Gln Glu Trp Glu Glu Leu Ile
        355                 360                 365

Pro Lys Ala
    370

<210>  3
<211>  1516
<212>  DNA
<213>  Artificial

<220>
<223>  HaHSFA4 full-length cDNA (Accession number HF549285)

<400>  3
   ggacactgac atggactgaa ggagtagaaa aagtagtgag tttcgacact tggggtgacg      60
   tttcaagcac tttggaagcc attttggata atattttggt tcaggggatt aggattagac     120
   tcatcaaaat tactttttag ctctgttttg gagttatttt tgggagtatt atcttgatgt     180
   agtttggaaa ttgaagtgat cttgattgtt gttggtatat cttggtcaat gatgaatgat     240
   gttcatggga atttgaattc gaattcgaat tcgggttcaa atgctctgcc cccttttatc     300
   gcgaaaacgt atgaaatggt cgatgatcct tctacaaatt ccatcgtttc gtggagtcag 360
   actaaccgga gtttcatcgt gtggaacccg cctgaattct cgggtgaatt gcttccaaga 420
   ttcttcaagc ataacaattt ctcaagcttc atcagacaac ttaatactta tggttttcga 480
   aaaatcgatc ctgaattatg ggaatttgct aatgaggatt tcattagagg tcaaccacat 540
   ctgttaaaga acatccatag aagaaaaccg gttcatagtc attcgattca caatcttgca 600
   aacaatggat catcatcgtc gtcgtctcca ttgaccgaat cagagaaaca acaatacata 660
   gaaaagatta gcaccctgca acacgagaaa gaatcgttgt ctatacagtt tcacatgcac 720
   aaacaagaac aggagaggat caaattcgaa gcacacgggt taacagaccg tttaaaacgg 780
   gctgcaaagc tgcaaaaaga catcctttgt tgcttagacg agttcttgca gaaaccacct 840
   caagaatgcg aattcacgcc tcggttgatg gagaatcgaa agaggagatt atcgtctgaa 900
   aacgttaacg aacaagtttg tcctttcgat atcccgattc gcgagacgat aaccgccgac 960
   gcgcttctag cgttgaacac cgaattggta gaacagttgg agtcctcagt catgtcatgg 1020
   gaaggtatat tcaaagaagt tgatgatgag tttgagatgc aaaaatggtc aatggaattg 1080
   gatcacgggc ccgtaggttg tgcggatagc ccaactattg attacccgtt aatcaatgtc 1140
   gaaacgggct ctaaagaaag tgagattgac atgaattcta aaccggttaa cccggttccg 1200
   actgaagaaa cggcggttaa cgatgttttt tgggagcagt tcttgaccga gaacccgggc 1260
   gggtcaacgg gtaataatgt aggcgaaatg gtgagtgaag atagaaaata tgggaagttt 1320
   tggtggaata tgagtagtgt aaataatctt gctgatcagc taggacagct tacttcagta 1380
   gagagaagtt gataatggtg gattgtctaa atttactatt aatctgttat ttgtttaata 1440
   ttggtggggt ggttgtaaat ttggtatgag ggtttatatg atttttgagc gttttgtaga 1500
   taaaaaaaaa aaaaaa 1516

<210> 4
<211> 387
<212> PRT
<213> Predicted HaHSFA4 protein (aa 1-387)

<400> 4

Met Met Asn Asp Val His Gly Asn Leu Asn Ser Asn Ser Asn Ser Gly
1               5                   10                  15

Ser Asn Ala Leu Pro Pro Phe Ile Ala Lys Thr Tyr Glu Met Val Asp
            20                  25                  30

Asp Pro Ser Thr Asn Ser Ile Val Ser Trp Ser Gln Thr Asn Arg Ser
        35                  40                  45

Phe Ile Val Trp Asn Pro Pro Glu Phe Ser Gly Glu Leu Leu Pro Arg
    50                  55                  60

Phe Phe Lys His Asn Asn Phe Ser Ser Phe Ile Arg Gln Leu Asn Thr
65                  70                  75                  80

Tyr Ser Phe Arg Lys Ile Asp Pro Glu Leu Trp Glu Phe Ala Asn Glu
                85                  90                  95

Asp Phe Ile Arg Gly Gln Pro His Leu Leu Lys Asn Ile His Arg Arg
            100                 105                 110

Lys Pro Val His Ser His Ser Ile His Asn Leu Ala Asn Asn Gly Ser
        115                 120                 125

Ser Ser Ser Ser Ser Pro Leu Thr Glu Ser Glu Lys Gln Gln Tyr Ile
    130                 135                 140

Glu Lys Ile Ser Thr Leu Gln His Glu Lys Glu Ser Leu Ser Ile Gln
145                 150                 155                 160

Phe His Met His Lys Gln Glu Gln Glu Arg Ile Lys Phe Glu Ala His
                165                 170                 175

Gly Leu Thr Asp Arg Leu Lys Arg Ala Ala Lys Leu Gln Lys Asp Ile
            180                 185                 190

Leu Cys Cys Leu Asp Glu Phe Leu Gln Lys Pro Pro Gln Glu Cys Glu
        195                 200                 205

Phe Thr Pro Arg Leu Met Glu Asn Arg Lys Arg Arg Leu Ser Ser Glu
    210                 215                 220

Asn Val Asn Glu Gln Val Cys Pro Phe Asp Ile Pro Ile Arg Glu Thr
225                 230                 235                 240

Ile Thr Ala Asp Ala Leu Leu Ala Leu Asn Thr Glu Leu Val Glu Gln
                245                 250                 255

Leu Glu Ser Ser Val Met Ser Trp Glu Gly Ile Phe Lys Glu Val Asp
            260                 265                 270

Asp Glu Phe Glu Met Gln Lys Trp Ser Met Glu Leu Asp His Gly Pro
        275                 280                 285

Val Gly Cys Ala Asp Ser Pro Thr Ile Asp Tyr Pro Leu Ile Asn Val
    290                 295                 300

Glu Thr Gly Ser Lys Glu Ser Glu Ile Asp Met Asn Ser Lys Pro Val
305                 310                 315                 320

Asn Pro Val Pro Thr Glu Glu Thr Ala Val Asn Asp Val Phe Trp Glu
                325                 330                 335

Gln Phe Leu Thr Glu Asn Pro Gly Gly Ser Thr Gly Asn Asn Val Gly
            340                 345                 350

Glu Met Val Ser Glu Asp Arg Lys Tyr Gly Lys Phe Trp Trp Asn Met
        355                 360                 365

Ser Ser Val Asn Asn Leu Ala Asp Gln Leu Gly Gln Leu Thr Ser Val
    370                 375                 380

Glu Arg Ser
385

<210>  5
<211>  94
<212>  PRT
<213>  Artificial

<220>
<223>  DBD of HaHSFA9

<400>  5

Pro Pro Pro Phe Leu Lys Lys Thr Phe Glu Met Val Asp Asp Pro Thr
1               5                   10                  15

Thr Asp Ser Ile Ile Ser Trp Ser Ser Ser Lys Asn Ser Phe Ile Leu
            20                  25                  30

Trp Asp Pro His Lys Phe Ser Thr Asp Leu Leu Pro Gln Arg Phe Lys
        35                  40                  45

His Asn Asn Phe Ser Ser Phe Val Arg Gln Leu Asn Thr Tyr Arg Phe
    50                  55                  60

Lys Lys Ile Asp Pro Asp Arg Trp Glu Phe Ala Asn Glu Phe Phe Gln
65                  70                  75                  80

Lys Gly Lys Lys His Leu Leu Arg Asp Ile Lys Arg Arg Thr
                85                  90

<210>  6
<211>  22
<212>  PRT
<213>  Artificial

<220>
<223>  Motifs in the OD of HaHSFA9 (HR-A)

<400>  6

Met Glu Thr Glu Leu Lys Asn Leu Arg Lys Glu Arg Ile Thr Leu Lys

1               5                   10                  15

Gln Glu Ile Leu Lys Met
            20

<210>  7
<211>  8
<212>  PRT
<213>  Artificial

<220>
<223>  Motifs in the OD of HaHSFA9 (HR-B)

<400>  7

Leu Leu Val Phe Met Ser Lys Ala
1               5

<210>  8
<211>  26
<212>  PRT
<213>  Artificial

<220>
<223>  Motifs in the OD of HaHSFA9 (bipartite NLS)

<220>
<221>  REPEAT
<222>  (16)..(16)
<223>  The aa at position 16 represented by a Xaa, is a repeat site of 1
       to 12 Xaa amino acids.  Where Xaa is any amino acid.

<400>  8

Gln Lys Gln Lys Thr Gly Ser Val Glu Met Cys Lys Lys Arg Lys Xaa
1               5                   10                  15

Phe Gln Glu Met Trp Asn Met Ile Glu Pro
            20                  25

<210>  9
<211>  10
<212>  PRT
<213>  Artificial

<220>
<223>  Motifs in the OD of HaHSFA9 (AHA1)

<400>  9

Phe Ile Leu Trp Glu Lys Leu Met Glu Asp
1               5                   10

<210>  10
<211>  9
<212>  PRT
<213>  Artificial

<220>
<223>  Motifs in the OD of HaHSFA9 (AHA2)

<400>  10

Leu Gln Glu Trp Glu Glu Leu Ile Pro
1               5

<210>  11
<211>  93
<212>  PRT
<213>  Artificial

<220>
<223>  HaHSFA4 DBD (aa 21-113)

<400>  11

Pro Pro Phe Ile Ala Lys Thr Tyr Glu Met Val Asp Asp Pro Ser Thr
1               5                   10                  15

Asn Ser Ile Val Ser Trp Ser Gln Thr Asn Arg Ser Phe Ile Val Trp
            20                  25                  30

Asn Pro Pro Glu Phe Ser Gly Glu Leu Leu Pro Arg Phe Phe Lys His
        35                  40                  45

Asn Asn Phe Ser Ser Phe Ile Arg Gln Leu Asn Thr Tyr Ser Phe Arg
    50                  55                  60

Lys Ile Asp Pro Glu Leu Trp Glu Phe Ala Asn Glu Asp Phe Ile Arg
65                  70                  75                  80

Gly Gln Pro His Leu Leu Lys Asn Ile His Arg Arg Lys
                85                  90

<210>  12
<211>  57
<212>  PRT
<213>  Artificial

<220>
<223>  HaHSFA4 OD (aa 143-199):

<400>  12

Tyr Ile Glu Lys Ile Ser Thr Leu Gln His Glu Lys Glu Ser Leu Ser
1               5                   10                  15

Ile Gln Phe His Met His Lys Gln Glu Gln Glu Arg Ile Lys Phe Glu
            20                  25                  30

Ala His Gly Leu Thr Asp Arg Leu Lys Arg Ala Ala Lys Leu Gln Lys
        35                  40                  45

Asp Ile Leu Cys Cys Leu Asp Glu Phe
    50                  55

<210>  13
<211>  7
<212>  PRT
<213>  Artificial

<220>
<223>  HaHSFA4 NLS (aa 216-222)

<400>  13

Asn Arg Lys Arg Arg Leu Ser
1               5

<210>  14
<211>  10
<212>  PRT
<213>  Artificial

<220>
<223>  HaHSFA4 AHA1 (aa 261-270)

<400>  14

Val Met Ser Trp Glu Gly Ile Phe Lys Glu
1               5                   10

<210>  15
<211>  10
<212>  PRT
<213>  Artificial

<220>
<223>  HaHSFA4 AHA2 (aa 332-341)

<400>  15

Asp Val Phe Trp Glu Gln Phe Leu Thr Glu
1               5                   10

<210>  16
<211>  8
<212>  PRT
<213>  Artificial

<220>
<223>  HaHSFA4 NES (aa 374-381)

<400>  16

Leu Ala Asp Gln Leu Gly Gln Leu
1               5

<210>  17
<211>  28
<212>  PRT
<213>  Artificial

<220>
<223>  HaHSFA9 LNK (aa 163 - 190)

<400>  17

Asn Gln Pro Gln Asn Thr Gln Lys Gln Glu Glu Ile Arg Lys Gln Glu
1               5                   10                  15

Gln Gln Gln Cys Cys Gly His Gln Thr Asn Ser Thr
            20                  25

<210>  18
<211>  124
<212>  PRT
<213>  Artificial

<220>
<223>  HaHSFA9 CTAD (aa 248 - 371)

<400>  18

Phe Arg Asn Pro Ile Phe Val Lys Leu Leu Gln His Leu Val Gln Lys
1               5                   10                  15

Gln Lys Thr Gly Ser Val Glu Met Cys Lys Lys Arg Lys Leu Glu Gln
            20                  25                  30

Met Leu Asn Thr Asp Asp Leu Asp Arg Phe Gln Glu Met Trp Asn Met
        35                  40                  45

Ile Glu Pro Asp Ala Tyr Thr Val Leu Ser Ser Asp Gly Ser Val Ser
    50                  55                  60

Pro Pro Glu Asp Gln Asn Thr Ser Asp Lys Ser Gly Ser Asn Gly Ser
65                  70                  75                  80

Asp Tyr Asn Ser Glu Ser Phe Ile Leu Trp Glu Lys Leu Met Glu Asp
                85                  90                  95

Glu Leu Ile Phe Gly Gly Glu Gln Ser Gly Lys Asp Gln Thr Glu Thr
            100                 105                 110

Tyr Leu Gln Glu Trp Glu Glu Leu Ile Pro Lys Ala
        115                 120

<210>  19
<211>  10
<212>  PRT
<213>  Artificial

<220>
<223>  HaHSFA9 CTAD AHA motif

<400>  19

Phe Gln Glu Met Trp Asn Met Ile Glu Pro
1               5                   10


<210>  20
<211>  10
<212>  PRT
<213>  Artificial

<220>
<223>  HaHSFA9 CTAD AHA motif

<400>  20

Phe Ile Leu Trp Glu Lys Leu Met Glu Asp
1               5                   10


<210>  21
<211>  8
<212>  PRT
<213>  Artificial

<220>
<223>  HaHSFA9 CTAD AHA motif

<400>  21

Gln Glu Trp Glu Glu Leu Ile Pro
1               5


<210>  22
<211>  17
<212>  DNA
<213>  Artificial

<220>
<223>  PCR Primer

<400>  22
catctcttca gacaaat                                                    17


<210>  23
<211>  23
<212>  DNA
<213>  Artificial

<220>
<223>  PCR Primer

<400>  23
actatccttc gcaagaccct tcc                                             23


<210>  24
<211>  17
<212>  DNA
<213>  Artificial

<220>
<223>  PCR Primer

<400>  24

catctcttca gacaaat                                                    17

<210>  25
<211>  20
<212>  DNA
<213>  Artificial

<220>
<223>  PCR Primer

<400>  25
ccaccacgtc atcataccac                                                 20

<210>  26
<211>  26
<212>  DNA
<213>  Artificial

<220>
<223>  HSFA4-RACE Primer

<400>  26
ggagacgacg acgatgatga tccatt                                          26

<210>  27
<211>  35
<212>  DNA
<213>  Artificial

<220>
<223>  PCR Primer

<400>  27
aaacgaattc gcctctagac actgacatgg actga                                35

<210>  28
<211>  21
<212>  DNA
<213>  Artificial

<220>
<223>  PCR Primer

<400>  28
tgatccattg tttgcaagat t                                               21

<210>  29
<211>  42
<212>  DNA
<213>  Artificial

<220>
<223>  Oligonucleotide

<400>  29
ggtatatctt ggtccatggt gaatgatgtt catgggaatt tg                        42

<210>  30
<211>  25

<212>  DNA
<213>  Artificial

<220>
<223>  Oligonucleotide

<400>  30
gagctcggta cccggggatc tgcag                                          25


<210>  31
<211>  49
<212>  DNA
<213>  Artificial

<220>
<223>  Mutagenic Primer

<400>  31
tgtaaataat gctgctgatc aggcaggaca ggctacttca gtagagaga                49


<210>  32
<211>  23
<212>  DNA
<213>  Artificial

<220>
<223>  PCR Primer

<400>  32
actatccttc gcaagaccct tcc                                            23


<210>  33
<211>  46
<212>  DNA
<213>  Artificial

<220>
<223>  PCR Primer

<400>  33
gtttttgtgt gttttgtggc tggcaagcac cagatccttg ttgatt                   46


<210>  34
<211>  22
<212>  DNA
<213>  Artificial

<220>
<223>  PCR Primer (OliC)

<400>  34
cagccacaaa acacacaaaa ac                                             22


<210>  35
<211>  22
<212>  DNA
<213>  Artificial

<220>
<223>  PCR Primer (OliD)

<400>  35
tcggaattaa ccctcactaa ag                                                22

<210>  36
<211>  17
<212>  DNA
<213>  Artificial

<220>
<223>  PCR Primer (OliD')

<400>  36
tctagaacta gtggatc                                                      17

<210>  37
<211>  41
<212>  DNA
<213>  Artificial

<220>
<223>  PCR Primer

<400>  37
gttgttggta tatctagatc aatgatgaat gatgttcatg g                           41

<210>  38
<211>  41
<212>  DNA
<213>  Artificial

<220>
<223>  PCR Primer

<400>  38
gtaaatttag acagtcgacc attatcaact tctctctact g                           41

<210>  39
<211>  20
<212>  DNA
<213>  Artificial

<220>
<223>  PCR Primer

<400>  39
tctagtaaaa atggcatacc                                                   20

<210>  40
<211>  20
<212>  DNA
<213>  Artificial

<220>
<223>  PCR Primer

<400>  40
ttatcaactt ctctctactg                                                   20